(19)

**Europäisches Patentamt**
**European Patent Office**
**Office européen des brevets**

(11) **EP 4 549 463 A2**

(12) **EUROPEAN PATENT APPLICATION**

(43) Date of publication:
**07.05.2025 Bulletin 2025/19**

(21) Application number: **24193902.4**

(22) Date of filing: **16.06.2017**

(51) International Patent Classification (IPC):
***C07K 16/22*** *(2006.01)*

(52) Cooperative Patent Classification (CPC):
**C07K 16/065; A61P 27/02; A61P 35/00;**
**C07K 1/165; C07K 1/18; C07K 1/22; C07K 1/36;**
**C07K 16/22;** C07K 2317/31; C07K 2317/526;
C07K 2317/54; C07K 2317/76

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **17.06.2016 US 201662351908 P**

(62) Document number(s) of the earlier application(s) in
accordance with Art. 76 EPC:
**17751870.1 / 3 472 177**

(27) Previously filed application:
**16.06.2017 US PCT/US2017/038007**

(71) Applicant: **F. Hoffmann-La Roche AG**
**4070 Basel (CH)**

(72) Inventors:
- **GIESE, Glen Scott**
  **South San Francisco, CA 94080 (US)**
- **ROSENBERG, Eva**
  **4070 Basel (CH)**
- **SALLIER, Bernard**
  **4070 Basel (CH)**
- **KONRAD, Susanne**
  **4070 Basel (CH)**

- **KOEHNLEIN, Wolfgang**
  **4070 Basel (CH)**
- **WILLMANN, Steffen**
  **4070 Basel (CH)**
- **BIALAS, Agathe**
  **4070 Basel (CH)**
- **KALEAS-CARROLL, Kimberly Ann**
  **South San Francisco, CA 94080 (US)**
- **YIGZAW, Yinges**
  **South San Francisco, CA 94080 (US)**

(74) Representative: **Heyse, Gero et al**
**Roche Diagnostics GmbH**
**Nonnenwald 2**
**82377 Penzberg (DE)**

Remarks:
•The complete document including Reference
Table(s) and the Sequence Listing(s) can be
downloaded from the EPO website
•This application was filed on 09.08.2024 as a
divisional application to the application mentioned
under INID code 62.

(54) **PURIFICATION OF MULTISPECIFIC ANTIBODIES**

(57) The present invention provides methods of purifying multispecific antibodies. The methods comprise the sequential steps of performing a capture chromatography, a first mixed mode chromatography and a second mixed mode chromatography. In some aspects, the invention provides compositions of multispecific antibodies, which compositions have reduced levels of one or more product-specific impurities and/or process-specific impurities.

EP 4 549 463 A2

**Description**

CROSS-REFERENCE TO RELATED APPLICATIONS

This application claims the priority benefit of U.S. Provisional Application Serial No. 62/351,908, filed June 17, 2016, which is incorporated herein by reference in its entirety.

SUBMISSION OF SEQUENCE LISTING ON ASCII TEXT FILE

The content of the following submission on ASCII text file is incorporated herein by reference in its entirety: a computer readable form (CRF) of the Sequence Listing (file name: 146392036340SEQLIST.TXT, date recorded: June 9, 2017, size: 32 KB).

FIELD OF THE INVENTION

Provided are methods for purifying multispecific antibodies from a composition comprising the multispecific antibody and at least one impurity, including at least one product-specific impurity. In some embodiments, the product-specific impurity is, for example, a precursor, aggregate, and/or variant of the multispecific antibody. Also provided are multispecific antibodies purified according to the methods, and compositions and formulations comprising such multispecific antibodies.

BACKGROUND OF THE INVENTION

For recombinant biopharmaceutical proteins to be acceptable for administration to human patients, it is important that residual impurities resulting from the manufacture and purification process are removed from the final biological product. These process components include culture medium proteins, immunoglobulin affinity ligands, viruses, endotoxin, DNA, and host cell proteins (HCPs). The development of new antibody formats, such as multispecific antibodies, presents new challenges as conventional manufacturing and purification processes are inadequate to sufficiently remove product-specific impurities, including non-paired antibody arms and misassembled antibodies.

As compared to the purification of standard antibodies, the purification of multispecific antibodies from production media presents unique challenges. While a standard mono-specific bivalent antibody results from the dimerization of identical heavy-chain/light-chain subunits, the production of a multispecific antibody requires dimerization of at least two different heavy-chain/light-chain subunits, each comprising a different heavy chain as well as a different light chain. The production and purification of the final correct and complete multispecific antibody, with minimal amounts of mispaired, mis-assembled, or incomplete molecules presents different challenges. Chain mispairings (e.g., homo-dimerization of identical heavy chain peptides or improper heavy-chain/light-chain associations) are often observed, as is incomplete protein assembly due to unbalanced host cell expression of the different antibody chains. Commonly observed product-specific impurities include half (½) antibodies (comprising a single heavy-chain/light-chain pair), three-quarter (¾) antibodies (comprising a complete antibody lacking a single light chain), and homodimers. Additional product-specific impurities may be observed depending on the multispecific format used. For example, where one variable domain of the multispecific antibody is constructed as a single-chain Fab (scFab), a 5/4 antibody by-product (comprising an additional heavy or light chain variable domain) may be observed. Such corresponding product-specific impurities would not arise in standard antibody production.

Conventional purification techniques designed to remove process-related impurities such as HCPs, DNA, endotoxins, and other materials that have very different characteristics and properties from the antibodies can be inadequate when implemented to remove impurities that are more similar to the multispecific antibodies. As such, there is a need to develop manufacturing and purification schemes that effectively remove product-specific impurities and yield sufficient amount of the correct and complete multispecific antibody.

All references cited herein, including patent applications and publications, are incorporated by reference in their entirety.

SUMMARY OF THE INVENTION

As described and exemplified herein, Applicants have discovered that the use of at least two mixed mode (also referred to herein as multi-modal or multimodal) chromatography steps after an initial capture chromatography step results in greater removal of product-specific impurities and an improved process for purifying multispecific antibodies. Thus, in certain embodiments, provided is a method for purifying a multispecific antibody from a composition comprising the multispecific antibody and an impurity, wherein the multispecific antibody comprises multiple arms, each arm comprising a VH/VL unit, the method comprising the sequential steps of: a) subjecting the composition to a capture chromatography to produce a capture chromatography eluate; b) subjecting the capture chromatography eluate to a first mixed mode chromatography to generate a first mixed mode eluate; c) subjecting the first mixed mode eluate to a second mixed mode chromatography to generate a second mixed mode eluate; and d) collecting a fraction comprising the multispecific antibody, wherein the method reduces the amount of a product-specific impurity from the composition. In some embodiments according to (or as applied to) any of the embodiments above, the capture chromatography eluate is subjected to an ion exchange chromatography (*e.g.*, anion exchange) or to hydrophobic interaction chromatography prior to the first mixed mode chromatography. In some embodiments according to (or as applied to) any of the embodiments above, the second mixed mode eluate is subjected to an ion exchange chromatography *(e.g.,* anion exchange) or to hydrophobic interaction chromatography.

In certain embodiments, provided is a method for purifying a multispecific antibody from a composition comprising the multispecific antibody and an impurity, wherein the multispecific antibody comprises multiple arms, each arm comprising a VH/VL unit, wherein each arm of the multispecific antibody is produced separately, the method comprising the sequential steps of a) subjecting each arm of the multispecific antibody to capture chromatography to produce capture eluates for each arm of the multispecific antibody, b) forming a mixture comprising capture eluates of each arm of the multispecific antibody under conditions sufficient to produce a composition comprising the multispecific antibody, c) subjecting the composition comprising the multispecific antibody to a first mixed mode chromatography to generate a first mixed mode eluate, and d) subjecting the first mixed mode eluate to a second mixed mode chromatography to generate a second mixed mode eluate; and e) collecting a fraction comprising the multispecific antibody, wherein the method reduces the amount of a product-specific impurity from the composition. In some embodiments according to (or as applied to) any of the embodiments above, the capture chromatography eluate is subjected to an ion exchange chromatography (*e.g.*, anion exchange) or to hydrophobic interaction chromatography prior to the first mixed mode chromatography. In some embodiments according to (or as applied to) any of the embodiments above, the second mixed mode eluate is subjected to an ion exchange chromatography (*e.g.*, anion exchange) or to hydrophobic interaction chromatography.

In some embodiments according to (or as applied to) any of the embodiments above, the capture chromatography is affinity chromatography. In some embodiments according to (or as applied to) any of the embodiments above, the affinity chromatography is protein L chromatography, protein A chromatography, protein G chromatography, protein A and protein G chromatography. In some embodiments according to (or as applied to) any of the embodiments above, the affinity chromatography is protein A chromatography. In some embodiments according to (or as applied to) any of the embodiments above, the capture chromatography is carried out in bind and elute mode.

In some embodiments according to (or as applied to) any of the embodiments above, the first mixed mode chromatography and the second mixed mode chromatography are contiguous. In some embodiments according to (or as applied to) any of the embodiments above, the first mixed mode chromatography is a mixed mode anion exchange chromatography. In some embodiments according to (or as applied to) any of the embodiments above, the second mixed mode chromatography is a mixed mode cation exchange chromatography. In some embodiments according to (or as applied to) any of the embodiments above, the first mixed mode chromatography is a mixed mode cation exchange chromatography. In some embodiments according to (or as applied to) any of the embodiments above, the second mixed mode chromatography is a mixed mode anion exchange chromatography.

In some embodiments according to (or as applied to) any of the embodiments above, the first mixed mode chromatography is carried out in bind and elute mode or in flow-through mode. In some embodiments according to (or as applied to) any of the embodiments in which the first mixed mode chromatography is carried out in bind and elute mode, the elution is a gradient elution.

In some embodiments according to (or as applied to) any of the embodiments above, the second mixed mode chromatography is carried out in bind and elute mode or in flow-through mode. In some embodiments according to (or as applied to) any of the embodiments in which the second mixed mode chromatography is carried out in bind and elute mode, the elution is a gradient elution.

In some embodiments according to (or as applied to) any of the embodiments above, the method further comprises the step of subjecting the second mixed mode eluate to ultrafiltration. In some embodiments according to (or as applied to) any of the embodiments above, the ultrafiltration comprises sequentially a first ultrafiltration, a diafiltration and a second ultrafiltration.

In some embodiments according to (or as applied to) any of the embodiments above, the protein A chromatography comprises protein A linked to agarose. In some embodiments according to (or as applied to) any of the embodiments above, the protein A chromatography is a MAbSelect™, MAbSelect™ SuRe and MAbSelect™ SuRe LX, Prosep-VA, Prosep-VA Ultra Plus, Protein A sepharose fast flow, or Toyopearl Protein A chromatography. In some embodiments according to (or as applied to) any of the embodiments above, the protein A chromatography uses one or more of a protein A equilibration buffer, a protein A loading buffer or a protein A wash buffer wherein the equilibration buffer, a loading buffer, and/or wash buffer is between about pH 7 and about pH 8. In some embodiments according to (or as applied to) any of the embodiments above, the protein A equilibration buffer is about pH 7.7. In some embodiments according to (or as applied to) any of the embodiments above, the protein A equilibration buffer comprises about 25 mM Tris and about 25 mM NaCl. In some embodiments according to (or as applied to) any of the embodiments above, the protein A chromatography is washed with equilibration buffer following load. In some embodiments according to (or as applied to) any of the embodiments above, the multispecific antibody is eluted from the protein A by applying a protein A elution buffer with low pH to the protein A chromatography. In some embodiments according to (or as applied to) any of the embodiments above, the protein A elution buffer comprises about 150 mM acetic acid, about pH 2.9. In some embodiments according to (or as applied to) any of the embodiments above, the multispecific antibody is eluted from the protein A chromatography by pH gradient.

In some embodiments according to (or as applied to) any of the embodiments above, the anion exchange mixed mode chromatography comprises a quaternary amine and a hydrophobic moiety. In some embodiments according to (or as applied to) any of the embodiments above, the anion exchange mixed mode chromatography comprises a quaternary amine and a hydrophobic moiety linked to highly crosslinked agarose. In some embodiments according to (or as applied to) any of the embodiments above, the mixed mode chromatography is a Capto™ Adhere chromatography or a Capto™ Adhere ImpRes chromatography. In some embodiments according to (or as applied to) any of the embodiments above, the cation exchange mixed mode chromatography comprises N-benzyl-n-methyl ethanolamine. In some embodiments according to (or as applied to) any of the embodiments above, the mixed mode chromatography is a Capto™ MMC chromatography or a Capto™ MMC ImpRes chromatography.

In some embodiments according to (or as applied to) any of the embodiments above, the first mixed mode chromatography uses one or more of a mixed mode pre-equilibration buffer, a mixed mode equilibration buffer, a mixed mode loading buffer, or a mixed mode wash buffer, wherein the mixed mode pre-equilibration buffer, the mixed mode equilibration buffer, the mixed mode loading buffer, and/or the mixed mode wash buffer is between about pH 6 and about pH 7. In some embodiments, an anion mixed mode equilibration buffer is between about pH 6.5 and about pH 8.

In some embodiments according to (or as applied to) any of the embodiments above, the second mixed mode chromatography uses one or more of a mixed mode pre-equilibration buffer, a mixed mode equilibration buffer, a mixed mode loading buffer or a mixed mode wash buffer wherein the mixed mode pre-equilibration buffer, the mixed mode equilibration buffer, and/or mixed mode wash buffer is between about pH 5 and about pH 8, optionally between about pH 5 and about pH 7, or between about pH 5 and about pH 6, or between about pH 6 and about pH 7.

In some embodiments according to (or as applied to) any of the embodiments above, the mixed mode pre-equilibration buffer, the mixed mode equilibration buffer, and/or mixed mode wash buffer is about pH 5.5. In some embodiments according to (or as applied to) any of the embodiments above, the mixed mode pre-equilibration buffer comprises about 500 mM acetate. In some embodiments according to (or as applied to) any of the embodiments above, the mixed mode equilibration buffer comprises about 50 mM acetate.

In some embodiments according to (or as applied to) any of the embodiments above, the first mixed mode chromatography is washed with wash buffer following load. In some embodiments according to (or as applied to) any of the embodiments above, the second mixed mode chromatography is washed with wash buffer following load. In some embodiments according to (or as applied to) any of the embodiments above, the multispecific antibody is eluted from the first mixed mode chromatography by salt gradient and/or pH gradient or pH step elution. In some embodiments according to (or as applied to) any of the embodiments above, the multispecific antibody is eluted from the first mixed mode chromatography by applying a mixed mode elution buffer with low pH to the mixed mode exchange chromatography. In some embodiments according to (or as applied to) any of the embodiments above, the multispecific antibody is eluted from the second mixed mode chromatography by salt gradient and/or pH gradient or pH step elution. In some embodiments according to (or as applied to) any of the embodiments above, the multispecific antibody is eluted from the second mixed mode chromatography by applying a mixed mode elution buffer with low pH to the mixed mode exchange chromatography. In some embodiments according to (or as applied to) any of the embodiments above, the mixed mode elution buffer comprises about 25 mM acetate, about pH 5.5.

In some embodiments according to (or as applied to) any of the embodiments above, the anion exchange chromatography comprises a quaternary amine. In some embodiments according to (or as applied to) any of the embodiments above, the anion exchange chromatography comprises a quaternary amine linked to crosslinked agarose. In some embodiments according to (or as applied to) any of the embodiments above, the anion exchange chromatography is a QSFF chromatography. In some embodiments according to (or as applied to) any of the embodiments above, the anion exchange chromatography uses one or more of an anion exchange pre-equilibration buffer, an anion exchange equilibration buffer or an anion exchange loading buffer wherein the anion exchange pre-equilibration buffer, the anion exchange equilibration buffer and/or anion exchange the load buffer is between about pH 8 and about pH 9. In some embodiments according to (or as applied to) any of the embodiments above, the anion exchange pre-equilibration buffer, the anion exchange equilibration buffer and/or the anion exchange load buffer is about pH 8.5. In some embodiments according to (or as applied to) any of the embodiments above, the anion exchange pre-equilibration buffer comprises about 50 mM Tris, 500 mM sodium acetate. In some embodiments according to (or as applied to) any of the embodiments above, the anion exchange equilibration buffer comprises about 50 mM Tris. In some embodiments according to (or as applied to) any of the embodiments above, the anion exchange chromatography is washed with anion exchange equilibration buffer following load. In some embodiments according to (or as applied to) any of the embodiments above, the multispecific antibody is eluted from the anion exchange chromatography by salt gradient. In some embodiments according to (or as applied to) any of the embodiments above, the multispecific antibody is eluted from the anion exchange chromatography by applying an anion exchange elution buffer with increased salt concentration to the anion exchange chromatography. In some embodiments according to (or as applied to) any of the embodiments above, the anion exchange elution buffer comprises about 50 mM Tris, 100 mM sodium acetate at about pH 8.5.

In some embodiments according to (or as applied to) any of the embodiments above, the arms of the multispecific antibody are produced in a cell. In some embodiments according to (or as applied to) any of the embodiments above, the cell is a prokaryotic cell. In some embodiments according to (or as applied to) any of the embodiments above, the prokaryotic cell is an *E. coli* cell. In some embodiments according to (or as applied to) any of the embodiments above, the cell is engineered to express one or more chaperones. In some embodiments according to (or as applied to) any of the embodiments above, the chaperone is one or more of FkpA, DsbA or DsbC. In some embodiments according to (or as applied to) any of the embodiments above, the chaperone is an *E. coli* chaperone. In some embodiments according to (or as applied to) the embodiments above, the cell is a eukaryotic cell. In some embodiments according to (or as applied to) the embodiments above, the eukaryotic cell is a yeast cell, an insect cell, or a mammalian cell. In some embodiments according to (or as applied to) the embodiments above, the eukaryotic cell is a CHO cell.

In some embodiments according to (or as applied to) any of the embodiments above, the cells are lysed to generate a cell lysate comprising the multispecific antibody or an arm of the multispecific antibody prior to capture chromatography. In some embodiments according to (or as applied to) any of the embodiments above, the cells are lysed using a microfluidizer. In some embodiments according to (or as applied to) any of the embodiments above, polyethlyeneimine (PEI) is added to the cell lysate prior to chromatography. In some embodiments according to (or as applied to) any of the embodiments above, the PEI is added to the lysate to a final concentration of about 0.4%. In some embodiments according to (or as applied to) any of the embodiments above, the cell lysate is clarified by centrifugation. In some embodiments according to (or as applied to) any of the embodiments above, the cell lysate from a mammalian cell, *e.g.,* a CHO cell, is subject to one or more the following treatments: heat inactivation, low pH inactivation, viral inactivation by addition of detergent.

In some embodiments according to (or as applied to) any of the embodiments above, the method reduces the amount of a process-specific impurity such as any one of host cell protein (HCP), leached protein A, nucleic acid, cell culture media components, or viral impurities in the composition.

In some embodiments according to (or as applied to) any of the embodiments above, the multispecific antibody is a bispecific antibody. In some embodiments according to (or as applied to) any of the embodiments above, the bispecific antibody is a knob-in-hole (KiH) antibody, *e.g*., a KiH bispecific antibody. In some embodiments according to (or as applied to) any of the embodiments above, the bispecific antibody is a CrossMab bispecific antibody.

In some embodiments according to (or as applied to) any of the methods above, a fraction is collected after the last chromatography step, comprising at least about 95%, at least about 96%, at least about 97%, at least about 98%, at least about 99%, or about 100% multispecific antibody. In some embodiments according to (or as applied to) any of the embodiments above, a fraction is collected after the last chromatography step, comprising a reduced amount of a product-specific impurity, wherein the product-specific impurity is one or more of: non-paired antibody arms, antibody homodimers, high molecular weight species (HMWS), low molecular weight species (LMWS), or ¾ antibodies. In some embodiments according to (or as applied to) any of the embodiments above, the fraction contains less than about 5%, less than about 4%, less than about 3%, less than about 2%, or less than about 1% non-paired antibody arms. In some embodiments according to (or as applied to) any of the embodiments above, the fraction contains less than about 5%, less than about 4%, less than about 3%, less than about 2%, or less than about 1% antibody homodimers. In some embodiments according to (or as applied to) any of the embodiments above, the fraction contains no more than about 1% or no more than about 2% HMWS. In some embodiments according to (or as applied to) any of the embodiments above, the fraction contains no more than about 2% or no more than about 1% LMWS. In some embodiments according to (or as applied to) any of the embodiments above, the fraction contains no more than about 5%, no more than about 4%, no more than about 3%, no more than about 2%, or no more than about 1% of ¾ antibodies.

In some embodiments according to (or as applied to) any of the embodiments above, the fraction contains

a) at least about 95% - 100% multispecific antibody;
b) less than about 1%- 5% non-paired antibody arms;
c) less than about 1%- 5% antibody homodimers;
d) no more than about 1% or 2% HMWS;
e) no more than about 1% or 2% LMWS; and/or
f) no more than about 5% of ¾ antibodies.

In certain embodiments, provided is a composition comprising a multispecific antibody purified by the method of any one of the methods described above.

In some embodiments according to (or as applied to) any of the methods above, provided is a composition comprising a multispecific antibody, wherein the composition comprises at least about 95%, at least about 96%, at least about 97%, at least about 98%, at least about 99%, or about 100% multispecific antibody. In some embodiments according to (or as applied to) any of the embodiments above, provided is a composition comprising a multispecific antibody, wherein the composition comprises a reduced amount of a product-specific impurity, wherein the product-specific impurity is one or more of: non-paired antibody arms, antibody homodimers, high molecular weight species (HMWS), low molecular weight species (LMWS), or ¾ antibodies. In some embodiments according to (or as applied to) any of the embodiments above, the composition less than about 5%, less than about 4%, less than about 3%, less than about 2%, or less than about 1% non-paired antibody arms. In some embodiments according to (or as applied to) any of the embodiments above, the composition contains less than about 5%, less than about 4%, less than about 3%, less than about 2%, or less than about 1% antibody homodimers. In some embodiments according to (or as applied to) any of the embodiments above, the composition contains no more than about 1% or no more than about 2% HMWS. In some embodiments according to (or as applied to) any of the embodiments above, the composition contains no more than about 2% or no more than about 1% LMWS. In some embodiments according to (or as applied to) any of the embodiments above, the composition contains no more than about 5%, no more than about 4%, no more than about 3%, no more than about 2%, or no more than about 1% of ¾ antibodies. In some embodiments according to (or as applied to) any of the embodiments above, the multispecific antibody in the composition is a bispecific antibody. In some embodiments according to (or as applied to) any of the embodiments above, the bispecific antibody is a knob-in-hole (KiH) antibody, *e.g.,* a KiH bispecific antibody. In some embodiments according to (or as applied to) any of the embodiments above, the bispecific antibody is a CrossMab bispecific antibody. In some embodiments according to (or as applied to) any of the embodiments above, the bispecific antibody binds to ANG-2 and VEGF. In some embodiments according to (or as applied to) any of the embodiments above, the bispecific antibody binds to ANG-2 and VEGF comprises a) the heavy chain and the light chain of a first full length antibody that comprises the first antigen-binding site; and b) the modified heavy chain and modified light chain of a full length antibody that comprises the second antigen-binding site, wherein the constant domains CL and CH1 are replaced by each other.

In some embodiments according to (or as applied to) any of the embodiments above, the composition contains: a) at least about 95% - 100% multispecific antibody; b) less than about 1%- 5% non-paired antibody arms; c) less than about 1%- 5% antibody homodimers; d) no more than about 1% or 2% HMWS; e) no more than about 1% or 2% LMWS; and/or f) no more than about 5% of ¾ antibodies. In some embodiments according to (or as applied to) any of the embodiments above, the multispecific antibody in the composition is a bispecific antibody. In some embodiments according to (or as applied to) any of the embodiments above, the bispecific antibody is a knob-in-hole (KiH) antibody, *e.g.,* a KiH bispecific antibody. In some embodiments according to (or as applied to) any of the embodiments above, the bispecific antibody is a CrossMab bispecific antibody. In some embodiments according to (or as applied to) any of the embodiments above, the bispecific antibody binds to ANG-2 and VEGF. In some embodiments according to (or as applied to) any of the embodiments above, the bispecific antibody binds to ANG-2 and VEGF comprises a) the heavy chain and the light chain of a first full length antibody that comprises the first antigen-binding site; and b) the modified heavy chain and modified light chain of a full length antibody that comprises the second antigen-binding site, wherein the constant domains CL and CH1 are replaced by each other.

In some embodiments according to (or as applied to) any of the embodiments above, provided is a composition comprising a multispecific or bispecific antibody (such as a bispecific antibody that binds ANG2 and VEGF) purified by any one of the methods described above for the treatment of cancer or eye disease.

In some embodiments according to (or as applied to) any of the embodiments above, provided is the use of a comprising a multispecific or bispecific antibody (such as a bispecific antibody that binds ANG2 and VEGF) purified by any one of the methods described above for the manufacture of a medicament for the treatment of cancer or eye disease.

In some embodiments according to (or as applied to) any of the embodiments above, the methods provided herein are used for the purification of an Fc-containing heterodimeric polypeptide.

In some embodiments according to (or as applied to) any of the embodiments above, provided is the use of the any of the methods provided herein for the reduction of Fc-containing heterodimeric polypeptide-related impurities in a composition.

In certain embodiments, provided is a method for purifying an Fc-region containing heterodimeric polypeptide with a multi-step chromatography method wherein the method comprises an affinity chromatography step followed by two different multimodal ion exchange chromatography steps, and thereby purifying the Fc-region containing heterodimeric polypeptide. In some embodiments according to (or as applied to) any of the embodiments above, the multi-step chromatography method comprises (i) an affinity chromatography step, followed by a multimodal anion exchange chromatography step, followed by a multimodal cation exchange chromatography step; or (ii) an affinity chromatography step, followed by a multimodal cation exchange chromatography step, followed by a multimodal anion exchange chromatography step.

In some embodiments according to (or as applied to) any of the embodiments above, the multi-step chromatography method comprises an affinity chromatography step, followed by a multimodal anion exchange chromatography step, followed by a multimodal cation exchange chromatography step. In some embodiments according to (or as applied to) any of the embodiments above, the multi-step chromatography method comprises exactly three chromatography steps. In some embodiments according to (or as applied to) any of the embodiments above, the multimodal anion exchange chromatography step is performed in flow-through mode. In some embodiments according to (or as applied to) any of the embodiments above, the multimodal anion exchange chromatography step the Fc-region containing heterodimeric polypeptide is applied in a solution with a conductivity value of less than 7 mS/cm. In some embodiments according to (or as applied to) any of the embodiments above, in the multimodal anion exchange chromatography step the Fc-region containing heterodimeric polypeptide is applied in a solution with a conductivity value in the range of about 6 mS/cm to about 2 mS/cm. In some embodiments according to (or as applied to) any of the embodiments above, in the multimodal anion exchange chromatography step the Fc-region containing heterodimeric polypeptide is applied in a solution with a conductivity value of about 4.5 mS/cm. In some embodiments according to (or as applied to) any of the embodiments above, the multimodal anion exchange chromatography step is performed at a pH of about 7. In some embodiments according to (or as applied to) any of the embodiments above, in the multimodal anion exchange chromatography step the Fc-region containing heterodimeric polypeptide is applied in a solution with a conductivity of about 4.5 mS/cm and a pH of about 7. In some embodiments according to (or as applied to) any of the embodiments above, in the multimodal anion exchange chromatography step the Fc-region containing heterodimeric polypeptide is applied in the range of from about 100 g to about 300 g per liter of chromatography material.

In some embodiments according to (or as applied to) any of the embodiments above, the multimodal anion exchange chromatography material is a multimodal strong anion exchange chromatography material. In some embodiments according to (or as applied to) any of the embodiments above, the multimodal anion exchange chromatography material has a matrix of high-flow agarose, a multimodal strong anion exchanger as ligand, an average particle size of 36-44 $\mu$m and an ionic capacity of 0.08 to 0.11 mmol Cl-/mL medium. In some embodiments according to (or as applied to) any of the embodiments above, the multimodal cation exchange chromatography medium is a multimodal weak cation exchange chromatography medium. In some embodiments according to (or as applied to) any of the embodiments above, the multimodal cation exchange chromatography medium has a matrix of high-flow agarose, a multimodal weak cation exchanger as ligand, an average particle size of 36-44 $\mu$m and ionic capacity of 25 to 39 $\mu$mol/mL. In some embodiments according to (or as applied to) any of the embodiments above, the multimodal anion exchange chromatography step is performed in bind and elute mode. In some embodiments according to (or as applied to) any of the embodiments above, the capture chromatography is carried out by affinity chromatography. In some embodiments, the affinity chromatography is a protein A affinity chromatography or a Protein G affinity chromatography or a single chain Fv ligand affinity chromatography or a chromatography step with CaptureSelect chromatography material or a chromatography step with CaptureSelect FcXL chromatography material. In some embodiments according to (or as applied to) any of the embodiments above, the affinity chromatography step is a protein A chromatography step. In some embodiments according to (or as applied to) any of the embodiments above, the affinity chromatography step is a chromatography step with CaptureSelect™ chromatography material.

In some embodiments according to (or as applied to) any of the embodiments above, the Fc-region containing heterodimeric polypeptide is an antibody, a bispecific antibody or Fc-fusion proteins. In some embodiments according to (or as applied to) any of the embodiments above, the Fc-region containing heterodimeric polypeptide is a bispecific antibody. In some embodiments according to (or as applied to) any of the embodiments above, the Fc-region containing heterodimeric polypeptide is a CrossMab. In some embodiments according to (or as applied to) any of the embodiments above, the Fc-region containing heterodimeric polypeptide is a bispecific antibody comprising a) a heavy chain and a light chain of a first full length antibody that specifically binds to a first antigen; and b) a modified heavy chain and a modified light chain of a full length antibody that specifically binds to a second antigen, wherein the constant domains CL and CH1 are replaced by each other.

In some embodiments according to (or as applied to) any of the embodiments above, the bispecific antibody binds to ANG2 and VEGF. In some embodiments according to (or as applied to) any of the embodiments above, the CrossMab binds to ANG2 and VEGF. In some embodiments according to (or as applied to) any of the embodiments above, the bispecific antibody is vanucizumab. In some embodiments according to (or as applied to) any of the embodiments above, the bispecific antibody comprises a first antigen-binding site that comprises as heavy chain variable domain (VH) the SEQ ID NO: 1, and as light chain variable domain (VL) the SEQ ID NO: 2; and a second antigen-binding site that comprises as heavy chain variable domain (VH) the SEQ ID NO: 3, and as light chain variable domain (VL) the SEQ ID NO: 4. In some embodiments according to (or as applied to) any of the embodiments above, the bispecific antibody comprises a first heavy chain with the amino acid sequence of SEQ ID NO: 9 and a second heavy chain with the amino acid sequence of SEQ ID NO: 10 and a first light chain with the amino acid sequence of SEQ ID NO: 11 and a second light chain with the amino acid sequence of SEQ ID NO: 12.

In some embodiments according to (or as applied to) any of the embodiments above, the bispecific antibody comprises a first antigen-binding site that comprises as heavy chain variable domain (VH) the SEQ ID NO: 5, and as light chain variable domain (VL) the SEQ ID NO: 6; and a second antigen-binding site that comprises as heavy chain variable domain (VH) the SEQ ID NO: 7, and as light chain variable domain (VL) the SEQ ID NO: 8. In some embodiments according to (or as applied to) any of the embodiments above, the bispecific antibody comprises a first heavy chain with the amino acid sequence of SEQ ID NO: 13 and a second heavy chain with the amino acid sequence of SEQ ID NO: 14 and a first light chain with the amino acid sequence of SEQ ID NO: 15 and a second light chain with the amino acid sequence of SEQ ID NO: 16.

In some embodiments according to (or as applied to) any of the embodiments above, the purified Fc-region containing heterodimeric polypeptide contains no more than about 5% of ¾ antibodies.

In certain embodiments, provided is a method for purifying a bispecific antibody that binds to ANG2 and VEGF with a multi-step chromatography method wherein the method comprises an affinity chromatography step, followed by a multimodal anion exchange chromatography step, followed by a multimodal cation exchange chromatography step, and thereby purifying the bispecific antibody that binds to ANG2 and VEGF, wherein bispecific antibody that binds to ANG2 and VEGF comprises a first antigen-binding site that comprises as heavy chain variable domain (VH) the SEQ ID NO: 1, and as light chain variable domain (VL) the SEQ ID NO: 2; and a second antigen-binding site that comprises as heavy chain variable domain (VH) the SEQ ID NO: 3, and as light chain variable domain (VL) the SEQ ID NO: 4 or that comprises a first antigen-binding site that comprises as heavy chain variable domain (VH) the SEQ ID NO: 5, and as light chain variable domain (VL) the SEQ ID NO: 6; and a second antigen-binding site that comprises as heavy chain variable domain (VH) the SEQ ID NO: 7, and as light chain variable domain (VL) the SEQ ID NO: 8. In some embodiments according to (or as applied to) any of the embodiments above, the bispecific antibody that binds to ANG2 and VEGF comprises a) the heavy chain and the light chain of a first full length antibody that comprises the first antigen-binding site; and b) the modified heavy chain and modified light chain of a full length antibody that comprises the second antigen-binding site, wherein the constant domains CL and CH1 are replaced by each other.

In some embodiments, provided is the use of any a method according to (or as applied to) any of the embodiments above for the reduction of Fc-containing heterodimeric polypeptide related impurities.

In some embodiments, provided is an Fc-containing heterodimeric polypeptide obtained with the method according to (or as applied to) any of the embodiments above for the manufacture of a medicament for the treatment of cancer or eye disease.

In some embodiments, provided is an Fc-containing heterodimeric polypeptide obtained from the method according to (or as applied to) any of the embodiments above for use in the treatment of cancer or eye disease.

In certain embodiments, provided is a method for producing an Fc-containing heterodimeric polypeptide comprising the steps of (i) cultivating a cell comprising a nucleic acid encoding an Fc-containing heterodimeric polypeptide; (ii) recovering the Fc-containing heterodimeric protein from the cell or the cultivation medium; (iii) purifying the Fc-containing heterodimeric polypeptide using a method according to (or as applied to) any of the embodiments above, and thereby producing the Fc-containing heterodimeric polypeptide.

In certain embodiments, provided is a method of producing a bispecific antibody that binds to ANG-2 and VEGF comprising the steps of: (i) cultivating a cell comprising a nucleic acid encoding the bispecific antibody; (ii) recovering the bispecific antibody from the cell or the cultivation medium; (iii) purifying the bispecific antibody using a method according to (or as applied to) any of the embodiments above, and thereby producing the bispecific antibody that binds to ANG-2 and VEGF.

BRIEF DESCRIPTION OF THE DRAWINGS

**FIG. 1A** depicts a first purification scheme used in Example 1.

**FIG. 1B** depicts a second purification scheme used in Example 1.

**FIG. 1C** depicts a third purification scheme used in Example 1.

**FIG. 2** depicts a purification scheme used in Example 2.

**FIG. 3A** depicts a first purification scheme used in Example 3.

**FIG. 3B** depicts a second purification scheme used in Example 3.

**FIG. 4** depicts a purification scheme used in Example 4.

**FIG. 5A** depicts a first purification scheme used in Example 6.

**FIG. 5B** depicts a second purification scheme used in Example 6.

**FIG. 6A** depicts a first purification scheme used in Example 7.

**FIG. 6B** depicts a second purification scheme used in Example 7.

**FIG. 7A** depicts a first purification scheme described in Example 8.

**FIG. 7B** depicts a second purification scheme described in Example 8.

**FIG. 7C** depicts a third purification scheme used described in Example 8.

DETAILED DESCRIPTION OF THE INVENTION

Provided herein are methods for purifying a multispecific antibody (such as a bispecific antibody or a divalent F(ab')$_2$) comprising the sequential steps of subjecting a composition comprising the multispecific antibody to a) capture chromatography, b) a first mixed mode chromatography, and c) a second mixed mode chromatography. In some aspects, provided are methods for purifying a multispecific antibody wherein individual arms of the multispecific antibody are each produced in separate cultures and each separately purified by capture chromatography. The purified antibody arms are then assembled to produce the multispecific antibody. The assembled multispecific antibody is then subjected to a first mixed mode anion exchange chromatography followed by a second mixed mode chromatography. As described in further detail below, each of the capture chromatography, first mixed mode chromatography, and/or second mixed mode chromatography are optionally preceded and/or followed by one or more additional chromatography steps. The terms mixed mode chromatography and multimodal chromatography are used interchangeably herein.

In some aspects, provided are compositions comprising multispecific antibodies that have reduced levels of one or more process specific and/or product specific impurities, such as unpaired antibody arms, homodimers, aggregates, low molecular weight species, acidic and basic variants. In some aspects, provided are compositions comprising multispecific antibodies that have reduced levels of one or more process specific impurities, such as, *e.g.*, prokaryotic host cell protein, eukaryotic host cell protein (such as CHO proteins or "CHOP"), nucleic acid, and chaperones (such prokaryotic chaperones, *e.g.,* FkpA, DsbA and DsbC). In certain embodiments, the compositions provided herein are obtained using a method provided herein. In certain embodiments, the compositions provided herein have reduced levels of one or more process specific and/or product specific impurities than compositions obtained using methods known in the art.

In some aspects provided are uses of the methods as reported herein for the purification of an Fc-containing heterodimeric polypeptide and for the reduction of Fc-containing heterodimeric polypeptide-related impurities. An improved reduction of product-specific impurities is achieved. In the case of CrossMab-specific impurities, a reduction of *e.g.* ¾ antibodies is achieved.

*Definitions*

The terms "polypeptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc.),* as well as other modifications known in the art. The terms "polypeptide" and "protein" as used herein specifically encompass antibodies.

"Purified" polypeptide (*e.g.*, antibody or immunoadhesin) means that the polypeptide has been increased in purity, such that it exists in a form that is more pure than it exists in its natural environment and/or when initially synthesized and/or amplified under laboratory conditions. Purity is a relative term and does not necessarily mean absolute purity. The terms "purifying," "separating," or "isolating," as used interchangeably herein, refer to increasing the degree of purity of a desired molecule (such as a multispecific antibody, *e.g.*, a bispecific antibody) from a composition or sample comprising the desired molecule and one or more impurities. Typically, the degree of purity of the desired molecule is increased by removing (completely or partially) at least one impurity from the composition.

A multispecific antibody "which binds an antigen of interest" is one that binds the antigen, *e.g.,* a protein, with sufficient affinity such that the multispecific antibody is useful as a diagnostic and/or therapeutic agent in targeting a protein or a cell or tissue expressing the protein, and does not significantly cross-react with other proteins. In such embodiments, the extent of binding of the multispecific antibody to a "non-target" protein will be less than about 10% of the binding of the multispecific antibody to its particular target protein as determined by, *e.g.*, fluorescence activated cell sorting (FACS) analysis, radioimmunoprecipitation (RIA), or ELISA, etc. With regard to the binding of a multispecific antibody to a target molecule, the term "specific binding" or "specifically binds to" or is "specific for" a particular polypeptide or an epitope on a particular polypeptide target means binding that is measurably different from a nonspecific interaction (*e.g.*, a non-specific interaction may be binding to bovine serum albumin or casein). Specific binding can be measured, for example, by determining binding of a molecule compared to binding of a control molecule. For example, specific binding can be determined by competition with a control molecule that is similar to the target, for example, an excess of non-labeled target. In this case, specific binding is indicated if the binding of the labeled target to a probe is competitively inhibited by excess unlabeled target. The term "specific binding" or "specifically binds to" or is "specific for" a particular polypeptide or an epitope on a particular polypeptide target as used herein can be exhibited, for example, by a molecule having a Kd for the target of at least about 200 nM, alternatively at least about 150 nM, alternatively at least about 100 nM, alternatively at least about 60 nM, alternatively at least about 50 nM, alternatively at least about 40 nM, alternatively at least about 30 nM, alternatively at least about 20 nM, alternatively at least about 10 nM, alternatively at least about 8 nM, alternatively at least about 6 nM, alternatively at least about 4 nM, alternatively at least about 2 nM, alternatively at least about 1 nM, or greater affinity. In one embodiment, the term "specific binding" refers to binding where a multispecific antigen-binding protein binds to a particular polypeptide or epitope on a particular polypeptide without substantially binding to any other polypeptide or polypeptide epitope.

"Binding affinity" generally refers to the strength of the sum total of noncovalent interactions between a single binding site of a molecule (*e.g.,* a multispecific antibody) and its binding partner (*e.g.,* an antigen). Unless indicated otherwise, as used herein, "binding affinity" refers to intrinsic binding affinity which reflects a 1:1 interaction between members of a binding pair (*e.g.,* antibody and antigen). The affinity of a molecule X for its partner Y can generally be represented by the dissociation constant (Kd). For example, the Kd can be about 200 nM or less, about 150 nM or less, about 100 nM or less, about 60 nM or less, about 50 nM or less, about 40 nM or less, about 30 nM or less, about 20 nM or less, about 10 nM or less, about 8 nM or less, about 6 nM or less, about 4 nM or less, about 2 nM or less, or about 1 nM or less. Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the methods and compositions provided herein.

In one embodiment, the "Kd" or "Kd value" according to this invention is measured by using surface plasmon resonance assays using a BIAcore™-2000 or a BIAcore™-3000 (BIAcore, Inc., Piscataway, NJ) at 25°C with immobilized target (*e.g.*, antigen) CM5 chips at -10 response units (RU). Briefly, carboxymethylated dextran biosensor chips (CM5, BIAcore Inc.) are activated with N-ethyi-N'-(3- dimethylaminopropyl)-carbodiimide hydrochloride (EDC) and N-hydroxysuccinimide (NHS) according to the supplier's instructions. Antigen is diluted with 10 mM sodium acetate, pH 4.8, into $5\mu g/ml$ ($\sim 0.2\mu M$) before injection at a flow rate of 5 $\mu l$/minute to achieve approximately 10 response units (RU) of coupled protein. Following the injection of antigen, 1M ethanolamine is injected to block unreacted groups. For kinetics measurements, two-fold serial dilutions of Fab (*e.g.,* 0.78 nM to 500 nM) are injected in PBS with 0.05% Tween 20 (PBST) at 25°C at a flow rate of approximately 25 $\mu l$/min. Association rates ($k_{on}$) and dissociation rates ($k_{off}$) are calculated using a simple one-to-one Langmuir binding model (BIAcore Evaluation Software version 3.2) by simultaneous fitting the association and dissociation sensorgram. The equilibrium dissociation constant (Kd) is calculated as the ratio $k_{off}/k_{on}$. See, *e.g.,* Chen et al., J. Mol. Biol. 293:865-881 (1999). If the on-rate exceeds $10^6 M^{-1} s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-Aminco spectrophotometer (ThermoSpectronic) with a stirred cuvette.

"Active" or "activity" for the purposes herein refers to form(s) of a polypeptide (such as a multispecific antibody) which retain a biological and/or an immunological activity of native or naturally-occurring polypeptide, wherein "biological" activity refers to a biological function (either inhibitory or stimulatory) caused by a native or naturally-occurring polypeptide other than the ability to induce the production of an antibody against an antigenic epitope possessed by a native or naturally-occurring polypeptide and an "immunological" activity refers to the ability to induce the production of an antibody against an antigenic epitope possessed by a native or naturally-occurring polypeptide.

"Biologically active" and "biological activity" and "biological characteristics" with respect to a multispecific antigen-binding protein provided herein, such as an antibody, fragment, or derivative thereof, means having the ability to bind to a biological molecule, except where specified otherwise.

The term "antibody" herein is used in the broadest sense and specifically covers monoclonal antibodies, polyclonal antibodies, multispecific antibodies (*e.g.* bispecific antibodies) formed from at least two intact antibodies, and antibody fragments so long as they exhibit the desired biological activity. The term "immunoglobulin" (Ig) is used interchangeable with antibody herein.

Antibodies are naturally occurring immunoglobulin molecules which have varying structures, all based upon the immunoglobulin fold. For example, IgG antibodies have two "heavy" chains and two "light" chains that are disulfide-bonded to form a functional antibody. Each heavy and light chain itself comprises a "constant" (C) and a "variable" (V) region. The V regions determine the antigen binding specificity of the antibody, whilst the C regions provide structural support and function in non-antigen-specific interactions with immune effectors. The antigen binding specificity of an antibody or antigen-binding fragment of an antibody is the ability of an antibody to specifically bind to a particular antigen.

The antigen binding specificity of an antibody is determined by the structural characteristics of the V region. The variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (*see* Kabat et al., Sequences of Proteins of Immunological Interest, 5th Ed. Public Health Service, National Institutes of Health, Bethesda, Md. (1991)). The constant domains are not involved directly in binding an antibody to an antigen, but exhibit various effector functions, such as participation of the antibody in antibody dependent cellular cytotoxicity (ADCC).

Each V region typically comprises three complementarity determining regions ("CDRs", each of which contains a "hypervariable loop"), and four framework regions. An antibody binding site, the minimal structural unit required to bind with substantial affinity to a particular desired antigen, will therefore typically include the three CDRs, and at least three, preferably four, framework regions interspersed there between to hold and present the CDRs in the appropriate conformation. Classical four chain antibodies have antigen binding sites which are defined by $V_H$ and $V_L$ domains in cooperation. Certain antibodies, such as camel and shark antibodies, lack light chains and rely on binding sites formed by heavy chains only. Single domain engineered immunoglobulins can be prepared in which the binding sites are formed by heavy chains or light chains alone, in absence of cooperation between $V_H$ and $V_L$.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (*see* Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody that are responsible for antigen binding. The hypervariable region may comprise amino acid residues from a "complementarity determining region" or "CDR" (*e.g.*, around about residues 24-34 (L1), 50-56 (L2) and 89-97 (L3) in the $V_L$, and around about 31-35B (H1), 50-65 (H2) and 95-102 (H3) in the $V_H$ (Kabat et al., Sequences of Proteins of Immunological Interest, 5th Ed. Public Health Service, National Institutes of Health, Bethesda, Md. (1991)) and/or those residues from a "hypervariable loop" (*e.g.* residues 26-32 (L1), 50-52 (L2) and 91-96 (L3) in the $V_L$, and 26-32 (H1), 52A-55 (H2) and 96-101 (H3) in the $V_H$ (Chothia and Lesk J. Mol. Biol. 196:901-917 (1987)).

"Framework" or "FR" residues are those variable domain residues other than the hypervariable region residues as herein defined.

"Hinge region" in the context of an antibody or half-antibody is generally defined as stretching from Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "lower hinge region" of an Fc region is normally defined as the stretch of residues immediately C-terminal to the hinge region, *i.e.* residues 233 to 239 of the Fc region. Prior to the present application, FcγR binding was generally attributed to amino acid residues in the lower hinge region of an IgG Fc region.

The "CH2 domain" of a human IgG Fc region usually extends from about residues 231 to about 340 of the IgG. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec. Immunol.22:161-206 (1985).

The "CH3 domain" comprises the stretch of residues C-terminal to a CH2 domain in an Fc region (i.e. from about amino acid residue 341 to about amino acid residue 447 of an IgG).

"Antibody fragments" comprise a portion of an intact antibody, preferably comprising the antigen binding region thereof. Examples of antibody fragments include Fab, Fab', $F(ab')_2$, and Fv fragments; diabodies; tandem diabodies (taDb), linear antibodies(e.g., U.S. Patent No. 5,641,870, Example 2; Zapata et al., Protein Eng. 8(10):1057-1062 (1995)); one-armed antibodies, single variable domain antibodies, minibodies, single-chain antibody molecules; multispecific antibodies formed from antibody fragments (e.g., including but not limited to, Db-Fc, taDb-Fc, taDb-CH3, (scFV)4-Fc, di-scFv, bi-scFv, or tandem (di,tri)-scFv); and Bi-specific T-cell engagers (BiTEs).

Papain digestion of antibodies produces two identical antigen-binding fragments, called "Fab" fragments, and a residual "Fc" fragment, a designation reflecting the ability to crystallize readily. The Fab fiagment consists of an entire L chain along with the variable region domain of the H chain (VH), and the first constant domain of one heavy chain (CH1). Pepsin treatment of an antibody yields a single large F(ab')2 fragment which roughly corresponds to two disulfide linked Fab fragments having divalent antigen-binding activity and is still capable of cross-linking antigen. Fab' fragments differ from Fab fragments by having additional few residues at the carboxy terminus of the CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

"Fv" is the minimum antibody fragment that contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the $V_H$-$V_L$ dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. $F(ab')_2$ antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa ($\kappa$) and lambda ($\lambda$), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), e.g., IgG1, IgG2, IgG3, IgG4, IgA, and IgA2. The heavy chain constant domains that correspond to the different classes of antibodies are called $\alpha$, $\delta$, $\epsilon$, $\gamma$, and $\mu$, respectively. The subunit structures and three-dimensional configurations of different classes of immunoglobulins are well known.

"Single-chain Fv" or "scFv" antibody fragments comprise the $V_H$ and $V_L$ domains of antibody, wherein these domains are present in a single polypeptide chain. In some embodiments, the Fv polypeptide further comprises a polypeptide linker between the $V_H$ and $V_L$ domains that enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy chain variable domain ($V_H$) connected to a light chain variable domain ($V_L$) in the same polypeptide chain ($V_H$ - $V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The term "half-antibody" or "hemimer" as used herein refers to a monovalent antigen binding polypeptide. In certain embodiments, a half antibody or hemimer comprises a VH/VL unit and optionally at least a portion of an immunoglobulin constant domain. In certain embodiments, a half antibody or hemimer comprises one immunoglobulin heavy chain associated with one immunoglobulin light chain, or an antigen binding fragment thereof. In certain embodiments, a half antibody or hemimer is mono-specific, i.e., binds to a single antigen or epitope. One skilled in the art will readily appreciate that a half-antibody may have an antigen binding domain consisting of a single variable domain, e.g.,

originating from a camelidae.

The term "VH/VL unit" refers to the antigen-binding region of an antibody that comprises at least one VH HVR and at least one VL HVR. In certain embodiments, the VH/VL unit comprises at least one, at least two, or all three VH HVRs and at least one, at least two, or all three VL HVRs. In certain embodiments, the VH/VL unit further comprises at least a portion of a framework region (FR). In some embodiments, a VH/VL unit comprises three VH HVRs and three VL HVRs. In some such embodiments, a VH/VL unit comprises at least one, at least two, at least three or all four VH FRs and at least one, at least two, at least three or all four VL FRs.

The term "multispecific antibody" is used in the broadest sense and specifically covers an antibody comprising an antigen-binding domain that has polyepitopic specificity (*i.e.,* is capable of specifically binding to two, or more, different epitopes on one biological molecule or is capable of specifically binding to epitopes on two, or more, different biological molecules). In some embodiments, an antigen-binding domain of a multispecific antibody (such as a bispecific antibody or a divalent F(ab')$_2$) comprises two VH/VL units, wherein a first VH/VL unit specifically binds to a first epitope and a second VH/VL unit specifically binds to a second epitope, wherein each VH/VL unit comprises a heavy chain variable domain (VH) and a light chain variable domain (VL). Such multispecific antibodies include, but are not limited to, full length antibodies, antibodies having two or more VL and VH domains, antibody fragments such as Fab, Fv, dsFv, scFv, diabodies, bispecific diabodies and triabodies, antibody fragments that have been linked covalently or non-covalently. A VH/VL unit that further comprises at least a portion of a heavy chain constant region and/or at least a portion of a light chain constant region may also be referred to as a "hemimer" or "half antibody." In some embodiments, a half antibody comprises at least a portion of a single heavy chain variable region and at least a portion of a single light chain variable region. In some such embodiments, a bispecific antibody that comprises two half antibodies and binds to two antigens comprises a first half antibody that binds to the first antigen or first epitope but not to the second antigen or second epitope and a second half antibody that binds to the second antigen or second epitope and not to the first antigen or first epitope. According to some embodiments, the multispecific antibody is an IgG antibody that binds to each antigen or epitope with an affinity of 5 M to 0.001 pM, 3 M to 0.001 pM, 1 M to 0.001 pM, 0.5 M to 0.001 pM, or 0.1 M to 0.001 pM. In some embodiments, a hemimer comprises a sufficient portion of a heavy chain variable region to allow intramolecular disulfide bonds to be formed with a second hemimer. In some embodiments, a hemimer comprises a knob mutation or a hole mutation, for example, to allow heterodimerization with a second hemimer or half antibody that comprises a complementary hole mutation or knob mutation. Knob mutations and hole mutations are discussed further below.

A "bispecific antibody" is a multispecific antibody comprising an antigen-binding domain that is capable of specifically binding to two different epitopes on one biological molecule or is capable of specifically binding to epitopes on two different biological molecules. A bispecific antibody may also be referred to herein as having "dual specificity" or as being "dual specific." Unless otherwise indicated, the order in which the antigens bound by a bispecific antibody are listed in a bispecific antibody name is arbitrary. In some embodiments, a bispecific antibody comprises two half antibodies, wherein each half antibody comprises a single heavy chain variable region and optionally at least a portion of a heavy chain constant region, and a single light chain variable region and optionally at least a portion of a light chain constant region. In certain embodiments, a bispecific antibody comprises two half antibodies, wherein each half antibody comprises a single heavy chain variable region and a single light chain variable region and does not comprise more than one single heavy chain variable region and does not comprise more than one single light chain variable region. In some embodiments, a bispecific antibody comprises two half antibodies, wherein each half antibody comprises a single heavy chain variable region and a single light chain variable region, and wherein the first half antibody binds to a first antigen and not to a second antigen and the second half antibody binds to the second antigen and not to the first antigen.

The term "knob-into-hole" or "KiH" technology as used herein refers to the technology directing the pairing of two polypeptides together in vitro or in vivo by introducing a protuberance (knob) into one polypeptide and a cavity (hole) into the other polypeptide at an interface in which they interact. For example, KiHs have been introduced in the Fc:Fc binding interfaces, CL:CH1 interfaces or VH/VL interfaces of antibodies (see, *e.g.,* US 2011/0287009, US2007/0178552, WO 96/027011, WO 98/050431, and Zhu et al., 1997, Protein Science 6:781-788). In some embodiments, KiHs drive the pairing of two different heavy chains together during the manufacture of multispecific antibodies. For example, multispecific antibodies having KiH in their Fc regions can further comprise single variable domains linked to each Fc region, or further comprise different heavy chain variable domains that pair with similar or different light chain variable domains. KiH technology can also be used to pair two different receptor extracellular domains together or any other polypeptide sequences that comprises different target recognition sequences (*e.g.*, including affibodies, peptibodies and other Fc fusions).

The term "knob mutation" as used herein refers to a mutation that introduces a protuberance (knob) into a polypeptide at an interface in which the polypeptide interacts with another polypeptide. In some embodiments, the other polypeptide has a hole mutation (see *e.g.,* US 5,731,168, US 5,807,706, US 5,821,333, US 7,695,936, US 8,216,805, each incorporated herein by reference in its entirety).

The term "hole mutation" as used herein refers to a mutation that introduces a cavity (hole) into a polypeptide at an interface in which the polypeptide interacts with another polypeptide. In some embodiments, the other polypeptide has a knob mutation (see *e.g.,* US 5,731,168, US 5,807,706, US 5,821,333, US 7,695,936, US 8,216,805, each incorporated herein by reference in its entirety).

The expression "single domain antibodies" (sdAbs) or "single variable domain (SVD) antibodies" generally refers to antibodies in which a single variable domain (VH or VL) can confer antigen binding. In other words, the single variable domain does not need to interact with another variable domain in order to recognize the target antigen. Examples of single domain antibodies include those derived from camelids (lamas and camels) and cartilaginous fish (*e.g.,* nurse sharks) and those derived from recombinant methods from humans and mouse antibodies (Nature (1989) 341:544-546; Dev Comp Immunol (2006) 30:43-56; Trend Biochem Sci (2001) 26:230-235; Trends Biotechnol (2003):21:484-490; WO 2005/035572; WO 03/035694; FEBS Lett (1994) 339:285-290; WO00/29004; WO 02/051870).

The term "monoclonal antibody" as used herein refers to an antibody obtained from a population of substantially homogeneous antibodies, *i.e.,* the individual antibodies comprising the population are identical and/or bind the same epitope, except for possible variants that may arise during production of the monoclonal antibody, such variants generally being present in minor amounts. In contrast to polyclonal antibody preparations that typically include different antibodies directed against different determinants (epitopes), each monoclonal antibody is directed against a single determinant on the antigen. In addition to their specificity, the monoclonal antibodies are advantageous in that they are uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the methods provided herein may be made by the hybridoma method first described by Kohler et al., Nature 256:495 (1975), or may be made by recombinant DNA methods (*see, e.g.,* U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA 81:6851-6855 (1984)). Chimeric antibodies of interest herein include "primatized" antibodies comprising variable domain antigen-binding sequences derived from a non-human primate (*e.g.* Old World Monkey, such as baboon, rhesus or cynomolgus monkey) and human constant region sequences (US Pat No. 5,693,780).

"Humanized" forms of non-human (*e.g.,* murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence, except for FR substitution(s) as noted above. The humanized antibody optionally also will comprise at least a portion of an immunoglobulin constant region, typically that of a human immunoglobulin. For further details, *see* Jones et al., Nature 321:522-525 (1986); Riechmann et al., Nature 332:323-329 (1988); and Presta, Curr. Op. Struct. Biol. 2:593-596 (1992).

For the purposes herein, an "intact antibody" is one comprising heavy and light variable domains as well as an Fc region. The constant domains may be native sequence constant domains (*e.g.* human native sequence constant domains) or amino acid sequence variant thereof. Preferably, the intact antibody has one or more effector functions.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

A "naked antibody" is an antibody (as herein defined) that is not conjugated to a heterologous molecule, such as a cytotoxic moiety or radiolabel.

As used herein, the term "immunoadhesin" designates molecules which combine the binding specificity of a heterologous protein (an "adhesin") with the effector functions of immunoglobulin constant domains. Structurally, the immunoadhesins comprise a fusion of an amino acid sequence with a desired binding specificity, which amino acid sequence is other than the antigen recognition and binding site of an antibody (*i.e.*, is "heterologous" compared to a constant region of an antibody), and an immunoglobulin constant domain sequence (*e.g.*, CH2 and/or CH3 sequence of an IgG). Exemplary adhesin sequences include contiguous amino acid sequences that comprise a portion of a receptor or a ligand that binds to a protein of interest. Adhesin sequences can also be sequences that bind a protein of interest, but are not receptor or ligand sequences (*e.g.*, adhesin sequences in peptibodies). Such polypeptide sequences can be selected or identified by various methods, include phage display techniques and high throughput sorting methods. The immunoglobulin constant domain sequence in the immunoadhesin can be obtained from any immunoglobulin, such as IgG-1, IgG-2, IgG-3, or IgG-4 subtypes, IgA (including IgA-1 and IgA-2), IgE, IgD, or IgM.

In certain embodiments the Fc-region containing heterodimeric polypeptide is an antibody, a bispecific antibody or Fc-fusion proteins

In certain embodiments, the Fc-fusion protein produced according to a method provided herein is a targeted immunocytokine. In certain embodiments, the targeted immunocytokine is a CEA-IL2v immuocytokine. In certain embodiments, the CEA-IL2v immuocytokine is RG7813. In certain embodiments, the targeted immunocytokine is a FAP-IL2v immuocytokine. In certain embodiments, the FAP-IL2v immunocytokine is RG7461.

In certain embodiments, a multispecific antibody (such as a bispecific antibody) produced according to a method provided herein binds CEA and at least one additional target molecule. In certain embodiments, a multispecific antibody (such as a bispecific antibody) produced according to a method provided herein binds a tumor targeted cytokine and at least one additional target molecule. In certain embodiments, a multispecific antibody produced according to a method provided herein is fused to IL2v (*i.e.,* an interleukin 2 variant) and at least one additional target molecule. In certain embodiments, a multispecific antibody produced according to a method provided herein is a T-cell bispecific antibody (*i.e.,* a bispecific T-cell engager or BiTE).

In some embodiments, antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors.

"Complement dependent cytotoxicity" or "CDC" refers to the ability of a molecule to lyse a target in the presence of complement. The complement activation pathway is initiated by the binding of the first component of the complement system (C1q) to a molecule (*e.g.* polypeptide (*e.g.,* an antibody)) complexed with a cognate antigen. To assess complement activation, a CDC assay, *e.g.* as described in Gazzano-Santoro et al., J. Immunol. Methods 202:163 (1996), may be performed.

"Antibody-dependent cell-mediated cytotoxicity" and "ADCC" refer to a cell-mediated reaction in which non-specific cytotoxic cells that express Fc receptors (FcRs) (*e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.*, in an animal model such as that disclosed in Clynes et al., Proc. Natl. Acad. Sci. (USA) 95:652-656 (1998).

"Human effector cells" are leukocytes that express one or more FcRs and perform effector functions. In some embodiments, the cells express at least FcγRIII and carry out ADCC effector function. Examples of human leukocytes that mediate ADCC include peripheral blood mononuclear cells (PBMC), natural killer (NK) cells, monocytes, cytotoxic T cells and neutrophils; with PBMCs and NK cells being preferred.

The terms "Fc receptor" or "FcR" are used to describe a receptor that binds to the Fc region of an antibody. In some embodiments, the FcR is a native sequence human FcR. Moreover, a preferred FcR is one that binds an IgG antibody (a gamma receptor) and includes receptors of the FcγRI, FcγRII, and Fcγ RIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (*see* Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

"Impurities" refer to materials that are different from the desired polypeptide product. The impurity may refer to product-specific polypeptides such as one-armed antibodies and misassembled antibodies, antibody variants including basic variants and acidic variants, and aggregates. Other impurities include process specific impurities including without limitation: host cell materials such as host cell protein (HCP); leached Protein A; nucleic acid; another polypeptide; endotoxin; viral contaminant; cell culture media component, etc. In some examples, the impurity may be an HCP from, for example but not limited to, a bacterial cell such as an *E. coli* cell (ECP), an insect cell, a prokaryotic cell, a eukaryotic cell, a yeast cell, a mammalian cell, an avian cell, a fungal cell. In some examples, the impurity may be an HCP from a mammalian cell, such as a CHO cell, *i.e.,* a CHO cell protein (CHOP). The impurity may refer to accessory proteins used to facilitate expression, folding or assembly of multispecific antibodies; for example, prokaryotic chaperones such as FkpA, DsbA and DsbC.

"Complex" or "complexed" as used herein refers to the association of two or more molecules that interact with each other through bonds and/or forces (*e.g.,* van der waals, hydrophobic, hydrophilic forces) that are not peptide bonds. In one embodiment, the complex is heteromultimeric. It should be understood that the term "protein complex" or "polypeptide complex" as used herein includes complexes that have a non-protein entity conjugated to a protein in the protein complex (*e.g.,* including, but not limited to, chemical molecules such as a toxin or a detection agent).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. In certain embodiments, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, *e.g.,* Kindt et al., Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, *e.g.,* Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "sequential" as used herein with regard to chromatography refers to chromatography steps in a specific sequence; *e.g.,* a first chromatography step followed by a second chromatography step followed by a third chromatography step, *etc.* Additional steps may be included between the sequential chromatography steps.

The term "continuous" as used herein with regard to chromatography refers to having a first chromatography material and a second chromatography material either directly connected or some other mechanism which allows for continuous flow between the two chromatography materials.

"Loading density" refers to the amount, *e.g.* grams, of composition put in contact with a volume of chromatography material, *e.g.* liters. In some examples, loading density is expressed in g/L.

A "sample" refers to a small portion of a larger quantity of material. Generally, testing according to the methods described herein is performed on a sample. The sample is typically obtained from a recombinant polypeptide preparation obtained, for example, from cultured recombinant polypeptide-expressing cell lines, also referred to herein as "product cell lines," or from cultured host cells. As used herein, "host cells" do not contain genes for the expression of recombinant polypeptides of interest or products. A sample may be obtained from, for example but not limited to, harvested cell culture fluid, from an in-process pool at a certain step in a purification process, or from the final purified product. The sample may also include diluents, buffers, detergents, and contaminating species, debris and the like that are found mixed with the desired molecule (such as a multispecific antibody, *e.g.*, a bispecific antibody).

Reference to "about" a value or parameter herein includes (and describes) variations that are directed to that value or parameter per se. For example, description referring to "about X" includes description of "X".

It is understood that aspects and embodiments of the invention described herein include "comprising," "consisting of," and "consisting essentially of" aspects and embodiments.

As used herein and in the appended claims, the singular forms "a," "or," and "the" include plural referents unless the context clearly dictates otherwise. It is understood that aspects and variations of the invention described herein include "consisting" and/or "consisting essentially of" aspects and variations.

All references cited herein, including patent applications and publications, are hereby incorporated by reference in their entirety.

### *Methods of Purification of a Multispecific Antibody*

Provided herein are methods for purifying a multispecific antibody. In certain embodiments the multispecific antibody is a bispecific antibody. In certain embodiments, the multispecific antibody is a divalent F(ab')$_2$ that comprises a first F(ab) that binds a first target and a second F(ab) that binds a second target. In certain embodiments, the multispecific antibody is a dual-specific antibody, *i.e.,* an antibody having two antigen-binding arms that are identical in amino acid sequence, and wherein each Fab arm is capable of recognizing two antigens (such as a dual action Fab antibody).

In some aspects, the purification of the multispecific antibody comprises the sequential steps of capture chromatography, a first mixed mode chromatography and a second mixed mode chromatography. In some embodiments, the multispecific antibody is assembled before capture chromatography. In some embodiments, the multispecific antibody is assembled after capture chromatography.

In some embodiments, the multispecific antibody (such as a bispecific antibody or a divalent F(ab')$_2$) comprises two or more antibody arms wherein different antibody arms bind different epitopes. In certain embodiments, the different epitopes are on the same antigen. In certain embodiments, the each epitope is on a different antigen. In certain embodiments, antibody arms comprise VH/VL units. In certain embodiments, the antibody arms comprise hemimers, also known as half-antibodies. To facilitate assembly, in certain embodiments the heavy chain of one antibody arm is modified to comprise a "knob" and the heavy chain of another antibody arm comprises a "hole" such that the knob of the first heavy chain fits into the hole of the second heavy chain.

In certain embodiments, each arm of the multispecific antibody is produced in a separate cell culture. Following expression of the antibody arm in the host cell, whole cell broth is collected and homogenized, and the antibody arm is extracted. In certain embodiments, polyethyleneimine (PEI) is added to the cell lysate prior to chromatography. In some embodiments, the cell lysate is centrifuged prior to chromatography. Each arm of the multispecific antibody is then purified by capture chromatography (such that each arm is purified on a separate chromatography column or membrane). In certain embodiments, the capture chromatography is affinity chromatography. In certain embodiments, the affinity chromatography is Protein A chromatography. In certain embodiments, the affinity chromatography is Protein G chromatography. In certain embodiments, the affinity chromatography is Protein A/G chromatography. In certain embodiments, the affinity chromatography is Protein L chromatography. Following capture chromatography, purified antibody arms may be analyzed; for example, by SDS-PAGE, SEC chromatography, mass spectrometry, etc. The purified arms of the multispecific antibody are then combined and allowed to assemble, as discussed in further detail elsewhere herein.

In other embodiments, each arm of the multispecific antibody is produced in a separate cell culture. Following expression of the antibody arm in the host cell, whole cell broth is collected and homogenized. The cell homogenates from each culture are then mixed and the combined antibody arms are extracted. In some embodiments, polyethyleneimine (PEI) is added to the cell lysate prior to chromatography. In some embodiments, the cell lysate is centrifuged prior to chromatography. The combined arms of the multispecific antibody are then purified by affinity chromatography. In some embodiments, the affinity chromatography is protein A chromatography. At this point, purified antibody arms may be analyzed; for example, by SDS-PAGE SEC chromatography, mass spectrometry, etc. The purified arms of the multispecific antibody are then combined and allowed to assemble by the methods described herein.

In other embodiments, each arm of the multispecific antibody is produced in the same cell culture. Following expression of the antibody arm in the host cell, whole cell broth is collected and homogenized and the antibody arms are extracted. In some embodiments, polyethyleneimine (PEI) is added to the cell lysate prior to chromatography. In some embodiments, the cell lysate is centrifuged prior to chromatography. The arms of the multispecific antibody are then purified by affinity chromatography. In some embodiments, the affinity chromatography is protein A chromatography. At this point, purified antibody arms may be analyzed; for example, by SDS-PAGE, SEC chromatography, mass spectrometry, etc. The purified arms of the multispecific antibody are then allowed to assemble by the methods described herein.

In some embodiments, the final concentration of PEI in the cell lysate is at least about any of 0.1%, 0.2%, 0.3%, 0.4%, 0.5%, 0.6%, 0.7%, 0.8%, 0.9%, 1.0%, 2.0%, 3.0%, 4.0%, or 5.0. In some embodiments, the final concentration of PEI in the cell lysate is between about any one of 0.1% and 5%, 0.1% and 1%, 0.1% and 0.5%, 0.5% and 5%, 0.5% and 1%, or 1% and 5%. In some embodiments, the cell lysate comprising PEI is held for more than about any of 1 hr, 2 hr, 3 hr, 4 hr, 5 hr, 6 hr, 7 hr, 8 hr, 9 hr, 10 hr, 12 hr, 14 hr, 16 hr, 18 hr, 20 hr, or 24 hr. In some embodiments, the cell lysate comprising PEI is held for between about any one of 1 hr and 24 hrs, 1 hr and 6 hrs, 6 hr and 12 hrs, 12 hrs and 18 hrs, 18 hrs and 24 hrs. In some embodiments, the cell lysate comprising PEI is held for between about any one of 10 hr and 14 hrs. In some embodiments, the cell lysate comprising PEI is held at between about 4°C and 37°C. In some embodiments, the cell lysate comprising PEI is held at about ambient temperature.

In some embodiments, the cell lysate is clarified by centrifugation prior to chromatography. In some embodiments, the cell lysate is filtered prior to chromatography. In some embodiments, the cell lysate is filtered through a 0.22 $\mu$m filter prior to chromatography.

Examples of affinity chromatography include, but are not limited to, *e.g.,* protein A chromatography, protein G chromatography, protein A/G chromatography, or protein L chromatography. Examples of affinity chromatography material include, but are not limited to, ProSep®-vA, ProSep® Ultra Plus, Protein A Sepharose® Fast Flow, Toyopearl® AF-rProtein A, MabSelect™, MabSelect SuRe™, MabSelect SuRe™ LX, KappaSelect, CaptureSelect™ and CaptureSelect™ FcXL. In certain embodiments, the affinity chromatography material is in a column. In certain embodiments, the affinity chromatography is performed in "bind and elute mode" (alternatively referred to as "bind and elute process"). "Bind and elute mode" refers to a product separation technique in which a product (such as the multispecific antibody) in the sample binds the affinity chromatography material and is subsequently eluted from the affinity chromatography material. In some embodiments, the elution is a step elution, in which the composition of the mobile phase is changed stepwise, at one or several occasions, during the elution process. In certain embodiments, the elution is gradient elution, in which the composition of the mobile phase is changed continuously during the elution process. In certain embodiments, the affinity chromatography material is a membrane. In certain embodiments, the affinity chromatography is protein A chromatography. In certain embodiments, the protein A chromatography is MAbSelect SuRe chromatography. In certain embodiments, the affinity chromatography is CaptureSelect chromatography. In certain embodiments, the affinity chromatography is CaptureSelect FcXL chromatography.

In certain embodiments, the eluate from the affinity chromatography step is subsequently applied to a first mixed mode chromatography. In certain embodiments, the first mixed mode material comprises functional groups capable of one of more of the following functionalities: anionic exchange, cationic exchange, hydrogen bonding, pi-pi bond interactions, hydrophilic interactions, thiophilic interactions, and hydrophobic interactions. In certain embodiments, the first mixed mode material comprises functional groups capable of anionic exchange and hydrophobic interactions. In certain embodiments, the first mixed mode material comprises functional groups capable of cationic exchange and hydrophobic interactions. In certain embodiments, the first mixed mode material contains N-benzyl-N-methyl ethanol amine, 4-mercapto-ethyl-pyridine, 2-benzamido-4-mercaptobutanoic acid, hexylamine, or phenylpropylamine, or cross-linked polyallylamine. Examples of the mixed mode materials include Capto™ Adhere resin, Capto™ MMC resin, MEP HyperCel™ resin, HEA HyperCel™ resin, PPA HyperCel™ resin, Eshmuno® HCX, Capto™Adhere ImpRes, Capto™MMC Impres, Nuvia™cPrime™ membrane. In some embodiments, the first mixed mode material is Capto™ Adhere resin. In certain embodiments the first mixed mode material is Capto™ Adhere resin. In certain embodiments, the first mixed mode material is Capto™ MMC. In certain embodiments, the first mixed mode chromatography does not include ceramic hydroxyapatite chromarography. In certain embodiments, the first mixed mode chromatography is performed in "bind and elute" mode. In some embodiments, the elution is a step elution. In certain embodiments, the elution is gradient elution. In certain embodiments, the first mixed mode chromatography is performed in "flow through" mode. In certain embodiments of the above, the first mixed mode material is in a column. In certain embodiments of the above, the first mixed mode material is in a membrane.

In certain embodiments, the capture chromatography and the first mixed mode chromatography are continuous, *e.g.*, wherein the capture chromatography material and the first mixed mode material are either directly connected or connected by some other mechanism that allows for continuous flow between the capture chromatography material and the first mixed mode material. In certain embodiments, the capture chromatography and the first mixed mode chromatography are contiguous, wherein the first mixed mode chromatography is performed directly after the capture chromatography.

In certain embodiments, the eluate from the capture chromatography is subject to one or more additional chromatography steps prior being applied to the first mixed mode resin. For example, the eluate from the capture chromatography can be subject to any one or more of the following chromatography steps in any order and/or in any combination prior to being subject to a first mixed mode chromatography: hydrophobic interaction (HIC) chromatography, anion exchange chromatography, cation exchange chromatography, size exclusion chromatography, affinity chromatography, ceramic hydroxyapatite (CHT) chromatography, hydrophilic interaction liquid chromatography (HILIC), etc.

Hydrophobic interaction chromatography is a liquid chromatography technique that separates biomolecules according to hydrophobicity. Examples of HIC chromatography materials include, but are not limited to, *e.g.*, Toyopearl® Hexyl-650, Toyopearl® Butyl-650, Toyopearl® Phenyl-650, Toyopearl® Ether-650, HiTrap® Sepharose, Octyl Sepharose®, Phenyl Sepharose® or Butyl Sepharose®. In some embodiments, the HIC chromatography material comprises phenyl sepharose. In certain embodiments, the HIC chromatography is performed in "bind and elute" mode. In some embodiments, the HIC chromatography is performed in "flow through" mode. In some embodiments of the above, the HIC chromatography material is in a column. In some embodiments of the above, the HIC chromatography material is in a membrane.

Anion exchange chromatography material is a solid phase that is positively charged and has free anions for exchange with anions in an aqueous solution (such as a composition comprising a multispecific antibody and an impurity) that is passed over or through the solid phase. In some embodiments of any of the methods described herein, the anion exchange material may be a membrane, a monolith, or resin. In an embodiment, the anion exchange material may be a resin. In some embodiments, the anion exchange material may comprise a primary amine, a secondary amine, a tertiary amine or a quaternary ammonium ion functional group, a polyamine functional group, or a diethylaminoethyl functional group. Examples of anion exchange materials are known in the art and include, but are not limited to Poros® HQ 50, Poros® PI 50, Poros® D, Mustang® Q, Q Sepharose® Fast Flow (QSFF), Accell™ Plus Quaternary Methyl Amine (QMA) resin, Sartobind STIC®, and DEAE-Sepharose®. In some embodiments, the anion exchange chromatography is performed in "bind and elute" mode. In some embodiments, the anion exchange chromatography is performed in "flow through" mode. In some embodiments of the above, the anion exchange chromatography material is in a column. In some embodiments of the above, the anion exchange chromatography material is a membrane.

Cation exchange chromatography material is a solid phase that is negatively charged and has free anions for exchange with cations in an aqueous solution (such as a composition comprising a multispecific antibody and an impurity) that is passed over or through the solid phase. In some embodiments of any of the methods described herein, the cation exchange material may be a membrane, a monolith, or resin. In some embodiments, the cation exchange material may be a resin. The cation exchange material may comprise a carboxylic acid functional group or a sulfonic acid functional group such as, but not limited to, sulfonate, carboxylic, carboxymethyl sulfonic acid, sulfoisobutyl, sulfoethyl, carboxyl, sulphopropyl, sulphonyl, sulphoxyethyl, or orthophosphate. In some embodiments of the above, the cation exchange chromatography material is a cation exchange chromatography column. In some embodiments of the above, the cation exchange chromatography material is a cation exchange chromatography membrane. Examples of cation exchange materials are known in the art include, but are not limited to Mustang® S, Sartobind® S, $SO_3$ Monolith (such as, *e.g.*, CIM®, CIMmultus® and CIMac® $SO_3$), S Ceramic HyperD®, Poros® XS, Poros® HS 50, Poros® HS 20, sulphopropyl-Sepharose® Fast Flow (SPSFF), SP-Sepharose® XL (SPXL), CM Sepharose® Fast Flow, Capto™ S, Fractogel® EMD Se Hicap, Fractogel® EMD $SO_3^-$, or Fractogel® EMD COO⁻. In some embodiments, the cation exchange chromatography is performed in "bind and elute" mode. In some embodiments, the cation exchange chromatography is performed in "flow through" mode. In some embodiments of the above, the cation exchange chromatography material is in a column. In some embodiments of the above, the cation exchange chromatography material is in a membrane.

The functional groups of hydroxyapatite ($Ca_{10}(PO_4)_6(OH)_2$) chromatography material comprise positively charged pairs of crystal calcium ions (C-sites) and clusters of six negatively charged oxygen atoms associated with triplets of crystal phosphates (P-sites). C-sites, P-sites, and hydroxyls are distributed in a fixed pattern on the crystal surface. Proteins are typically adsorbed to hydroxyapatite in a low concentration (*e.g.*, 10-25mM) of phosphate buffer, although certain acidic proteins can be adsorbed if loaded in water, saline, or a nonphosphate buffer. Proteins are usually eluted by an increasing phosphate gradient, although gradients of $Ca^{2+}$, $Mg^{2+}$, or $Cl^-$ ions can also be used, such as for the selective elution of basic proteins. In some embodiments of any of the methods described herein, the hydroxyapatite chromatography material may be a resin. In some embodiments, the hydroxyapatite chromatography material may be a resin. In some embodiments of the above, the hydroxyapatite chromatography material is a column. Examples of hydroxyapatite chromatography materials are known in the art include, but are not limited to CHT™ Ceramic Hydroxyapatite, CHT Ceramic Hydroxyapatite Type I support, CHT Ceramic Hydroxyapatite Type II support. In some embodiments, the hydroxyapatite chromatography is performed in "bind and elute" mode. In some embodiments, the hydroxyapatite chromatography is performed in "flow through" mode.

In one embodiment the method as reported herein in addition can comprise a method of separating a bispecific antibody comprising an Fc domain from a solution comprising said bispecific antibody, said method comprising (a) contacting said solution with a hydroxyapatite chromatography medium, (b) adsorbing said bispecific antibodies to said hydroxyapatite chromatography medium, and (c) eluting said bispecific antibody from said hydroxyapatite chromatography medium in the presence of chloride ions, wherein said solution further comprises one or more fragments of said bispecific antibody, which one or more fragments comprise an Fc domain; and/or wherein said solution further comprises one or more polypeptides having a molecular weight greater than the molecular weight of said bispecific antibody and comprises at least one of the two heavy chains of said bispecific antibody, which one or more polypeptides further comprise an Fc domain as referred in WO2015024896.

In certain embodiments, the eluate from the capture chromatography is subject to anion exchange chromatography. In certain embodiments, the anion exchange chromatography material is Q Sepharose® Fast Flow (QSFF). In certain embodiments, the anion exchange chromatography is performed in "bind and elute" mode.

In certain embodiments, an eluate collected following the first mixed mode chromatography is subsequently applied to a second mixed mode chromatography. In certain embodiments, the second mixed mode material comprises functional groups capable of one of more of the following functionalities: anionic exchange, cationic exchange, hydrogen bonding, pi-pi bond interactions, hydrophilic interactions, thiophilic interactions, and hydrophobic interactions. In certain embodiments, the second mixed mode material comprises functional groups capable of anionic exchange and hydrophobic interactions. In certain embodiments, the second mixed mode material comprises functional groups capable of cationic exchange and hydrophobic interactions. In certain embodiments, the second mixed mode material contains N-benzyl-N-methyl ethanol amine, 4-mercapto-ethyl-pyridine, 2-benzamido-4-mercaptobutanoic acid, hexylamine, or phenylpropylamine, or cross-linked polyallylamine. Examples of the mixed mode materials include Capto™ Adhere resin, Capto™ MMC resin, MEP HyperCel™ resin, HEA HyperCel™ resin, Eshmuno® HCX, Capto™Adhere ImpRes, Capto™MMC Impres, Nuvia™cPrime™ membrane. In some embodiments, the second mixed mode material is Capto™ Adhere resin. In certain embodiments, the second mixed mode material is Capto™ Adhere resin. In certain embodiments, the second mixed mode material is Capto™ MMC. In certain embodiments, the second mixed mode chromatography does not include ceramic hydroxyapatite chromarography. In certain embodiments, the second mixed mode chromatography is performed in "bind and elute" mode. In some embodiments, the elution is a step elution. In certain embodiments, the elution is gradient elution. In certain embodiments, the first mixed mode chromatography is performed in "flow through" mode. In certain embodiments of the above, the second mixed mode material is in a column. In certain embodiments of the above, the second mixed mode material is a membrane.

In certain embodiments, the first mixed mode chromatography and the second mixed mode chromatography are continuous, *e.g.*, wherein the capture chromatography material and the first mixed mode material are either directly connected or connected by some other mechanism that allows for continuous flow between the capture chromatography material and the first mixed mode material. In certain embodiments, the first mixed mode chromatography and the second mixed mode chromatography are contiguous, wherein the second mixed mode chromatography is performed directly after the first mixed mode chromatography.

In certain embodiments, the eluate from the first mixed mode chromatography is subject to one or more additional chromatography operations prior being applied to the second mixed mode resin. For example, the eluate from the first mixed mode chromatography can be subject to any one or more of the following chromatography steps in any order and/or in any combination prior to being subject to a second mixed mode chromatography: hydrophobic interaction (HIC) chromatography, anion exchange chromatography, cation exchange chromatography, size exclusion chromatography, affinity chromatography, ceramic hydroxyapatite (CHT) chromatography, hydrophilic interaction liquid chromatography (HILIC), etc.

In certain embodiments of any of the methods described herein, the eluate from the second mixed mode chromatography is subject to one or more additional chromatography steps. For example, the eluate from the second mixed mode chromatography can be subject to any one or more of the following chromatography steps in any order and/or in any combination: hydrophobic interaction (HIC) chromatography, anion exchange chromatography, cation exchange chromatography, size exclusion chromatography, affinity chromatography, ceramic hydroxyapatite (CHT) chromatography, hydrophilic interaction liquid chromatography (HILIC), mixed mode chromatography, etc.

In certain embodiments of any of the methods described herein, the methods comprise using a buffer. Various buffers can be employed during the purification of the multispecific antibody depending, for example, on the desired pH of the buffer, the desired conductivity of the buffer, the characteristics of the multispecific antibody that is being purified, and the purification method. The buffer can be a loading buffer, an equilibration buffer, or a wash buffer. In certain embodiments, one or more of the loading buffer, the equilibration buffer, and/or the wash buffer are the same. In certain embodiments, the loading buffer, the equilibration buffer, and/or the wash buffer are different. In certain embodiments of any of the methods described herein, the buffer comprises a salt. In certain embodiments, the buffer comprises sodium chloride, sodium acetate, Tris HCl, Tris acetate, sodium phosphate, potassium phosphate, MES, CHES, MOPS, BisTris, arginine, arginine HCl, or a mixture thereof. In certain embodiments, the buffer is a sodium chloride buffer. In some embodiments, the buffer is a sodium acetate buffer. In certain embodiments, the buffer is Tris, arginine, phosphate, MES, CHES, or MOPS buffer.

"Load" refers to the composition being loaded onto a chromatography material. Loading buffer is the buffer used to load the composition (*e.g.*, a composition comprising a multispecific antibody and an impurity or a composition comprising an antibody arm and an impurity) onto a chromatography material (such as any one of the chromatography materials described herein). The chromatography material may be equilibrated with an equilibration buffer prior to loading the composition which is to be purified. The wash buffer is used after loading the composition onto a chromatography material. An elution buffer is used to elute the polypeptide of interest from the solid phase.

Loading of a composition comprising the multispecific antibody (such as a composition comprising the multispecific antibody and an impurity) on any of the chromatography materials described herein may be optimized for separation of the multispecific antibody from the impurity. In some embodiments, loading of the composition comprising the multispecific antibody (such as a composition comprising the multispecific antibody and an impurity) onto the chromatography material is optimized for binding of the multispecific antibody to the chromatography material when the chromatography is performed in bind and elute mode (*e.g.*, affinity chromatography, mixed mode chromatography and ion exchange chromatography, as designated herein).

Conductivity refers to the ability of an aqueous solution to conduct an electric current between two electrodes. In solution, the current flows by ion transport. Therefore, with an increasing amount of ions present in the aqueous solution, the solution will have a higher conductivity. The basic unit of measure for conductivity is the Siemen (mS/cm) or ohms (mho), and can be measured using a conductivity meter, such as various models of Orion conductivity meters. Since electrolytic conductivity is the capacity of ions in a solution to carry electrical current, the conductivity of a solution may be altered by changing the concentration of ions therein. For example, the concentration of a buffering agent and/or the concentration of a salt (*e.g.* sodium chloride, sodium acetate, or potassium chloride) in the solution may be altered in order to achieve the desired conductivity. Preferably, the salt concentration of the various buffers is modified to achieve the desired conductivity.

For example, in certain embodiments, the composition comprising the multispecific antibody (such as a composition comprising the multispecific antibody and an impurity) is loaded onto the chromatography material, *e.g.* a chromatography column comprising any one of the chromatography materials described herein, in a loading buffer at a number of different pH values while the conductivity of the loading buffer is constant. Alternatively, the solution comprising the multispecific antibody may be loaded onto the chromatography material in a loading buffer at a number of different conductivities while the pH of the loading buffer is constant. Upon completion of loading the composition comprising the multispecific antibody (such as a composition comprising the multispecific antibody and an impurity) on the chromatography material and elution of the multispecific antibody from the chromatography material into a pool fraction, the amount of impurity remaining in the pool fraction provides information regarding the separation of the multispecific antibody from the impurity for a given pH or conductivity. Likewise, for chromatography where the multispecific antibody flows through the chromatography material the loading buffer is optimized for pH and conductivity such that the multispecific antibody flows through the chromatography whereas the impurity is retained by the chromatography material or flows through the chromatography material at a different rate than the multispecific antibody.

In some embodiments, the loading density of the solution comprising the multispecific antibody or antibody arms is greater than about any of 10 g/L, 20 g/L, 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the affinity chromatography material (*e.g.*, protein A chromatography material). In some embodiments, the loading density of the solution comprising the multispecific antibody or antibody arms is between about any of 10 g/L and 20 g/L, 20 g/L and 30 g/L, 30 g/L and 40 g/L, 40 g/L and 50 g/L, 50 g/L and 60 g/L, 60 g/L and 70 g/L, 70 g/L and 80 g/L, 80 g/L and 90 g/L, 90 g/L and 100 g/L, of the capture chromatography material (such as an affinity chromatography material, *e.g.*, a Protein A chromatography material, a Protein G chromatography material, a Protein A/G chromatography material, or a Protein L chromatography material).

In some embodiments of any of the methods described herein, the eluate obtained following the capture chromatography is loaded onto an anion exchange chromatography material (*e.g.*, Q Sepharose® Fast Flow (QSFF)). In some embodiments of any of the methods described herein, the eluate obtained following the capture chromatography is loaded onto an anion exchange chromatography material at a loading density of the multispecific antibody of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the anion chromatography material (*e.g.*, Q Sepharose® Fast Flow (QSFF)). In some embodiments, the eluate obtained following the capture chromatography is loaded onto an anion exchange chromatography material at a loading density of the multispecific antibody between about any of 10 g/L and 20 g/L, 20 g/L and 30 g/L, 30 g/L and 40 g/L, 40 g/L and 50 g/L, 50 g/L and 60 g/L, 60 g/L and 70 g/L, 70 g/L and 80 g/L, 80 g/L and 90 g/L, 90 g/L and 100 g/L of the anion exchange chromatography material (*e.g.*, Q Sepharose® Fast Flow (QSFF)).

In some embodiments of any of the methods described herein, the eluate obtained following the capture chromatography (optionally following capture chromatography and one or more additional chromatography steps comprising any of the chromatography operations described herein) is loaded onto a first mixed mode chromatography material at a loading density of the multispecific antibody of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the first mixed mode chromatography material (*e.g.*, Capto™ Adhere chromatography material or a Capto™ MMC chromatography material). In some embodiments, the eluate obtained following the capture chromatography is loaded onto a first mixed mode chromatography material at a loading density of the multispecific antibody between about any of 10 g/L and 20 g/L, 20 g/L and 30 g/L, 30 g/L and 40 g/L, 40 g/L and 50 g/L, 50 g/L and 60 g/L, 60 g/L and 70 g/L, 70 g/L and 80 g/L, 80 g/L and 90 g/L, 90 g/L and 100 g/L of the first mixed mode chromatography material (*e.g.*, Capto™ Adhere chromatography material or a Capto™ MMC chromatography material).

In some embodiments of any of the methods described herein, the eluate obtained following the first mixed mode chromatography is loaded onto a second mixed mode chromatography material at a loading density of the multispecific antibody of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the second mixed mode chromatography material (e.g., Capto™ Adhere chromatography material or a Capto™ MMC chromatography material). In some embodiments, the eluate obtained following the first mixed mode chromatography is loaded onto the second mixed mode chromatography material at a loading density of the multispecific antibody between about any of 10 g/L and 20 g/L, 20 g/L and 30 g/L, 30 g/L and 40 g/L, 40 g/L and 50 g/L, 50 g/L and 60 g/L, 60 g/L and 70 g/L, 70 g/L and 80 g/L, 80 g/L and 90 g/L, 90 g/L and 100 g/L, of the mixed mode chromatography material (*e. g.*, Capto™ Adhere chromatography material or a Capto™ MMC chromatography material).

In some embodiments of any of the methods described herein, the eluate obtained following the second mixed mode chromatography is loaded onto a subsequent chromatography material (such as a hydrophobic interaction (HIC) chromatography material, anion exchange chromatography material, cation exchange chromatography material, size exclusion chromatography material, affinity chromatography material, or an additional mixed mode chromatography material) at a loading density of the multispecific antibody of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the subsequent chromatography material. In some embodiments, the eluate obtained following the second mixed mode chromatography is loaded onto the subsequent chromatography material (such as a hydrophobic interaction (HIC) chromatography material, anion exchange chromatography material, cation exchange chromatography material, size exclusion chromatography material, affinity chromatography material, or an additional mixed mode chromatography material) at a loading density of the multispecific antibody between about any of 10 g/L and 20 g/L, 20 g/L and 30 g/L, 30 g/L and 40 g/L, 40 g/L and 50 g/L, 50 g/L and 60 g/L, 60 g/L and 70 g/L, 70 g/L and 80 g/L, 80 g/L and 90 g/L, 90 g/L and 100 g/L, of the subsequent chromatography material.

Elution, as used herein, is the removal of the product, e.g. multispecific antibody or antibody arm, from the chromatography material. Elution buffer is the buffer used to elute the multispecific antibody or other product of interest from a chromatography material. In many cases, an elution buffer has a different physical characteristic than the loading buffer. For example, the elution buffer may have a different conductivity than the loading buffer or a different pH than the loading buffer. In some embodiments, the elution buffer has a lower conductivity than the loading buffer. In some embodiments, the elution buffer has a higher conductivity than the loading buffer. In some embodiments, the elution buffer has a lower pH than the load buffer. In some embodiments, the elution buffer has a higher pH than the load buffer. In some embodiments the elution buffer has a different conductivity and a different pH than the load buffer. The elution buffer can have any combination of higher or lower conductivity and higher or lower pH.

In certain embodiments, elution of the multispecific antibody from the chromatography material is optimized for yield of product with minimal impurity and at minimal elution volume or pool volume. For example, the composition containing the multispecific antibody (*e.g.*, bispecific antibody) or antibody arms may be loaded onto the chromatography material, *e.g.* a chromatography column, in a loading buffer. Upon completion of load, the multispecific antibody or antibody arm is eluted with buffers at a number of different pH values while the conductivity of the elution buffer is constant. Alternatively, the multispecific antibody or antibody arm may be eluted from the chromatography material in an elution buffer at a number of different conductivities while the pH of the elution buffer is constant. Upon completion of elution of the multispecific antibody (*e.g.*, bispecific antibody) or antibody arm from the chromatography material, the amount of an impurity in the pool fraction provides information regarding the separation of the multispecific antibody or antibody arm from the impurities for a given pH or conductivity. Elution of the multispecific antibody or antibody arm in a high number of column volumes (*e.g.* eight column volumes) indicates "tailing" of the elution profile. In some embodiments, tailing of the elution is minimized.

Various buffers which can be employed depending, for example, on the desired pH of the buffer, the desired conductivity of the buffer, the characteristics of the protein of interest, the chromatography material, and the purification process (*e.g.*, "bind and elute" or "flow through" mode). In some embodiments of any of the methods described herein, the methods comprise the use of at least one buffer. The buffer can be a loading buffer, an equilibration buffer, an elution buffer or a wash buffer. In some embodiments, one or more of the loading buffer, the equilibration buffer, the elution buffer and/or the wash buffer (such as a loading buffer, an equilibration buffer, and/or a wash buffer used for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) are the same. In some embodiments, the loading buffer, the equilibration buffer, and/or the wash buffer (such as a loading buffer, an equilibration buffer, and/or a wash buffer used for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) are different. In some embodiments of any of the methods described herein, the buffer comprises a salt. The loading buffer (such as a loading buffer, an equilibration buffer, and/or a wash buffer used for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) may comprise sodium chloride, sodium acetate, Tris, arginine, phosphate, MOPS, MES, CHES, BisTris, ammonium sulfate, sodium sulfate, citrate, succinate, or mixtures thereof. In certain embodiments, the buffer is a sodium chloride buffer. In some embodiments, the buffer is a sodium acetate buffer. In certain embodiments, the buffer is Tris, arginine, phosphate, MES, CHES, or MOPS buffer. In some embodiments, the buffer comprises Tris. In some embodiments, the buffer comprises arginine.

In some embodiments of any of the methods described herein, the loading buffer (such as a loading buffer used for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) has a conductivity of greater than about any of 1.0 mS/cm, 1.5 mS/cm, 2.0 mS/cm, 2.5 mS/cm, 3.0 mS/cm, 3.5 mS/cm, 4.0 mS/cm, 4.5 mS/cm, 5.0 mS/cm, 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, 10 mS/cm or 20 mS/cm. The conductivity may be between about any of 1 mS/cm and 20 mS/cm, 4 mS/cm and 10 mS/cm, 4 mS/cm and 7 mS/cm, 5 mS/cm and 17 mS/cm, 5 mS/cm and 10 mS/cm, or 5 mS/cm and 7 mS/cm. In some embodiments, the conductivity is about any of 1.0 mS/cm, 1.5 mS/cm, 2.0 mS/cm, 2.5 mS/cm, 3.0 mS/cm, 3.5 mS/cm, 4 mS/cm, 4.5 mS/cm, 5.0 mS/cm, 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, 10 mS/cm or 20 mS/cm. In one aspect, the conductivity is the conductivity of the loading buffer, the equilibration buffer, and/or the wash buffer. In some embodiments, the conductivity of one or more of the loading buffer, the equilibration buffer, and the wash buffer (such as a loading buffer, an equilibration buffer, and/or a wash buffer used for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) are the same. In some embodiments, the conductivity of the loading buffer is different from the conductivity of the wash buffer and/or equilibration buffer.

In some embodiments, the elution buffer (such as an elution buffer for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) has a conductivity less than the conductivity of the loading buffer. In some embodiments of any of the methods described herein, the elution buffer (such as the elution buffer for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) has a conductivity of less than about any of 0 mS/cm, 0.5 mS/cm, 1.0 mS/cm, 1.5 mS/cm, 2.0 mS/cm, 2.5 mS/cm, 3.0 mS/cm, 3.5 mS/cm, 4.0 mS/cm, 4.5 mS/cm, 5.0 mS/cm, 5.5 mS/cm, 6.0 mS/cm, 6.5 mS/cm, or 7.0 mS/cm. The conductivity may be between about any of 0 mS/cm and 7 mS/cm, 1 mS/cm and 7 mS/cm, 2 mS/cm and 7 mS/cm, 3 mS/cm and 7 mS/cm, or 4 mS/cm and 7 mS/cm, 0 mS/cm and 5.0 mS/cm, 1 mS/cm and 5 mS/cm, 2 mS/cm and 5 mS/cm, 3 mS/cm and 5 mS/cm, or 4 mS/cm and 5 mS/cm. In some embodiments, the conductivity of the elution buffer (such as the elution buffer for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) is about any of 0 mS/cm, 0.5 mS/cm, 1.0 mS/cm, 1.5 mS/cm, 2.0 mS/cm, 2.5 mS/cm, 3.0 mS/cm, 3.5 mS/cm, 4 mS/cm, 4.5 mS/cm, 5.0 mS/cm, 5.5 mS/cm, 6.0 mS/cm, 6.5 mS/cm, or 7.0 mS/cm.

In some embodiments, the elution buffer has a conductivity greater than the conductivity of the loading buffer. In some embodiments of any of the methods described herein, the elution buffer (such as the elution buffer for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) has a conductivity of greater than about any of 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, 10 mS/cm, 11 mS/cm, 12 mS/cm, 13 mS/cm, 14 mS/cm, 15 mS/cm, 16 mS/cm, 17.0 mS/cm, 18.0 mS/cm, 19.0 mS/cm, 20.0 mS/cm, 21.0 mS/cm, 22.0 mS/cm, 23.0 mS/cm, 24.0 mS/cm, 25.0 mS/cm, 26.0 mS/cm, 27.0 mS/cm, 28.0 mS/cm, 29.0 mS/cm, or 30.0 mS/cm. The conductivity may be between about any of 5.5 mS/cm and 30 mS/cm, 6.0 mS/cm and 30 mS/cm, 7 mS/cm and 30 mS/cm, 8 mS/cm and 30 mS/cm, 9 mS/cm and 30 mS/cm, or 10 mS/cm and 30 mS/cm. In some embodiments, the conductivity of the elution buffer is about any of 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, 10 mS/cm, 11 mS/cm, 12 mS/cm, 13 mS/cm, 14 mS/cm, 15 mS/cm, 16 mS/cm, 17.0 mS/cm 18.0 mS/cm, 19.0 mS/cm, 20.0 mS/cm, 21.0 mS/cm, 22.0 mS/cm, 23.0 mS/cm, 24.0 mS/cm, 25.0 mS/cm, 26.0 mS/cm, 27.0 mS/cm, 28.0 mS/cm, 29.0 mS/cm, or 30.0 mS/cm. In some aspects of any of the above embodiments, the conductivity of the elution buffer (such as the elution buffer for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) is changed from the load and/or wash buffer by step gradient or by linear gradient.

In some embodiments, the solution comprising the multispecific antibody is loaded onto the first mixed mode chromatography material in a loading buffer with a conductivity of about <6.5 mS/cm and the polypeptide is eluted from first mixed chromatography material in an elution buffer with a conductivity of about 1.5 mS/cm. In some embodiments, the loading buffer has a conductivity of about 6.5 mS/cm and the elution buffer has a conductivity of about 3 mS/cm. In some embodiments, the loading buffer has a conductivity of about 5.5 mS/cm and the elution buffer has a conductivity of about 2 mS/cm. In some embodiments, the loading buffer has a conductivity of about 5.5 mS/cm and the elution buffer has a conductivity of about 1 mS/cm. In further embodiments of the above embodiments, the first mixed mode chromatography material is a Capto™ Adhere resin. In further embodiments of the above embodiments, the first mixed mode chromatography material is a Capto™ MMC resin.

In some aspects of any of the above embodiments, the conductivity of the elution buffer is changed from the load and/or wash buffer by step gradient or by linear gradient. In some embodiments, the composition comprising a multispecific antibody is loaded onto a first mixed mode chromatography (e.g., a Capto™ Adhere chromatography or a Capto™ MMC chromatography) at <6.5 mS/cm and the multispecific antibody is eluted from the first mixed mode chromatography by a step conductivity gradient to about 1.5 mS/cm.

In some embodiments, the solution comprising the multispecific antibody is loaded onto the second mixed mode chromatography material in a loading buffer with a conductivity of about <6.5 mS/cm and the polypeptide is eluted from second mixed chromatography material in an elution buffer with a conductivity of about 1.5 mS/cm. In some embodiments, the loading buffer has a conductivity of about 6.5 mS/cm and the elution buffer has a conductivity of about 3 mS/cm. In some embodiments, the loading buffer has a conductivity of about 5.5 mS/cm and the elution buffer has a conductivity of about 2 mS/cm. In some embodiments, the loading buffer has a conductivity of about 5.5 mS/cm and the elution buffer has a conductivity of about 1 mS/cm. In further embodiments of the above embodiments, the second mixed mode chromatography material is a Capto™ Adhere resin. In further embodiments of the above embodiments, the second mixed mode chromatography material is a Capto™ MMC resin.

In some aspects of any of the above embodiments, the conductivity of the elution buffer is changed from the load and/or wash buffer by step gradient or by linear gradient. In some embodiments, the composition comprising a multispecific antibody is loaded onto a second mixed mode chromatography (e.g., a Capto™ Adhere chromatography or a Capto™ MMC chromatography) at <6.5 mS/cm and the multispecific antibody is eluted from the second mixed mode chromatography by a step conductivity gradient to about 1.5 mS/cm.

In some embodiments, the composition comprising a multispecific antibody is loaded onto an anion exchange chromatography (e.g., a QSFF chromatography) at <2.5 mS/cm and the multispecific antibody is eluted from the anion exchange chromatography by a step conductivity gradient to about 8.6 mS/cm.

In some embodiments, the composition comprising a multispecific antibody is loaded onto a cation exchange chromatography (e.g., a POROS 50HS chromatography) at about 5.0 mS/cm and the multispecific antibody is eluted from the cation exchange chromatography by a step conductivity gradient to about 27.5 mS/cm.

In some embodiments of any of the methods described herein, the loading buffer (such as a loading buffer used for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) has a pH of less than about any of 10, 9, 8, 7, 6, or 5, including any range in between these values. In some embodiments of any of the methods described herein, the loading buffer (such as a loading buffer used for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) has a pH of greater than about any of 4, 5, 6, 7, 8, or 9, including any range in between these values. The loading buffer (such as a loading buffer used for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) may have a pH of between about any of 4 and 9, 4 and 8, 4 and 7, 5 and 9, 5 and 8, 5 and 7, 5 and 6, including any range in between these values. In some embodiments, the pH of the loading buffer (such as a loading buffer used for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) has a pH of about any of 4, 4.5, 5, 5.5, 6, 6.5, 7, 7.5, or 8, including any range in between these values. The pH can be the pH of the loading buffer, the equilibration buffer, or the wash buffer (such as a loading buffer, equilibration buffer, and/or wash buffer used for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.). In some embodiments, the pH of one or more of the loading buffer, the equilibration buffer, and/or the wash buffer are the same. In some embodiments, the pH of the loading buffer is different from the pH of the equilibration buffer and/or the wash buffer.

In some embodiments, the elution buffer (such as an elution buffer for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) has a pH less than the pH of the load buffer. In some embodiments of any of the methods described herein, the elution buffer has a pH of less than about any of 8, 7, 6, 5, 4, 3 or 2, including any range in between these values. The pH of the elution buffer may be between about any of 4 and 9, 4 and 8, 4 and 7, 4 and 6, 4 and 5, 5 and 9, 5 and 8, 5 and 7, 5 and 6, 6 and 9, 6 and 8, 6 and 7, including any range in between these values. In some embodiments, the pH of the elution buffer (such as an elution buffer for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) is about any of 4.0, 4.5, 5.0, 5.5, 6.0, 6.5, 7.0, 7.5, 8.0, 8.5 or 9.0, including any range in between these values.

In some embodiments, the elution buffer (such as an elution buffer for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) has a pH greater than the pH of the loading buffer. In some embodiments of any of the methods described herein, the elution buffer (such as an elution buffer for the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) has a pH of greater than about any of 5, 6, 7, 8, or 9, including any range in between these values. In some embodiments of any of the methods described herein, the elution buffer (such as an elution buffer for the capture chromatography) has a pH of greater than about any of 2, 4, or 4, including any range in between these values. The pH of the elution buffer (such as an elution buffer for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) may be between about any of 2 and 9, 3 and 9, 4 and 9, 2 and 8, 3 and 8, 4 and 8, 2 and 7, 3 and 7, 4 and 7, 2 and 6, 3 and 6, and 4 and 6, including any range in between these values. In some embodiments, the pH of the elution buffer is about any of 2.0, 2.5, 3.0, 3.5, 4.0, including any range in between these values.

In some embodiments, the solution comprising a multispecific antibody or antibody arm is loaded onto an affinity chromatography (*e.g.*, a Protein A chromatography) at about pH 7 and the multispecific antibody or antibody arm is eluted from the affinity chromatography by a step gradient to pH of about 2.9.

In some aspects of any of the above embodiments, the pH of the elution buffer (such as an elution buffer for the capture chromatography, the first mixed mode chromatography, the second mixed mode chromatography, and/or any additional chromatography, such as anion exchange chromatography, cation exchange chromatography, HIC chromatography, size exclusion chromatography, an additional mixed mode chromatography, etc.) is changed from the load and/or wash buffer by step gradient or by linear gradient.

In some embodiments of any of the methods described herein, the flow rate is less than about any of 50 CV/hr, 40 CV/hr, or 30 CV/hr. The flow rate may be between about any of 5 CV/hr and 50 CV/hr, 10 CV/hr and 40 CV/hr, or 18 CV/hr and 36 CV/hr. In some embodiments, the flow rate is about any of 9 CV/hr, 18 CV/hr, 25 CV/hr, 30 CV/hr, 36 CV/hr, or 40 CV/hr. In some embodiments of any of the methods described herein, the flow rate is less than about any of 100 cm/hr, 75 cm/hr, or 50 cm/hr. The flow rate may be between about any of 25 cm/hr and 150 cm/hr, 25 cm/hr and 100 cm/hr, 50 cm/hr and 100 cm/hr, or 65 cm/hr and 85 cm/hr.

Bed height is the height of chromatography material used. In some embodiments of any of the method described herein, the bed height is greater than about any of 5 cm, 10 cm, 15 cm, 20 cm, 25 cm, 30 cm, 35 cm, 40 cm, 45 cm, or 50 cm. In some embodiments, the bed height is between about 5 cm and 50 cm. In some embodiments, bed height is determined based on the amount of polypeptide or contaminants in the load.

In some embodiments, the chromatography is in a column or vessel with a volume of greater than about 1 mL, 2 mL, 3 mL, 4 mL, 5 mL, 6 mL, 7 mL, 8 mL, 9 mL, 10 mL, 15 mL, 20 mL, 25 mL, 30 mL, 40 mL, 50 mL, 75 mL, 100 mL, 200 mL, 300 mL, 400 mL, 500 mL, 600 mL, 700 mL, 800 mL, 900 mL, 1 L, 2 L, 3 L, 4 L, 5 L, 6 L, 7 L, 8 L, 9 L, 10 L, 25 L, 50 L, 100 L, 200 L, 300 L, 400 L, 500 L, 600 L, 700 L, 800 L, 900 L or 1000 L.

In some embodiments, fractions are collected from the chromatography. In some embodiments, fractions collected are greater than about 0.01 CV, 0.02 CV, 0.03 CV, 0.04 CV, 0.05 CV, 0.06 CV, 0.07 CV, 0.08 CV, 0.09 CV, 0.1 CV, 0.2 CV, 0.3 CV, 0.4 CV, 0.5 CV, 0.6 CV, 0.7 CV, 0.8 CV, 0.9 CV, 1.0 CV, 2.0 CV, 3.0 CV, 4.0 CV, 5.0 CV, 6.0 CV, 7.0 CV, 8.0 CV, 9.0 CV, or 10.0 CV.

In certain embodiments, fractions containing the purified or partially purified product, *e.g.*, the multispecific antibody (such as a bispecific antibody or a divalent F(ab')$_2$) or antibody arm or Fab, are pooled. The amount of polypeptide in a fraction can be determined by one skilled in the art; for example, the amount of polypeptide in a fraction can be determined by UV spectroscopy. In certain embodiments, fractions are collected when the $OD_{280}$ is greater than about any of 0.5, 0.6, 0.7, 0.8, 0.9 and 1.0. In certain embodiments, fractions are collected when the $OD_{280}$ is between about any of 0.5 and 1.0, 0.6 and 1.0, 0.7 and 1.0, 0.8 and 1.0, or 0.9 and 1.0. In certain embodiments, fractions containing detectable multispecific antibody (*e.g.*, bispecific antibody) or antibody arm are pooled.

In certain embodiments of any of the methods described herein, the impurity is a product specific impurity. Examples of product specific impurities include, but are not limited to, unpaired half-antibody, un-paired antibody light chains, unpaired heavy chains, antibody fragments, homodimers (*e.g.*, paired half-dimers of a bispecific antibody that comprise the same heavy and light chain), aggregates, high molecular weight species (MHWS) (such as very high molecular weight species (vHMWS)), multispecific antibodies with mispaired disulfides, light chain dimers, heavy chain dimers, low molecular weight species (LMWS), and charge variants (such as acidic variants and basic variants of the antibody).

In certain embodiments, the methods provided herein remove or reduce the level of unpaired half-antibody from a composition comprising a multispecific antibody (*e.g.,* a bispecific antibody) and unpaired half-antibody. Methods of measuring the presence or level of unpaired half-antibody in a composition are known in the art; for example, by mass spectrometry (such as liquid chromatography-mass spectrometry),CE-SDS, Reverse Phase HPLC, HIC HPLC. In certain embodiments of any of the methods described herein, the amount of unpaired half-antibody in a composition (such as a chromatography fraction) recovered from one or more purification step(s) is reduced by more than about any of 5%, 10 %, 15%, 20 %, 25%, 30 %, 35%, 40 %, 45%, 50 %, 55%, 60 %, 65%, 70 %, 75%, 80 %, 85%, 90 %, 95%, or 99%, including any range in between these values. In certain embodiments, the amount of unpaired half-antibody in a composition (such as a chromatography fraction) recovered from one or more purification step(s) is reduced by between about any of 10 and 95%; 10% and 99%; 20% and 95%; 20% and 99%; 30% and 95%; 30% and 99%; 40% and 95%; 40% and 99%; 50% and 95%; 50% and 99%; 60% and 95%; 60% and 99%; 70% and 95%; 70% and 99%; 80% and 95%; 80% and 99%; 90% and 95%; or 90% and 99%. In some embodiments, the amount of unpaired half-antibody in a composition (such as a chromatography fraction) is reduced by about any of 10 %, 20%, 30%, 40%, 50%, 60%, 70%, 80%, 90%, or 95%. In certain embodiments, the reduction in the presence or level of unpaired half-antibody is determined by comparing the amount of unpaired half-antibody in the composition (such as a chromatography fraction) recovered from a purification step(s) to the amount of unpaired half-antibody in the composition prior to the purification step(s).

In certain embodiments, the methods provided herein remove or reduce the level of homodimer from a composition comprising a multispecific antibody (*e.g.*, a bispecific antibody) and homodimer. Methods of measuring the presence or level of homodimer in a composition are known in the art; for example, by mass spectrometry (such as liquid chromatography-mass spectrometry, Reverse Phase HPLC, and HIC HPLC. In certain embodiments of any of the methods described herein, the amount of homodimer in a composition (such as a chromatography fraction) recovered from one or more purification step(s) is reduced by more than about any of 5%, 10 %, 15%, 20 %, 25%, 30 %, 35%, 40 %, 45%, 50 %, 55%, 60 %, 65%, 70 %, 75%, 80 %, 85%, 90 %, 95%, or 99%, including any range in between these values. In certain embodiments, the amount of homodimer in a composition (such as a chromatography fraction) recovered from one or more purification step(s) is reduced by between about any of 10 and 95%; 10% and 99%; 20% and 95%; 20% and 99%; 30% and 95%; 30% and 99%; 40% and 95%; 40% and 99%; 50% and 95%; 50% and 99%; 60% and 95%; 60% and 99%; 70% and 95%; 70% and 99%; 80% and 95%; 80% and 99%; 90% and 95%; or 90% and 99%. In some embodiments, the amount of homodimer in a composition (such as a chromatography fraction) is reduced by about any of 10 %, 20%, 30%, 40%, 50%, 60%, 70%, 80%, 90%, or 95%. In certain embodiments, the reduction in the presence or level of homodimer is determined by comparing the amount of homodimer in the composition (such as a chromatography fraction) recovered from a purification step(s) to the amount of homodimer in the composition prior to the purification step(s).

In certain embodiments, the methods provided herein remove or reduce the level of high molecular weight species (HMWS) protein from a composition comprising a multispecific antibody (*e.g.*, a bispecific antibody) and HMWS protein. HMWS protein can comprise, *e.g.*, aggregated polypeptide (such as aggregated multispecific antibody, aggregated half antibody, aggregated homodimer, etc.) In certain embodiments, the aggregated polypeptide comprises heavy chain multimers, light chain multimers, and/or multimers of the multispecific antibody. The HMWS protein may be a comprise 2, 3, 4, 5, 6, 7, or 8 or more monomers of a heavy chain or light chain, or 2, 3, 4, 5, 6, 7, or 8 or more aggregated multispecific antibodies. Methods of measuring aggregated protein (*e.g.*, HMWS protein) are known in the art and are described in, *e.g.,* WO 2011/150110. Such methods include, *e.g.,* size exclusion chromatography, capillary electrophoresis-sodium dodecyl sulfate (CE-SDS) and liquid chromatography-mass spectrometry (LC-MS). In certain embodiments of any of the methods described herein, the amount of HMWS protein in a composition (such as a chromatography fraction) recovered from one or more purification step(s) is reduced by more than about any of 5%, 10 %, 15%, 20 %, 25%, 30 %, 35%, 40 %, 45%, 50 %, 55%, 60 %, 65%, 70 %, 75%, 80 %, 85%, 90 %, 95%, or 99%, including any range in between these values. In certain embodiments, the amount of HMWS protein in a composition (such as a chromatography fraction) recovered from one or more purification step(s) is reduced by between about any of 10 and 95%; 10% and 99%; 20% and 95%; 20% and 99%; 30% and 95%; 30% and 99%; 40% and 95%; 40% and 99%; 50% and 95%; 50% and 99%; 60% and 95%; 60% and 99%; 70% and 95%; 70% and 99%; 80% and 95%; 80% and 99%; 90% and 95%; or 90% and 99%. In some embodiments, the amount of HMWS protein in a composition (such as a chromatography fraction) is reduced by about any of 10 %, 20%, 30%, 40%, 50%, 60%, 70%, 80%, 90%, or 95%. In certain embodiments, the reduction in the presence or level of HMWS protein is determined by comparing the amount of HMWS protein in the composition (such as a chromatography fraction) recovered from a purification step(s) to the amount of HMWS protein in the composition prior to the purification step(s).

In certain embodiments, the methods provided herein remove or reduce the level of low molecular weight species (LMWS) protein from a composition comprising a multispecific antibody (*e.g.*, a bispecific antibody) and LMWS protein. LMWS protein can comprise fragmented polypeptide. In certain embodiments, the fragmented polypeptide is a fragment of the multispecific antibody, a fragment of an antibody arm, a heavy chain fragment, or a light chain fragment. Examples of LMWS protein include, but not limited to, a Fab (i.e., *f*ragment *a*ntigen *b*inding), Fc (*f*ragment, *c*rystallizable), regions or combinations of both, or any random fragmented part of a multispecific antibody, heavy chain, or light chain of interest, or ½ antibodies (containing a one antibody light chain/heavy chain pair) or ¾ antibodies (containing a heterodimer or a homodimer of antibody heavy chains and a single antibody light chain; also denoted as HHL herein). Methods of measuring fragmented protein (*e.g.,* LMWS protein) are known in the art and are descried in, *e.g.,* WO 2011/150110. Such methods include, *e.g.,* size exclusion chromatography, capillary electrophoresis-sodium dodecyl sulfate (CE-SDS) and liquid chromatography-mass spectrometry (LC-MS). In certain embodiments of any of the methods described herein, the amount of LMWS protein in a composition (such as a chromatography fraction) recovered from one or more purification step(s) is reduced by more than about any of 5%, 10 %, 15%, 20 %, 25%, 30 %, 35%, 40 %, 45%, 50 %, 55%, 60 %, 65%, 70 %, 75%, 80 %, 85%, 90 %, 95%, or 99%, including any range in between these values. In certain embodiments, the amount of LMWS protein in a composition (such as a chromatography fraction) recovered from one or more purification step(s) is reduced by between about any of 10 and 95%; 10% and 99%; 20% and 95%; 20% and 99%; 30% and 95%; 30% and 99%; 40% and 95%; 40% and 99%; 50% and 95%; 50% and 99%; 60% and 95%; 60% and 99%; 70% and 95%; 70% and 99%; 80% and 95%; 80% and 99%; 90% and 95%; or 90% and 99%. In some embodiments, the amount of LMWS protein in a composition (such as a chromatography fraction) is reduced by about any of 10 %, 20%, 30%, 40%, 50%, 60%, 70%, 80%, 90%, or 95%. In certain embodiments, the reduction in the presence or level of LMWS protein is determined by comparing the amount of LMWS protein in the composition (such as a chromatography fraction) recovered from a purification step(s) to the amount of LMWS protein in the composition prior to the purification step(s).

In certain embodiments, the methods provided herein remove or reduce the level of acidic and/or basic variants from a composition comprising a multispecific antibody (*e.g.*, a bispecific antibody) and acidic and/or basic variants. Acidic variants of an antibody (such as a multispecific antibody, *e.g.,* a bispecific antibody) are variants in which the pI of the antibody is less than the pI of the native intact antibody. Basic variants of an antibody (such as a multispecific antibody, *e.g.*, a bispecific antibody) are variants in which the pI of the antibody is greater that the pI of the native intact antibody. Such charge variants (e.g., acidic and basic variants) may be the result of natural processes such as oxidation, deamidation, C-terminal processing of lysine residues, N-terminal pyroglutamate formation, and glycation of the antibody. Methods of measuring charge variants are known in the art; for example, imaging capillary IsoElectric Focusing (iCIEF). In certain embodiments of any of the methods described herein, the amount of charged variants in a composition (such as a chromatography fraction) recovered from one or more purification step(s) is reduced by more than about any of 5%, 10 %, 15%, 20 %, 25%, 30 %, 35%, 40 %, 45%, 50 %, 55%, 60 %, 65%, 70 %, 75%, 80 %, 85%, 90 %, 95%, or 99%, including any range in between these values. In certain embodiments, the amount of charged variants in a composition (such as a chromatography fraction) recovered from one or more purification step(s) is reduced by between about any of 10 and 95%; 10% and 99%; 20% and 95%; 20% and 99%; 30% and 95%; 30% and 99%; 40% and 95%; 40% and 99%; 50% and 95%; 50% and 99%; 60% and 95%; 60% and 99%; 70% and 95%; 70% and 99%; 80% and 95%; 80% and 99%; 90% and 95%; or 90% and 99%. In some embodiments, the amount of charged variants in a composition (such as a chromatography fraction) is reduced by about any of 10 %, 20%, 30%, 40%, 50%, 60%, 70%, 80%, 90%, or 95%. In certain embodiments, the reduction in the presence or level of charged variants is determined by comparing the amount of charged variants in the composition (such as a chromatography fraction) recovered from a purification step(s) to the amount of charged variants in the composition prior to the purification step(s).

In certain embodiments of any of the methods described herein, the impurity is a process-specific impurity. For example, the process-specific impurity can comprise one or more of: leached Protein A; host cell materials; nucleic acids; other polypeptides; endotoxin; viral contaminants; cell culture media components, carboxypeptidase B, gentamicin, etc. In certain embodiments, the process specific impurity may be a host cell protein (HCP) from, *e.g.,* a prokaryotic cell, a bacterial cell (such as an *E. coli* cell), an insect cell, a eukaryotic cell, a fungal cell, a yeast cell, an avian cell, or a mammalian cell, *e.g.,* a CHO cell.

In certain embodiments, the methods provided herein remove or reduce the level of leached Protein A from a composition comprising a multispecific antibody (*e.g.*, a bispecific antibody) and leached Protein A. Leached Protein A is Protein A detached or washed from a solid phase to which it is bound. For example, leached Protein A can be leached from Protein A chromatography column. The amount of Protein A may be measured, for example, by ELISA, as described in WO 2011/150110. In certain embodiments, the reduction in the presence or level of leached Protein A is determined by comparing the amount of leached Protein A in the composition (such as a chromatography fraction) recovered from a purification step(s) to the amount of leached Protein A in the composition prior to the purification step(s).

In certain embodiments, the methods provided herein remove or reduce the level of host cell proteins (HCP) from a composition comprising a multispecific antibody (*e.g.*, a bispecific antibody) and HCP. HCP are proteins from the host cells in which the multispecific antibody (such as a bispecific antibody) was produced. In certain embodiments, the HCP proteins are proteins from prokaryotic cells. In certain embodiments, the HCP are proteins from *E. coli* cells (*i.e.*, *E. coli* proteins or ECP). Examples of prokaryotic HCP (such as ECP) include, but are not limited to, prokaryotic chaperones such as FkpA, DsbA, and DsbC. In certain embodiments, the HCP are proteins from eukaryotic host cells, such as those described elsewhere herein. In certain embodiments, the HCP are proteins from mammalian cells, such as CHO cell proteins (*i.e.,* Chinese Hamster Ovary Proteins or CHOP). In certain embodiments, the amount of HCP (*e.g.,* ECP, FkpA, DsbA, or DsbC, or, *e.g.,* CHOP) is measured by enzyme-linked immunosorbent assay ("ELISA"). For example, antibodies may be generated against ultrapure compositions of FkpA, DsbA or DsbC. In certain embodiments, the amount of FkpA, DsbA and/or DsbC is determined by mass spectrometry. In some embodiments of any of the methods described herein, the amount of HCP (*e.g.,* ECP, FkpA, DsbA, or DsbC, or, *e.g.,* CHOP). In certain embodiments of any of the methods described herein, the amount of HCP (*e.g.,* ECP, FkpA, DsbA, or DsbC, or, *e.g.,* CHOP) in a composition (such as a chromatography fraction) recovered from one or more purification step(s) is reduced to less than about 100 ppm, 75 ppm, 50 ppm, 25 ppm, 20 ppm, 10 ppm, 5, ppm, 2 ppm, or 1 ppm, including any range in between these values. In some embodiments, the amount of HCP (*e.g.,* ECP, FkpA, DsbA, or DsbC, or, *e.g.,* CHOP) in a composition (such as a chromatography fraction) is reduced to less than about 100 ppm, 75 ppm, 50 ppm, 25 ppm, 20 ppm, 10 ppm, 5, ppm, 2 ppm, or 1 ppm, including any range in between these values. In certain embodiments, the reduction in the presence or level of HCP (*e.g.,* ECP, FkpA, DsbA, or DsbC, or, *e.g.,* CHOP) is determined by comparing the amount of HCP in the composition (such as a chromatography fraction) recovered from a purification step(s) to the amount of HCP in the composition prior to the purification step(s).

In certain embodiments, the methods provided herein remove or reduce the level of nucleic acid (such as host cell DNA and/or RNA) from a composition comprising a multispecific antibody (*e.g.*, a bispecific antibody) and nucleic acid. Methods of measuring nucleic acid (such as host cell DNA and/or RNA) are known in the art and described in, *e.g.,* WO 2011/150110. Such methods include, *e.g.,* PCR for host cell DNA or RNA. In certain embodiments of any of the methods described herein, the amount of nucleic acid in a composition (such as a chromatography fraction) recovered from one or more purification step(s) is reduced by more than about any of 5%, 10 %, 15%, 20 %, 25%, 30 %, 35%, 40 %, 45%, 50 %, 55%, 60 %, 65%, 70 %, 75%, 80 %, 85%, 90 %, 95%, or 99%, including any range in between these values. In certain embodiments, the amount of nucleic acid in a composition (such as a chromatography fraction) recovered from one or more purification step(s) is reduced by between about any of 10 and 95%; 10% and 99%; 20% and 95%; 20% and 99%; 30% and 95%; 30% and 99%; 40% and 95%; 40% and 99%; 50% and 95%; 50% and 99%; 60% and 95%; 60% and 99%; 70% and 95%; 70% and 99%; 80% and 95%; 80% and 99%; 90% and 95%; or 90% and 99%. In some embodiments, the amount of nucleic acid in a composition (such as a chromatography fraction) is reduced by about any of 10 %, 20%, 30%, 40%, 50%, 60%, 70%, 80%, 90%, or 95%. In certain embodiments, the reduction in the presence or level of nucleic acid is determined by comparing the amount of nucleic acid in the composition (such as a chromatography fraction) recovered from a purification step(s) to the amount of nucleic acid in the composition prior to the purification step(s).

In certain embodiments, the methods provided herein remove or reduce the level of a cell culture medium component from a composition comprising a multispecific antibody (*e.g.*, a bispecific antibody) and a cell culture medium component. "Cell culture medium component" refers to a component present in a cell culture medium. In certain embodiments, "cell culture medium" refers to the cell culture medium at the time the host cell(s) expressing the multispecific antibody (*e.g.*, bispecific antibody) or arms thereof are harvested. In certain embodiments, the cell culture medium component is insulin, or tetracycline. In certain embodiments, the amount of insulin, or tetracycline is be measured by ELISA. In certain embodiments of any of the methods described herein, the amount of a cell culture medium component in a composition (such as a chromatography fraction) recovered from one or more purification step(s) is reduced by more than about any of 5%, 10 %, 15%, 20 %, 25%, 30 %, 35%, 40 %, 45%, 50 %, 55%, 60 %, 65%, 70 %, 75%, 80 %, 85%, 90 %, 95%, or 99%, including any range in between these values.

In certain embodiments, the amount of a cell culture medium component in a composition (such as a chromatography fraction) recovered from one or more purification step(s) is reduced by between about any of 10 and 95%; 10% and 99%; 20% and 95%; 20% and 99%; 30% and 95%; 30% and 99%; 40% and 95%; 40% and 99%; 50% and 95%; 50% and 99%; 60% and 95%; 60% and 99%; 70% and 95%; 70% and 99%; 80% and 95%; 80% and 99%; 90% and 95%; or 90% and 99%. In some embodiments, the amount of a cell culture medium component in a composition (such as a chromatography fraction) is reduced by about any of 10 %, 20%, 30%, 40%, 50%, 60%, 70%, 80%, 90%, or 95%. In certain embodiments, the reduction in the presence or level of a cell culture medium component is determined by comparing the amount of the cell culture medium component in the composition (such as a chromatography fraction) recovered from a purification step(s) to the amount of the cell culture medium component in the composition prior to the purification step(s).

In certain embodiments, provided herein is a method of purifying a bispecific antibody comprising a first arm and a second arm, wherein the first and second arms are produced separately, the method comprising: subjecting the first and second arms to capture chromatography (such as any one or combination of capture chromatography steps described elsewhere herein) operated in bind and elute mode to produce first and second capture eluates; forming a mixture comprising the first and second capture eluates under conditions sufficient to produce a composition comprising the multispecific antibody, subjecting the composition comprising the multispecific antibody to anion exchange chromatography (*e.g.*, Q Sepharose® Fast Flow (QSFF) chromatography) in bind and elute mode to produce an anion exchange eluate, wherein the elution a gradient elution; subjecting the anion exchange eluate to anion exchange mixed mode chromatography (*e.g.*, Capto™Adhere chromatography) in bind and elute mode to produce a first mixed mode eluate, wherein the elution is a gradient elution; and subjecting the first mixed mode eluate to cation exchange mixed mode chromatography (*e.g.*, Capto™MMC chromatography) in bind and elute mode to produce a second mixed mode eluate, wherein the elution is a gradient elution, and collecting a fraction comprising the bispecific antibody, wherein the method reduces an amount of an impurity in the fraction relative to the mixture comprising the first and second arms.

In certain embodiments, provided herein is a method of purifying a bispecific antibody comprising a first arm and a second arm, wherein the first and second arms are produced separately, the method comprising: subjecting the first and second arms to capture chromatography (such as any one or combination of capture chromatography steps described elsewhere herein) in bind and elute mode to produce first and second capture eluates; forming a mixture comprising the first and second capture eluates under conditions sufficient to produce a composition comprising the multispecific antibody, subjecting the composition comprising the multispecific antibody to cation exchange mixed mode chromatography (*e.g.*, Capto™MMC chromatography) in bind and elute mode to produce a first mixed mode eluate; wherein the elution is a pH and salt step elution, and subjecting the first mixed mode eluate to anion exchange mixed mode chromatography (*e.g.*, Capto™Adhere chromatography) in flow through mode to produce a second mixed mode eluate, and collecting a fraction comprising the bispecific antibody, wherein the method reduces an amount of an impurity in the fraction relative to the mixture comprising the first and second arms.

In certain embodiments, provided herein is a method of purifying a bispecific antibody comprising a first arm and a second arm, wherein the first and second arms are produced separately, the method comprising: subjecting the first and second arms to capture chromatography (such as any one or combination of capture chromatography steps described elsewhere herein) in bind and elute mode to produce first and second capture eluates; forming a mixture comprising the first and second capture eluates under conditions sufficient to produce a composition comprising the multispecific antibody, subjecting the composition comprising the multispecific antibody to anion exchange mixed mode chromatography (*e.g.*, Capto™Adhere chromatography) in bind and elute mode, wherein the elution is a step elution, to produce a first mixed mode eluate; and subjecting the first mixed mode eluate to cation exchange mixed mode chromatography (*e.g.*, Capto™MMC chromatography) in bind and elute mode to produce a second mixed mode eluate, wherein the elution is a step elution, subjecting the second mixed mode eluate to hydrophobic interaction chromatography (*e.g.*, Hexyl-650C chromatography) in flow through mode to produce a hydrophobic interaction eluate; and collecting a fraction comprising the bispecific antibody, wherein the method reduces an amount of an impurity in the fraction relative to the mixture comprising the first and second arms.

In certain embodiments, provided herein is a method of purifying a bispecific antibody (such as a bispecific F(ab') 2) comprising a first arm and a second arm, wherein the first and second arms are produced separately, the method comprising: subjecting the first arm to capture chromatography (such as any one or combination of capture chromatography steps described elsewhere herein) in bind and elute mode to produce a first capture eluate; subjecting the first capture eluate to cation exchange mixed mode chromatography (*e.g.*, Capto™ MMC chromatography) in bind and elute mode to produce a first mixed mode eluate; subjecting the second arm to capture chromatography (such as any one or combination of capture chromatography steps described elsewhere herein) in bind and elute mode to produce a second capture eluate; forming a mixture comprising the first mixed mode eluate and second capture eluates under conditions sufficient to produce a composition comprising the multispecific antibody, subjecting the composition comprising the multispecific antibody to anion exchange mixed mode chromatography (*e.g.*, such as Capto™ Adhere chromatography) to produce a second mixed mode eluate; and subjecting the second mixed mode eluate to cation exchange chromatography (*e.g.*, such as POROS® 50 HS chromatography) in bind and elute mode to produce a cation exchange eluate; subjecting the cation exchange eluate to subsequent cation exchange mixed mode chromatography in bind and elute mode to produce a third mixed mode eluate; and collecting a fraction comprising the bispecific antibody, wherein the method reduces an amount of an impurity in the fraction relative to the mixture comprising the first and second arms.

In certain embodiments, provided herein is a method of purifying a bispecific antibody (such as a bispecific F(ab')2) comprising a first arm and a second arm, wherein the first and second arms are produced separately, the method comprising: subjecting the first arm to capture chromatography (such as any one or combination of capture chromatography steps described elsewhere herein) in bind and elute mode to produce a first capture eluate; subjecting the first capture eluate to cation exchange mixed mode chromatography (*e.g.*, Capto™ MMC chromatography) in bind and elute mode to produce a first mixed mode eluate; subjecting the second arm to capture chromatography (such as any one or combination of capture chromatography steps described elsewhere herein) in bind and elute mode to produce a second capture eluate; forming a mixture comprising the first mixed mode eluate and second capture eluates under conditions sufficient to produce a composition comprising the multispecific antibody, subjecting the composition comprising the multispecific antibody to anion exchange mixed mode chromatography (*e.g.*, such as Capto™ Adhere chromatography) to produce a second mixed mode eluate; and subjecting the second mixed mode eluate to subsequent cation exchange mixed mode chromatography (*e.g.*, Capto™ MMC chromatography) in bind and elute mode to produce a third mixed mode eluate; and collecting a fraction comprising the bispecific antibody, wherein the method reduces an amount of an impurity in the fraction relative to the mixture comprising the first and second arms.

In some embodiments, the multispecific antibody (such as bispecific antibody) is further purified by viral filtration. Viral filtration is the removal of viral contaminants in a polypeptide purification feedstream. Examples of viral filtration include, *e.g.*, ultrafiltration and microfiltration. In some embodiments the polypeptide is purified using a parvovirus filter.

In some embodiments, the multispecific antibody is concentrated after chromatography *(e.g.,* after the second mixed mode chromatography or after one or more chromatography steps performed following the second mixed mode chromatography). Examples of concentration methods are known in the art and include, but are not limited to, *e.g.*, ultrafiltration and diafiltration (UFDF). In some embodiments, the multispecific antibody is concentrated by a first ultrafiltraton, a diafiltration, and a second ultrafiltration. In some embodiments, the ultrafiltration and/or diafiltration uses a filter with a cut off of less than about any of 5 kDal, 10 kDal, 15 kDal, 20 kDal, or 25 kDal or 30 kDal. In some embodiments, the retentate of the first ultrafiltration is diafiltered into a pharmaceutical formulation.

In some embodiments, the concentration of multispecific antibody following concentration is about any of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, 200 mg/mL, or 300 mg/mL. In some embodiments, the concentration of multispecific antibody is between about any of 10 mg/mL and 20 mg/mL, 20 mg/mL and 30 mg/mL, 30 mg/mL and 40 mg/mL, 40 mg/mL and 50 mg/mL, 50 mg/mL and 60 mg/mL, 60 mg/mL and 70 mg/mL, 70 mg/mL and 80 mg/mL, 80 mg/mL and 90 mg/mL, 90 mg/mL and 100 mg/mL, 100 mg/mL and 110 mg/mL, 110 mg/mL and 120 mg/mL, 120 mg/mL and 130 mg/mL, 130 mg/mL and 140 mg/mL, 140 mg/mL and 150 mg/mL, 150 mg/mL and 160 mg/mL, 160 mg/mL and 170 mg/mL, 170 mg/mL and 180 mg/mL, 180 mg/mL and 190 mg/mL, 190 mg/mL and 200 mg/mL, 200 mg/mL or 300 mg/mL.

In some embodiments of any of the methods described herein, the methods further comprise combining the purified polypeptide of the methods of purification with a pharmaceutically acceptable carrier. In some embodiments, the multispecific antibody is formulated into a pharmaceutical formulation by ultrafiltration/diafiltration.

In certain embodiments, the methods provided herein produce a composition comprising a multispecific antibody that is more than about any of 50%, 55%, 60%, 65%, 70%, 75%, 80%, 85%, 90%, 95% pure. In certain embodiments, the multispecific antibody in the composition is more than about any of 96%, 97%, 98%, or 99% pure.

In certain embodiments, the methods provided herein produce a composition comprising the multispecific antibody contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% unpaired antibody arms. In certain embodiments, the methods provided herein produce a composition comprising the multispecific antibody contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% homodimer. In certain embodiments, the methods provided herein produce a composition comprising the multispecific antibody contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, or 5% aggregated protein. In certain embodiments, the methods provided herein produce a composition comprising the multispecific antibody contains no more than about any of 0.1%, 0.5%, 1%, 5%, 10%, 15%, 20%, 25% 30%, or 35% HMWS. In certain embodiments, the methods provided herein produce a composition comprising the multispecific antibody contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% LMWS. In certain embodiments, the methods provided herein produce a composition comprising the multispecific antibody contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 15%, 20%, 25% 30%, 35%, 40%, 45%, or 50% acidic variants. In certain embodiments, the methods provided herein produce a composition comprising the multispecific antibody contains no more than about any of 0.1%, 0.5%, 1%, 5%, 10%, 15%, 20%, 25% 30%, or 35% basic variants. In certain embodiments, the methods provided herein produce a composition comprising the multispecific antibody contains no more than about any of 0.1 ppm, 0.2 ppm, 0.3 ppm, 0.4 ppm, 0.5 ppm, 0.6 ppm, 0.7 ppm, 0.8 ppm, 0.9 ppm, 1 ppm, 1.5 ppm, 2 ppm, 2.5 ppm, 3 ppm, 3.5 ppm, 4 ppm, 4.5 ppm, 5 ppm, 5.5 ppm, 6 ppm, 6.5 ppm, 7 ppm, 7.5 ppm, 8 ppm, 8.5 ppm, 9 ppm, 9.5 ppm, or 10 ppm leached Protein A. In certain embodiments, the methods provided herein produce a composition comprising the multispecific antibody contains no more than about any of 0.1 ppm, 0.2 ppm, 0.3 ppm, 0.4 ppm, 0.5 ppm, 0.6 ppm, 0.7 ppm, 0.8 ppm, 0.9 ppm, 1 ppm, 1.5 ppm, 2 ppm, 2.5 ppm, 3 ppm, 3.5 ppm, 4 ppm, 4.5 ppm, 5 ppm, 5.5 ppm, 6 ppm, 6.5 ppm, 7 ppm, 7.5 ppm, 8 ppm, 8.5 ppm, 9 ppm, 9.5 ppm, 10 ppm, 15 ppm, 20 ppm, 25 ppm, 30 ppm, or 35 ppm HCP. In certain embodiments, the methods provided herein produce a composition comprising the multispecific antibody contains less than about any of 2 ppm, 2.5 ppm, 3 ppm, 3.5 ppm, 4 ppm, 4.5 ppm, 5 ppm, 5.5 ppm, 6 ppm, 6.5 ppm, 7 ppm, 7.5 ppm, 8 ppm, 8.5 ppm, 9 ppm, 9.5 ppm, or 10 ppm, nucleic acid. In certain embodiments, the composition comprising the multispecific antibody comprises no more than 0 ppm nucleic acid. In certain embodiments, the nucleic acid in the composition comprising the multispecific antibody is below the level of detection. In certain embodiments, the methods provided herein produce a composition comprising the multispecific antibody contains no more than about any of 0.1%, 0.5%, 1%, 5%, 10%, 15%, 20%, 25% 30%, or 35% cell culture medium component.

In certain embodiments, provided is a composition comprising a multispecific antibody purified according to any one of the methods described herein.

In certain embodiments, the multispecific antibody in the composition is more than about any of 50%, 55%, 60%, 65%, 70%, 75%, 80%, 85%, 90%, 95% pure. In certain embodiments, the multispecific antibody in the composition is more than about any of 96%, 97%, 98%, or 99% pure.

In certain embodiments, the composition comprising the multispecific antibody contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% unpaired antibody arms. In certain embodiments, composition comprising the multispecific antibody contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% homodimer. In certain embodiments, composition comprising the multispecific antibody contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, or 5% aggregated protein. In certain embodiments, composition comprising the multispecific antibody contains no more than about any of 0.1%, 0.5%, 1%, 5%, 10%, 15%, 20%, 25% 30%, or 35% HMWS. In certain embodiments, composition comprising the multispecific antibody contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% LMWS. In certain embodiments, composition comprising the multispecific antibody contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 15%, 20%, 25% 30%, 35%, 40%, 45%, or 50% acidic variants. In certain embodiments, composition comprising the multispecific antibody contains no more than about any of 0.1%, 0.5%, 1%, 5%, 10%, 15%, 20%, 25% 30%, or 35% basic variants. In certain embodiments, composition comprising the multispecific antibody contains no more than about any of 0.1 ppm, 0.2 ppm, 0.3 ppm, 0.4 ppm, 0.5 ppm, 0.6 ppm, 0.7 ppm, 0.8 ppm, 0.9 ppm, 1 ppm, 1.5 ppm, 2 ppm, 2.5 ppm, 3 ppm, 3.5 ppm, 4 ppm, 4.5 ppm, 5 ppm, 5.5 ppm, 6 ppm, 6.5 ppm, 7 ppm, 7.5 ppm, 8 ppm, 8.5 ppm, 9 ppm, 9.5 ppm, or 10 ppm leached Protein A. In certain embodiments, composition comprising the multispecific antibody contains no more than about any of 0.1 ppm, 0.2 ppm, 0.3 ppm, 0.4 ppm, 0.5 ppm, 0.6 ppm, 0.7 ppm, 0.8 ppm, 0.9 ppm, 1 ppm, 1.5 ppm, 2 ppm, 2.5 ppm, 3 ppm, 3.5 ppm, 4 ppm, 4.5 ppm, 5 ppm, 5.5 ppm, 6 ppm, 6.5 ppm, 7 ppm, 7.5 ppm, 8 ppm, 8.5 ppm, 9 ppm, 9.5 ppm, 10 ppm, 15 ppm, 20 ppm, 25 ppm, 30 ppm, or 35 ppm HCP. In certain embodiments, composition comprising the multispecific antibody contains no more than about any of 0.1 ppm, 0.2 ppm, 0.3 ppm, 0.4 ppm, 0.5 ppm, 0.6 ppm, 0.7 ppm, 0.8 ppm, 0.9 ppm, 1 ppm, 1.5 ppm, 2 ppm, 2.5 ppm, 3 ppm, 3.5 ppm, 4 ppm, 4.5 ppm, 5 ppm, 5.5 ppm, 6 ppm, 6.5 ppm, 7 ppm, 7.5 ppm, 8 ppm, 8.5 ppm, 9 ppm, 9.5 ppm, 10 ppm, 15 ppm, 20 ppm, 25 ppm, 30 ppm, or 35 ppm nucleic acid. In certain embodiments, composition comprising the multispecific antibody contains no more than about any of 0.1%, 0.5%, 1%, 5%, 10%, 15%, 20%, 25% 30%, or 35% cell culture medium component.

In some embodiments, provided is a composition comprising a multispecific antibody, wherein the composition contains: a) at least about 95% - 100% multispecific antibody; b) less than about 1%- 5% non-paired antibody arms; c) less than about 1%- 5% antibody homodimers; d) no more than about 1% or 2% HMWS; e) no more than about 1% or 2% LMWS; and/or f) no more than about 5% of ¾ antibodies.

In certain embodiments, provided is a composition comprising a bispecific antibody purified according to any one of the methods described herein. In certain embodiments, the bispecific antibody is a knob-in-hole (KiH) antibody, *e.g.*, a KiH bispecific antibody. In some embodiments, the bispecific antibody is a CrossMab bispecific antibody.

In certain embodiments, provided is a composition comprising a bispecific antibody that is more than about any of 50%, 55%, 60%, 65%, 70%, 75%, 80%, 85%, 90%, 95% pure. In certain embodiments, the bispecific antibody in the composition is more than about any of 96%, 97%, 98%, or 99% pure. In certain embodiments, the bispecific antibody is a knob-in-hole (KiH) antibody, *e.g.*, a KiH bispecific antibody. In some embodiments, the bispecific antibody is a CrossMab bispecific antibody.

In certain embodiments, provided is a composition comprising a bispecific antibody that contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% unpaired antibody arms. In certain embodiments, provided is a composition comprising a bispecific antibody that contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% homodimer. In certain embodiments, provided is a composition comprising a bispecific antibody that contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, or 5% aggregated protein. In certain embodiments, provided is a composition comprising a bispecific antibody that contains no more than about any of 0.1%, 0.5%, 1%, 5%, 10%, 15%, 20%, 25% 30%, or 35% HMWS. In certain embodiments, provided is a composition comprising a bispecific antibody that contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or10% LMWS. In certain embodiments, provided is a composition comprising a bispecific antibody that contains no more than about any of 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 15%, 20%, 25% 30%, 35%, 40%, 45%, or 50% acidic variants. In certain embodiments, provided is a composition comprising a bispecific antibody that contains no more than about any of 0.1%, 0.5%, 1%, 5%, 10%, 15%, 20%, 25% 30%, or 35% basic variants. In certain embodiments, provided is a composition comprising a bispecific antibody that contains no more than about any of 0.1 ppm, 0.2 ppm, 0.3 ppm, 0.4 ppm, 0.5 ppm, 0.6 ppm, 0.7 ppm, 0.8 ppm, 0.9 ppm, 1 ppm, 1.5 ppm, 2 ppm, 2.5 ppm, 3 ppm, 3.5 ppm, 4 ppm, 4.5 ppm, 5 ppm, 5.5 ppm, 6 ppm, 6.5 ppm, 7 ppm, 7.5 ppm, 8 ppm, 8.5 ppm, 9 ppm, 9.5 ppm, or 10 ppm leached Protein A. In certain embodiments, provided is a composition comprising a bispecific antibody that contains no more than about any of 0.1 ppm, 0.2 ppm, 0.3 ppm, 0.4 ppm, 0.5 ppm, 0.6 ppm, 0.7 ppm, 0.8 ppm, 0.9 ppm, 1 ppm, 1.5 ppm, 2 ppm, 2.5 ppm, 3 ppm, 3.5 ppm, 4 ppm, 4.5 ppm, 5 ppm, 5.5 ppm, 6 ppm, 6.5 ppm, 7 ppm, 7.5 ppm, 8 ppm, 8.5 ppm, 9 ppm, 9.5 ppm, 10 ppm, 15 ppm, 20 ppm, 25 ppm, 30 ppm, or 35 ppm HCP. In certain embodiments, provided is a composition comprising a bispecific antibody that contains less than about any of 2 ppm, 2.5 ppm, 3 ppm, 3.5 ppm, 4 ppm, 4.5 ppm, 5 ppm, 5.5 ppm, 6 ppm, 6.5 ppm, 7 ppm, 7.5 ppm, 8 ppm, 8.5 ppm, 9 ppm, 9.5 ppm, or 10 ppm, nucleic acid. In certain embodiments, the composition comprising the bispecific antibody comprises no more than 0 ppm nucleic acid. In certain embodiments, the nucleic acid in the composition comprising the bispecific antibody is below the level of detection. In certain embodiments, provided is a composition comprising a bispecific antibody that contains no more than about any of 0.1%, 0.5%, 1%, 5%, 10%, 15%, 20%, 25% 30%, or 35% cell culture medium component. In certain embodiments, the bispecific antibody is a knob-in-hole (KiH) antibody, *e.g.*, a KiH bispecific antibody. In some embodiments, the bispecific antibody is a CrossMab bispecific antibody.

In some embodiments, provided is a composition comprising a bispecific antibody, wherein the composition contains: a) at least about 95% - 100% bispecific antibody; b) less than about 1%- 5% non-paired antibody arms; c) less than about 1%- 5% antibody homodimers; d) no more than about 1% or 2% HMWS; e) no more than about 1% or 2% LMWS; and/or f) no more than about 5% of ¾ antibodies. In certain embodiments, the bispecific antibody is a knob-in-hole (KiH) antibody, *e.g.*, a KiH bispecific antibody. In some embodiments, the bispecific antibody is a CrossMab bispecific antibody.

One aspect as reported herein is a method for purifying an Fc-region containing heterodimeric protein/polypeptide with a multi-step chromatography method wherein the method comprises an affinity chromatography step followed by two different multimodal ion exchange chromatography steps, and thereby purifying the Fc-region containing heterodimeric protein/polypeptide.

In certain embodiments the method comprises i. an affinity chromatography step, followed by a multimodal anion exchange chromatography step, followed by a multimodal cation exchange chromatography step or ii. an affinity chromatography step, followed by a multimodal cation exchange chromatography step, followed by a multimodal anion exchange chromatography step.

One aspect as reported herein is a method for producing an Fc-region containing heterodimeric protein/polypeptide comprising the steps of i. cultivating a cell comprising a nucleic acid encoding the Fc-region containing heterodimeric protein/polypeptide, ii. recovering the Fc-region containing heterodimeric protein/polypeptide from the cell or the cultivation medium, iii. purifying the Fc-region containing heterodimeric protein/polypeptide with a method as reported herein, and thereby producing the Fc-region containing heterodimeric protein. It has been found that the performance of an antibody purification process depends on the sequence of the employed chromatography steps. By choosing a certain sequence/order of the chromatography steps, an improved process can be obtained.

The methods provided herein are based at least in part on the finding that by performing a multimodal anion exchange chromatography step (directly) after the (initial) affinity chromatography step and before a multimodal cation exchange chromatography step, an Ultrafiltration/Diafiltration step can be omitted. This step is necessary if the multimodal cation exchange chromatography step is performed before the multimodal anion exchange chromatography step.

In certain embodiments the multi-step chromatography method comprises an affinity chromatography step, followed by a multimodal anion exchange chromatography step, followed by a multimodal cation exchange chromatography step.

It has been found that with the method described herein, good purity and yield can be achieved with only three chromatography steps.

In certain embodiments the multi-step chromatography method comprises exactly three chromatography steps.

It has been found that the removal of host cell proteins can be improved if the multimodal anion exchange chromatography method/step is performed in flow-through mode. In certain embodiments the multimodal anion exchange chromatography method/step is performed in flow-through mode.

It has been found that the pH of the load of the multimodal anion exchange chromatography step influences the HCP, by-product and DNA removal. In one preferred embodiment of all aspects the multimodal anion exchange chromatography step is performed at a pH of about 7.0.

*Table 1*

| | | Yield | SEC Σ HMW | HCP | Caliper Pre-Peaks | HHL | DNA |
|---|---|---|---|---|---|---|---|
| Chromatography pH | Conductivity [mS/cm] | [%] | [%] | [ng/mg] | [%] | [%] | [pg/mg] |
| 6.5 | 5.75 | 85 | 3.34 | 139 | 8.132 | 2.428 | <0.3 |
| 7.0 | 5.76 | 69 | 1.51 | 55 | 6.186 | 1.830 | <0.4 |
| 7.5 | 6.73 | 67 | 1.23 | 35 | 5.671 | 1.555 | 22.1 |

The conductivity of a solution may have an influence on different parameters during a purification process. Here it has been found that low conductivity values in the load (*i.e.* the solution comprising the Fc-region containing heterodimeric polypeptide which is to be applied to the chromatography material) of the multimodal anion exchange chromatography step lead to improved HCP and DNA removal.

*Table 2*

| Conductivity of load [mS/cm] | HCP [ng/mg] | DNA [pg/mg] |
|---|---|---|
| 16.92 | 303 | 78.6 |
| 5.86 | 35 | 22.1 |
| 3.64 | 14 | 0 |

In certain embodiments in the multimodal anion exchange chromatography step the Fc-region containing heterodimeric polypeptide is applied in a solution with a conductivity value of less than 7 mS/cm. In certain embodiments in the multimodal anion exchange chromatography step the Fc-region containing heterodimeric polypeptide is applied in a solution with a conductivity value of less than 6 mS/cm. In certain embodiments in the multimodal anion exchange chromatography step the Fc-region containing heterodimeric polypeptide is applied in a solution with a conductivity value in the range of about 6 mS/cm to about 2 mS/cm. In certain embodiments in the multimodal anion exchange chromatography step the Fc-region containing heterodimeric polypeptide is applied in a solution with a conductivity value in the range of about 5 mS/cm to about 4 mS/cm. In certain embodiments in the multimodal anion exchange chromatography step the Fc-region containing heterodimeric polypeptide is applied in a solution with a conductivity value of about 4.5 mS/cm.

In certain embodiments in the multimodal anion exchange chromatography step the Fc-region containing heterodimeric polypeptide is applied in a solution with a conductivity of about 4.5 mS/cm and a pH of about 7.

The methods provided herein are based at least in part on the finding that the protein load amount of the multimodal anion exchange chromatography step also influences the performance of the purification process. If the load is in a defined range the overall purification process is improved, e.g. the removal of DNA contamination.

*Table 3: Starting amount of DNA: 80 pg/mg*

| Load of polypeptide per Capto adhere ImpRes material | 220 g/L | 180 g/L | 150 g/L | 120 g/L |
|---|---|---|---|---|
| DNA content after chromatography step | 0.8 pg/mg | 0 pg/mg | 0 pg/mg | 0 pg/mg |

In certain embodiments in the multimodal anion exchange chromatography step the Fc-region containing heterodimeric polypeptide is applied in the range of from about 100 g to about 300 g per liter of chromatography material , *i.e.* the load is in the range of about 100 g/L to about 300 g/L. In certain embodiments in the multimodal anion exchange chromatography step the Fc-region containing heterodimeric polypeptide is applied in the range of from about 120 g to about 240 g per liter of chromatography material. In certain embodiments in the multimodal anion exchange chromatography step the Fc-region containing heterodimeric polypeptide is applied in the range of from about 160 g to about 200 g per liter of chromatography material.

It has been found that certain multimodal resin materials are especially useful when applied in the method as reported herein.

In certain embodiments the multimodal anion exchange chromatography material is a multimodal strong anion exchange chromatography material. In certain embodiments the multimodal anion exchange chromatography material has a matrix of high-flow agarose, a multimodal strong anion exchanger as ligand, an average particle size of 36-44 $\mu$m and an ionic capacity of 0.08 to 0.11 mmol Cl/mL medium. In certain embodiments the multimodal anion exchange chromatography material is "Capto adhere ImpRes".

In certain embodiments the multimodal cation exchange chromatography medium is a multimodal weak cation exchange chromatography medium. In certain embodiments the multimodal cation exchange chromatography medium has a matrix of high-flow agarose, a multimodal weak cation exchanger as ligand, an average particle size of 36-44 $\mu$m and ionic capacity of 25 to 39 $\mu$mol/mL. In certain embodiments the multimodal cation exchange chromatography medium is "Capto MMC ImpRes".

In certain embodiments the multimodal cation exchange chromatography method/step is performed in bind and elute mode.

In certain embodiments the affinity chromatography step is protein A chromatography step or a Protein G affinity chromatography or a single chain Fv ligand affinity chromatography or a chromatography step with KappaSelect chromatography material or a chromatography step with CaptureSelect chromatography material or a chromatography step with CaptureSelect FcXL chromatography material. In certain embodiments the affinity chromatography step is protein A chromatography step. In certain embodiments the affinity chromatography step is a CaptureSelect™ chromatography step. In certain embodiments the affinity chromatography step is a protein A chromatography step.

In certain embodiments the Fc-region containing heterodimeric protein/polypeptide is an antibody, a bispecific antibody or Fc-fusion proteins. In certain embodiments the Fc-region containing heterodimeric protein/polypeptide is a bispecific antibody. In certain embodiments the Fc-region containing heterodimeric protein/polypeptide is a CrossMab. In certain embodiments the Fc-region containing heterodimeric protein/polypeptide is an Fc-fusion protein. In certain embodiments the Fc-region containing heterodimeric protein/polypeptide is a bispecific antibody comprising a) a heavy chain and a light chain of a first full length antibody that specifically binds to a first antigen; and b) a modified heavy chain and a modified light chain of a full length antibody that specifically binds to a second antigen, wherein the constant domains CL and CH1 are replaced by each other.

In certain embodiments the bispecific antibody is a bispecific antibody that binds to ANG2 and VEGF. In certain embodiments the Fc-region containing heterodimeric protein/polypeptide is a CrossMab that binds to ANG2 and VEGF. In certain embodiments the bispecific antibody is vanucizumab.

In certain embodiments the bispecific antibody comprises a first antigen-binding site that comprises as heavy chain variable domain (VH) the SEQ ID NO: 1, and as light chain variable domain (VL) the SEQ ID NO: 2; and a second antigen-binding site that comprises as heavy chain variable domain (VH) the SEQ ID NO: 3, and as light chain variable domain (VL) the SEQ ID NO: 4. In certain embodiments the bispecific antibody comprises a first heavy chain with the amino acid sequence of SEQ ID NO: 9 and a second heavy chain with the amino acid sequence of SEQ ID NO: 10 and a first light chain with the amino acid sequence of SEQ ID NO: 11 and a second light chain with the amino acid sequence of SEQ ID NO: 12. In certain embodiments the bispecific antibody comprises a first antigen-binding site that comprises as heavy chain variable domain (VH) the SEQ ID NO: 5, and as light chain variable domain (VL) the SEQ ID NO: 6; and a second antigen-binding site that comprises as heavy chain variable domain (VH) the SEQ ID NO: 7, and as light chain variable domain (VL) the SEQ ID NO: 8. In certain embodiments the bispecific antibody comprises a first heavy chain with the amino acid sequence of SEQ ID NO: 13 and a second heavy chain with the amino acid sequence of SEQ ID NO: 14 and a first light chain with the amino acid sequence of SEQ ID NO: 15 and a second light chain with the amino acid sequence of SEQ ID NO: 16. The amino acid sequences of SEQ ID NOs: 1-16 are provided in Table 4 below:

**Table 4**

| | |
|---|---|
| SEQ ID NO: 1 | EVQLVESGGG LVQPGGSLRL SCAASGYTFT NYGMNWVRQA PGKGLEWVGW INTYTGEPTY AADFKRRFTF SLDTSKSTAY LQMNSLRAED TAVYYCAKYP HYYGSSHWYF DVWGQGTLVT VSS |
| SEQ ID NO: 2 | DIQMTQSPSS LSASVGDRVT ITCSASQDIS NYLNWYQQKP GKAPKVLIYF TSSLHSGVPS RFSGSGSGTD FTLTISSLQP EDFATYYCQQ YSTVPWTFGQ GTKVEIK |
| SEQ ID NO: 3 | QVQLVQSGAE VKKPGASVKV SCKASGYTFT GYYMHWVRQA PGQGLEWMGW INPNSGGTNY AQKFQGRVTM TRDTSISTAY MELSRLRSDD TAVYYCARSP NPYYYDSSGY YYPGAFDIWG QGTMVTVS |
| SEQ ID NO: 4 | QPGLTQPPSV SVAPGQTARI TCGGNNIGSK SVHWYQQKPG QAPVLVVYDD SDRPSGIPER FSGSNSGNTA TLTISRVEAG DEADYYCQVW DSSSDHYVFG TGTKVTVL |
| SEQ ID NO: 5 | EVQLVESGGG LVQPGGSLRL SCAASGYDFT HYGMNWVRQA PGKGLEWVGW INTYTGEPTY AADFKRRFTF SLDTSKSTAY LQMNSLRAED TAVYYCAKYP YYYGTSHWYF DVWGQGTLVT VSS |
| SEQ ID NO: 6 | DIQLTQSPSS LSASVGDRVT ITCSASQDIS NYLNWYQQKP GKAPKVLIYF TSSLHSGVPS RFSGSGSGTD FTLTISSLQP EDFATYYCQQ YSTVPWTFGQ GTKVEIK |
| SEQ ID NO: 7 | QVQLVQSGAE VKKPGASVKV SCKASGYTFT GYYMHWVRQA PGQGLEWMGW INPNSGGTNY AQKFQGRVTM TRDTSISTAY MELSRLRSDD TAVYYCARSP NPYYYDSSGY YYPGAFDIWG QGTMVTVSS |
| SEQ ID NO: 8 | SYVLTQPPSV SVAPGQTARI TCGGNNIGSK SVHWYQQKPG QAPVLVVYDD SDRPSGIPER FSGSNSGNTA TLTISRVEAG DEADYYCQVW DSSSDHWVFG GGTKLTVLGQ |
| SEQ ID NO: 9 | QVQLVQSGAE VKKPGASVKV SCKASGYTFT GYYMHWVRQA PGQGLEWMGW INPNSGGTNY AQKFQGRVTM TRDTSISTAY MELSRLRSDD TAVYYCARSP NPYYYDSSGY YYPGAFDIWG QGTMVTVSSA SVAAPSVFIF PPSDEQLKSG TASVVCLLNN FYPREAKVQW KVDNALQSGN SQESVTEQDS KDSTYSLSST LTLSKADYEK HKVYACEVTH QGLSSPVTKS FNRGECDKTH TCPPCPAPEL LGGPSVFLFP PKPKDTLMIS RTPEVTCVVV DVSHEDPEVK FNWYVDGVEV HNAKTKPREE QYNSTYRVVS VLTVLHQDWL NGKEYKCKVS NKALPAPIEK TISKAKGQPR EPQVCTLPPS RDELTKNQVS LSCAVKGFYP SDIAVEWESN GQPENNYKTT PPVLDSDGSF FLVSKLTVDK SRWQQGNVFS CSVMHEALHN HYTQKSLSLS PGK |
| SEQ ID NO: 10 | EVQLVESGGG LVQPGGSLRL SCAASGYTFT NYGMNWVRQA PGKGLEWVGW INTYTGEPTY AADFKRRFTF SLDTSKSTAY LQMNSLRAED TAVYYCAKYP HYYGSSHWYF DVWGQGTLVT VSSASTKGPS VFPLAPSSKS TSGGTAALGC LVKDYFPEPV TVSWNSGALT SGVHTFPAVL QSSGLYSLSS VVTVPSSSLG TQTYICNVNH KPSNTKVDKK VEPKSCDKTH TCPPCPAPEL LGGPSVFLFP PKPKDTLMIS RTPEVTCVVV DVSHEDPEVK FNWYVDGVEV HNAKTKPREE QYNSTYRVVS VLTVLHQDWL NGKEYKCKVS NKALPAPIEK TISKAKGQPR EPQVYTLPPC RDELTKNQVS LWCLVKGFYP SDIAVEWESN GQPENNYKTT PPVLDSDGSF FLYSKLTVDK SRWQQGNVFS CSVMHEALHN HYTQKSLSLS PGK |

(continued)

| | |
|---|---|
| SEQ ID NO: 11 | QPGLTQPPSV SVAPGQTARI TCGGNNIGSK SVHWYQQKPG QAPVLVVYDD<br>SDRPSGIPER FSGSNSGNTA TLTISRVEAG DEADYYCQVW DSSSDHYVFG<br>TGTKVTVLSS ASTKGPSVFP LAPSSKSTSG GTAALGCLVK DYFPEPVTVS<br>WNSGALTSGV HTFPAVLQSS GLYSLSSVVT VPSSSLGTQT YICNVNHKPS<br>NTKVDKKVEP KSC |
| SEQ ID NO: 12 | DIQMTQSPSS LSASVGDRVT ITCSASQDIS NYLNWYQQKP GKAPKVLIYF<br>TSSLHSGVPS RFSGSGSGTD FTLTISSLQP EDFATYYCQQ YSTVPWTFGQ<br>GTKVEIKRTV AAPSVFIFPP SDEQLKSGTA SVVCLLNNFY PREAKVQWKV<br>DNALQSGNSQ ESVTEQDSKD STYSLSSTLT LSKADYEKHK VYACEVTHQG<br>LSSPVTKSFN RGEC |
| SEQ ID NO: 13 | EVQLVESGGG LVQPGGSLRL SCAASGYDFT HYGMNWVRQA PGKGLEWVGW<br>INTYTGEPTY AADFKRRFTF SLDTSKSTAY LQMNSLRAED TAVYYCAKYP<br>YYYGTSHWYF DVWGQGTLVT VSSASTKGPS VFPLAPSSKS TSGGTAALGC<br>LVKDYFPEPV TVSWNSGALT SGVHTFPAVL QSSGLYSLSS VVTVPSSSLG<br>TQTYICNVNH KPSNTKVDKK VEPKSCDKTH TCPPCPAPEA AGGPSVFLFP<br>PKPKDTLMAS RTPEVTCVVV DVSHEDPEVK FNWYVDGVEV HNAKTKPREE<br><br>QYNSTYRVVS VLTVLAQDWL NGKEYKCKVS NKALGAPIEK TISKAKGQPR<br>EPQVYTLPPC RDELTKNQVS LWCLVKGFYP SDIAVEWESN GQPENNYKTT<br>PPVLDSDGSF FLYSKLTVDK SRWQQGNVFS CSVMHEALHN AYTQKSLSLS<br>PGK |
| SEQ ID NO: 14 | QVQLVQSGAE VKKPGASVKV SCKASGYTFT GYYMHWVRQA PGQGLEWMGW<br>INPNSGGTNY AQKFQGRVTM TRDTSISTAY MELSRLRSDD TAVYYCARSP<br>NPYYYDSSGY YYPGAFDIWG QGTMVTVSSA SVAAPSVFIF PPSDEQLKSG<br>TASVVCLLNN FYPREAKVQW KVDNALQSGN SQESVTEQDS KDSTYSLSST<br>LTLSKADYEK HKVYACEVTH QGLSSPVTKS FNRGECDKTH TCPPCPAPEA<br>AGGPSVFLFP PKPKDTLMAS RTPEVTCVVV DVSHEDPEVK FNWYVDGVEV<br>HNAKTKPREE QYNSTYRVVS VLTVLAQDWL NGKEYKCKVS NKALGAPIEK<br>TISKAKGQPR EPQVCTLPPS RDELTKNQVS LSCAVKGFYP SDIAVEWESN<br>GQPENNYKTT PPVLDSDGSF FLVSKLTVDK SRWQQGNVFS CSVMHEALHN<br>AYTQKSLSLS PGK |
| SEQ ID NO: 15 | DIQLTQSPSS LSASVGDRVT ITCSASQDIS NYLNWYQQKP GKAPKVLIYF<br>TSSLHSGVPS RFSGSGSGTD FTLTISSLQP EDFATYYCQQ YSTVPWTFGQ<br>GTKVEIKRTV AAPSVFIFPP SDEQLKSGTA SVVCLLNNFY PREAKVQWKV<br>DNALQSGNSQ ESVTEQDSKD STYSLSSTLT LSKADYEKHK VYACEVTHQG<br>LSSPVTKSFN RGEC |
| SEQ ID NO: 16 | SYVLTQPPSV SVAPGQTARI TCGGNNIGSK SVHWYQQKPG QAPVLVVYDD<br>SDRPSGIPER FSGSNSGNTA TLTISRVEAG DEADYYCQVW DSSSDHWVFG<br>GGTKLTVLSS ASTKGPSVFP LAPSSKSTSG GTAALGCLVK DYFPEPVTVS<br>WNSGALTSGV HTFPAVLQSS GLYSLSSVVT VPSSSLGTQT YICNVNHKPS<br>NTKVDKKVEP KSC |

In certain embodiments the purified Fc-region containing heterodimeric polypeptide contains no more than about 5% of ¾ antibodies. In certain embodiments the purified Fc-region containing heterodimeric polypeptide contains no more than about 4% of ¾ antibodies. In certain embodiments the purified Fc-region containing heterodimeric polypeptide contains no more than about 3% of ¾ antibodies. In certain embodiments the purified Fc-region containing heterodimeric polypeptide contains no more than about 2% of ¾ antibodies. In certain embodiments the purified Fc-region containing heterodimeric polypeptide contains no more than about 1% of ¾ antibodies.

One aspect as reported herein is a method for purifying a bispecific antibody that binds to ANG2 and VEGF with a multi-step chromatography method wherein the method comprises an affinity chromatography step, followed by a multimodal anion exchange chromatography step, followed by a multimodal cation exchange chromatography step, and thereby purifying the bispecific antibody that binds to ANG2 and VEGF, wherein bispecific antibody that binds to ANG2 and VEGF comprises a first antigen-binding site that comprises as heavy chain variable domain (VH) the SEQ ID NO: 1, and as light chain variable domain (VL) the SEQ ID NO: 2; and a second antigen-binding site that comprises as heavy chain variable domain (VH) the SEQ ID NO: 3, and as light chain variable domain (VL) the SEQ ID NO: 4 or that comprises a first antigen-binding site that comprises as heavy chain variable domain (VH) the SEQ ID NO: 5, and as light chain variable domain (VL) the SEQ ID NO: 6; and a second antigen-binding site that comprises as heavy chain variable domain (VH) the SEQ ID NO: 7, and as light chain variable domain (VL) the SEQ ID NO: 8.

In one embodiment the bispecific antibody that binds to ANG2 and VEGF comprises a) the heavy chain and the light chain of a first full length antibody that comprises the first antigen-binding site; and b) the modified heavy chain and modified light chain of a full length antibody that comprises the second antigen-binding site, wherein the constant domains CL and CH1 are replaced by each other.

One aspect as reported herein is the use of the method as reported herein for the purification of an Fc-containing heterodimeric polypeptide.

One aspect as reported herein is the use of the method as reported herein for the reduction of Fc-containing heterodimeric polypeptide related impurities.

One aspect as reported herein is an Fc-containing heterodimeric polypeptide obtained with the method as reported herein for the manufacture of a medicament for the treatment of cancer or eye disease.

One aspect as reported herein is an Fc-containing heterodimeric polypeptide obtained with the method as reported herein for use in the treatment of cancer or eye disease.

### *Polypeptides*

### *Monoclonal antibodies*

In some embodiments, antibodies are monoclonal antibodies. Monoclonal antibodies are obtained from a population of substantially homogeneous antibodies, *i.e.,* the individual antibodies comprising the population are identical and/or bind the same epitope except for possible variants that arise during production of the monoclonal antibody, such variants generally being present in minor amounts. Thus, the modifier "monoclonal" indicates the character of the antibody as not being a mixture of discrete or polyclonal antibodies.

For example, the monoclonal antibodies may be made using the hybridoma method first described by Kohler et al., Nature 256:495 (1975), or may be made by recombinant DNA methods (U.S. Patent No. 4,816,567).

In the hybridoma method, a mouse or other appropriate host animal, is immunized as herein described to elicit lymphocytes that produce or are capable of producing antibodies that will specifically bind to the polypeptide used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

In some embodiments, the myeloma cells are those that fuse efficiently, support stable high-level production of antibody by the selected antibody-producing cells, and are sensitive to a medium such as HAT medium. Among these, in some embodiments, the myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol. 133:3001 (1984); Brodeur et al., Monoclonal Antibody Production Techniques and Applications pp. 51-63 (Marcel Dekker, Inc., New York, 1987)).

Culture medium in which hybridoma cells are growing is assayed for production of monoclonal antibodies directed against the antigen. In some embodiments, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem. 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice pp. 59-103 (Academic Press, 1986)). Suitable culture media for this purpose include, for example, D-MEM or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, polypeptide A-Sepharose, hydroxylapatite chromatography, gel electrophoresis, dialysis, affinity chromatography, or ion exchange chromatography.

DNA encoding the monoclonal antibodies is readily isolated and sequenced using conventional procedures (e.g., by using oligonucleotide probes that are capable of binding specifically to genes encoding the heavy and light chains of murine antibodies). In some embodiments, the hybridoma cells serve as a source of such DNA. Once isolated, the DNA may be placed into expression vectors, which are then transfected into host cells such as *E. coli* cells, simian COS cells, human embryonic kidney (HEK) 293 cells, Chinese Hamster Ovary (CHO) cells, or myeloma cells that do not otherwise produce immunoglobulin polypeptide, to obtain the synthesis of monoclonal antibodies in the recombinant host cells. Review articles on recombinant expression in bacteria of DNA encoding the antibody include Skerra et al., Curr. Opinion in Immunol. 5:256-262 (1993) and Plückthun, Immunol. Revs., 130:151-188 (1992).

In a further embodiment, antibodies or antibody fragments can be isolated from antibody phage libraries generated using the techniques described in McCafferty et al., Nature 348:552-554 (1990). Clackson et al., Nature 352:624-628 (1991) and Marks et al., J. Mol. Biol. 222:581-597 (1991) describe the isolation of murine and human antibodies, respectively, using phage libraries. Subsequent publications describe the production of high affinity (nM range) human antibodies by chain shuffling (Marks et al., Bio/Technology 10:779-783 (1992)), as well as combinatorial infection and in vivo recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light chain constant domains in place of the homologous murine sequences (U.S. Patent No. 4,816,567; Morrison et al., Proc. Natl Acad. Sci. USA 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

In some embodiments of any of the methods described herein, the antibody is IgA, IgD, IgE, IgG, or IgM. In some embodiments, the antibody is an IgG monoclonal antibody.

### Antibody fragments

In some embodiments, the antibody is an antibody fragment. Various techniques have been developed for the production of antibody fragments. Traditionally, these fragments were derived via proteolytic digestion of intact antibodies (see, *e.g.,* Morimoto et al., Journal of Biochemical and Biophysical Methods 24:107-117 (1992) and Brennan et al., Science 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')2 fragments (Carter et al., Bio/Technology 10:163-167 (1992)). According to another approach, F(ab')2 fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent No. 5,571,894; and US Patent No. 5,587,458. The antibody fragment may also be a "linear antibody," *e.g.*, as described in US Patent 5,641,870 for example. Such linear antibody fragments may be monospecific or bispecific.

In some embodiments, fragments of the antibodies described herein are provided. In some embodiments, the antibody fragment is an antigen binding fragment. In some embodiments, the antigen binding fragment is selected from the group consisting of a Fab fragment, a Fab' fragment, a F(ab')2 fragment, a scFv, a Fv, and a diabody.

### Polypeptide Variants and Modifications

In certain embodiments, amino acid sequence variants of the proteins herein are contemplated. For example, it may be desirable to improve the binding affinity and/or other biological properties of the protein. Amino acid sequence variants of a protein may be prepared by introducing appropriate modifications into the nucleotide sequence encoding the protein, or by peptide synthesis. Such modifications include, for example, deletions from, and/or insertions into and/or substitutions of residues within the amino acid sequences of the protein. Any combination of deletion, insertion, and substitution can be made to arrive at the final construct, provided that the final construct possesses the desired characteristics.

"Polypeptide variant" means a polypeptide, for example, an active polypeptide, as defined herein having at least about 80% amino acid sequence identity with a full-length native sequence of the polypeptide, a polypeptide sequence lacking the signal peptide, an extracellular domain of a polypeptide, with or without the signal peptide. Such polypeptide variants include, for instance, polypeptides wherein one or more amino acid residues are added, or deleted, at the N or C-terminus of the full-length native amino acid sequence. Ordinarily, a polypeptide variant will have at least about 80% amino acid sequence identity, alternatively at least about any of 85%, 90%, 95%, 96%, 97%, 98%, or 99% amino acid sequence identity, to a full-length native sequence polypeptide sequence, a polypeptide sequence lacking the signal peptide, an extracellular domain of a polypeptide, with or without the signal peptide. Optionally, variant polypeptides will have no more than one conservative amino acid substitution as compared to the native polypeptide sequence, alternatively no more than about any of 2, 3, 4, 5, 6, 7, 8, 9, or 10 conservative amino acid substitution as compared to the native polypeptide sequence.

The variant polypeptide may be truncated at the N-terminus or C-terminus, or may lack internal residues, for example, when compared with a full length native polypeptide. Certain variant polypeptides may lack amino acid residues that are not essential for a desired biological activity. These variant polypeptides with truncations, deletions, and insertions may be prepared by any of a number of conventional techniques. Desired variant polypeptides may be chemically synthesized. Another suitable technique involves isolating and amplifying a nucleic acid fragment encoding a desired variant polypeptide, by polymerase chain reaction (PCR). Oligonucleotides that define the desired termini of the nucleic acid fragment are employed at the 5' and 3' primers in the PCR. Preferably, variant polypeptides share at least one biological and/or immunological activity with the native polypeptide disclosed herein.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme or a polypeptide which increases the serum half-life of the antibody.

For example, it may be desirable to improve the binding affinity and/or other biological properties of the polypeptide. Amino acid sequence variants of the polypeptide are prepared by introducing appropriate nucleotide changes into the antibody nucleic acid, or by peptide synthesis. Such modifications include, for example, deletions from, and/or insertions into and/or substitutions of, residues within the amino acid sequences of the polypeptide. Any combination of deletion, insertion, and substitution is made to arrive at the final construct, provided that the final construct possesses the desired characteristics. The amino acid changes also may alter post-translational processes of the polypeptide (e.g., antibody), such as changing the number or position of glycosylation sites.

Guidance in determining which amino acid residue may be inserted, substituted or deleted without adversely affecting the desired activity may be found by comparing the sequence of the polypeptide with that of homologous known polypeptide molecules and minimizing the number of amino acid sequence changes made in regions of high homology.

A useful method for identification of certain residues or regions of the polypeptide (*e.g.,* antibody) that are preferred locations for mutagenesis is called "alanine scanning mutagenesis" as described by Cunningham and Wells, Science 244:1081-1085 (1989). Here, a residue or group of target residues are identified (e.g., charged residues such as Arg, Asp, His, Lys, and Glu) and replaced by a neutral or negatively charged amino acid (most preferably Alanine or Polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed antibody variants are screened for the desired activity.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. If such substitutions result in a change in biological activity, then more substantial changes, denominated "exemplary substitutions" in the Table 5, or as further described below in reference to amino acid classes, may be introduced and the products screened.

*Table 5*

| Original Residue | Exemplary Substitutions | Conservative Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |

(continued)

| Original Residue | Exemplary Substitutions | Conservative Substitutions |
|---|---|---|
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in the biological properties of the polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, Biochemistry second ed., pp. 73-75, Worth Publishers, New York (1975)):

(1) non-polar: Ala (A), Val (V), Leu (L), Ile (I), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the polypeptide to improve its stability (particularly where the antibody is an antibody fragment such as an Fv fragment).

One example of substitutional variant involves substituting one or more hypervariable region residues of a parent antibody (e.g., a humanized antibody). Generally, the resulting variant(s) selected for further development will have improved biological properties relative to the parent antibody from which they are generated. A convenient way for generating such substitutional variants involves affinity maturation using phage display. Briefly, several hypervariable region sites (e.g., 6-7 sites) are mutated to generate all possible amino substitutions at each site. The antibody variants thus generated are displayed in a monovalent fashion from filamentous phage particles as fusions to the gene **III** product of M13 packaged within each particle. The phage-displayed variants are then screened for their biological activity (e.g., binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and target. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the polypeptide alters the original glycosylation pattern of the antibody. The polypeptide may comprise non-amino acid moieties. For example, the polypeptide may be glycosylated. Such glycosylation may occur naturally during expression of the polypeptide in the host cell or host organism, or may be a deliberate modification arising from human intervention. By altering is meant deleting one or more carbohydrate moieties found in the polypeptide, and/or adding one or more glycosylation sites that are not present in the polypeptide.

Glycosylation of polypeptide is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be used.

Addition of glycosylation sites to the polypeptide is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Removal of carbohydrate moieties present on the polypeptide may be accomplished chemically or enzymatically or by mutational substitution of codons encoding for amino acid residues that serve as targets for glycosylation. Enzymatic cleavage of carbohydrate moieties on polypeptides can be achieved by the use of a variety of endo- and exo-glycosidases.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the γ-amino groups of lysine, arginine, and histidine side chains, acetylation of the N-terminal amine, and amidation of any C-terminal carboxyl group.

### Chimeric Polypeptides

The polypeptide described herein may be modified in a way to form chimeric molecules comprising the polypeptide fused to another, heterologous polypeptide or amino acid sequence. In some embodiments, a chimeric molecule comprises a fusion of the polypeptide with a tag polypeptide which provides an epitope to which an anti-tag antibody can selectively bind. The epitope tag is generally placed at the amino- or carboxyl-terminus of the polypeptide. The presence of such epitope-tagged forms of the polypeptide can be detected using an antibody against the tag polypeptide. Also, provision of the epitope tag enables the polypeptide to be readily purified by affinity purification using an anti-tag antibody or another type of affinity matrix that binds to the epitope tag.

### Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for c-met and the other is for any other antigen. In certain embodiments, bispecific antibodies may bind to two different epitopes of c-met. Bispecific antibodies may also be used to localize cytotoxic agents to cells which express c-met. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983), WO 93/08829, and Traunecker et al., EMBO J. 10: 3655

(1991)), and "knob-in-hole" engineering (see, *e.g.,* U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, *e.g.,* US Patent No. 4,676,980, and Brennan et al., Science, 229: 81 (1985)); using leucine zippers to produce bi-specific antibodies (see, *e.g.,* Kostelny et al., J. Immunol., 148(5):1547-1553 (1992)); using "diabody" technology for making bispecific antibody fragments (see, *e.g.,* Hollinger et al., Proc. Natl. Acad. Sci. USA, 90:6444-6448 (1993)); and using single-chain Fv (sFv) dimers (see, *e.g.* Gruber et al., J. Immunol., 152:5368

(1994)); and preparing trispecific antibodies as described, *e.g.,* in Tutt et al. J. Immunol. 147: 60 (1991).

The antibody or fragment herein also includes multispecific antibodies described in WO 2009/080251, WO 2009/080252, WO 2009/080253, WO 2009/080254, WO 2010/112193, WO 2010/115589, WO 2010/136172, WO 2010/145792, and WO 2010/145793.

Engineered antibodies with three or more functional antigen binding sites, including "Octopus antibodies," are also included herein (see, *e.g.* US 2006/0025576A1).

The antibody or fragment herein also includes a "Dual Acting Fab" or "Dual Action Fab" (DAF) comprising an antigen binding site that binds to a first epitope *(e.g.,* on a first antigen) as well as another, different epitope *(e.g.,* on the first antigen or on a second, different antigen) *(see, e.g.,* US 2008/0069820; Bostrom et al. (2009) Science, 5921, 1610-1614).

Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1), containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

### Knobs in Holes Technology

According to another approach, the interface between a pair of antibody molecules can be engineered to maximize the percentage of heterodimers which are recovered from recombinant cell culture. The preferred interface comprises at least a part of the CH3 domain of an antibody constant domain. In this method, one or more small amino acid side chains from the interface of the first antibody molecule are replaced with larger side chains (e.g., tyrosine or tryptophan) (knobs or protuberances). Compensatory "cavities" (holes) of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers. Knobs and holes are further described herein.

The use of knobs into holes as a method of producing multispecific antibodies and/or one-armed antibodies and/or immunoadhesins is well known in the art. See US Pat. No. 5,731,168 granted 24 March 1998 assigned to Genentech, PCT Pub. No. WO2009089004 published 16 July 2009 and assigned to Amgen, and US Pat. Pub. No. 20090182127 published 16 July 2009 and assigned to Novo Nordisk A/S. See also Marvin and Zhu, Acta Pharmacologica Sincia (2005) 26(6):649-658 and Kontermann (2005) Acta Pharacol. Sin., 26:1-9. A brief discussion is provided here.

A "protuberance" refers to at least one amino acid side chain which projects from the interface of a first polypeptide and is therefore positionable in a compensatory cavity in the adjacent interface (*i.e.* the interface of a second polypeptide) so as to stabilize the heteromultimer, and thereby favor heteromultimer formation over homomultimer formation, for example. The protuberance may exist in the original interface or may be introduced synthetically (e.g. by altering nucleic acid encoding the interface). Normally, nucleic acid encoding the interface of the first polypeptide is altered to encode the protuberance. To achieve this, the nucleic acid encoding at least one "original" amino acid residue in the interface of the first polypeptide is replaced with nucleic acid encoding at least one "import" amino acid residue which has a larger side chain volume than the original amino acid residue. It will be appreciated that there can be more than one original and corresponding import residue. The upper limit for the number of original residues which are replaced is the total number of residues in the interface of the first polypeptide. The side chain volumes of the various amino residues are shown in the following table.

*Table 6: Properties of Amino Acids*

| Amino Acid | One-Letter Abbreviation | MASS[a] (daltons) | VOLUME[b] (Angstrom$^3$) | Accessible Surface Area[c] (Angstrom$^2$) |
|---|---|---|---|---|
| Alanine (Ala) | A | 71.08 | 88.6 | 115 |
| Arginine (Arg) | R | 156.20 | 173.4 | 225 |
| Asparagine (Asn) | N | 114.11 | 117.7 | 160 |
| Aspartic acid (Asp) | D | 115.09 | 111.1 | 150 |
| Cysteine (Cys) | C | 103.14 | 108.5 | 135 |
| Glutamine (Gln) | Q | 128.14 | 143.9 | 180 |
| Glutamic acid (Glu) | E | 129.12 | 138.4 | 190 |
| Glycine (Gly) | G | 57.06 | 60.1 | 75 |
| Histidine (His) | H | 137.15 | 153.2 | 195 |
| Isoleucine (Ile) | I | 113.17 | 166.7 | 175 |
| Leucine (Leu) | L | 113.17 | 166.7 | 170 |
| Lysine (Lys) | K | 128.18 | 168.6 | 200 |
| Methionine (Met) | M | 131.21 | 162.9 | 185 |
| Phenylalinine (Phe) | F | 147.18 | 189.9 | 210 |
| Proline (Pro) | P | 97.12 | 122.7 | 145 |
| Serine (Ser) | S | 87.08 | 89.0 | 115 |
| Threonine (Thr) | T | 101.11 | 116.1 | 140 |
| Tryptophan (Trp) | W | 186.21 | 227.8 | 255 |
| Tyrosine (Tyr) | Y | 163.18 | 193.6 | 230 |
| Valine (Val) | V | 99.14 | 140.0 | 155 |

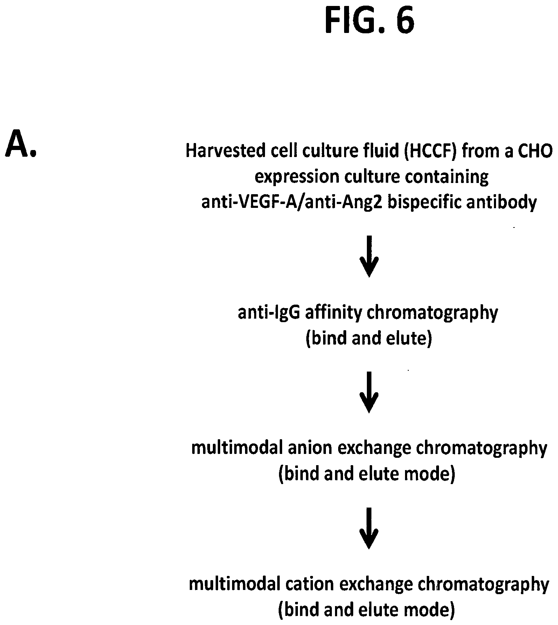

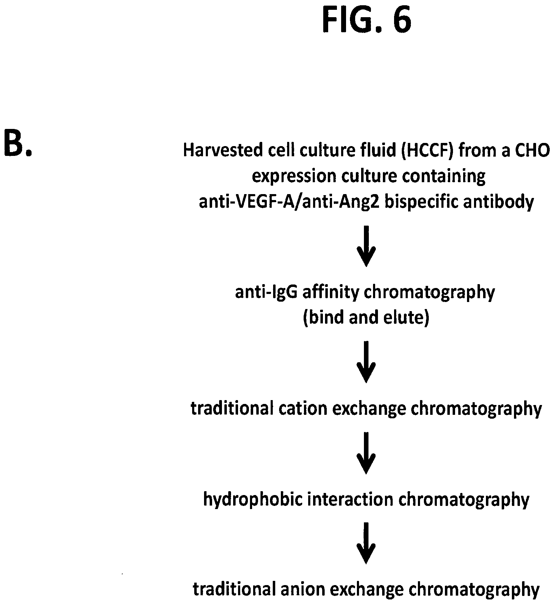

[a]Molecular weight amino acid minus that of water. Values from Handbook of Chemistry and Physics, 43rd ed. Cleveland, Chemical Rubber Publishing Co., 1961.
[b]Values from A.A. Zamyatnin, Prog. Biophys. Mol. Biol. 24:107-123, 1972.
[c]Values from C. Chothia, J. Mol. Biol. 105:1-14, 1975. The accessible surface area is defined in Figures 6-20 of this reference.

The preferred import residues for the formation of a protuberance are generally naturally occurring amino acid residues and are preferably selected from arginine (R), phenylalanine (F), tyrosine (Y) and tryptophan (W). Most preferred are tryptophan and tyrosine. In one embodiment, the original residue for the formation of the protuberance has a small side chain volume, such as alanine, asparagine, aspartic acid, glycine, serine, threonine or valine. Exemplary amino acid substitutions in the CH3 domain for forming the protuberance include without limitation the T366W substitution.

A "cavity" refers to at least one amino acid side chain which is recessed from the interface of a second polypeptide and therefore accommodates a corresponding protuberance on the adjacent interface of a first polypeptide. The cavity may exist in the original interface or may be introduced synthetically (e.g. by altering nucleic acid encoding the interface). Normally, nucleic acid encoding the interface of the second polypeptide is altered to encode the cavity. To achieve this, the nucleic acid encoding at least one "original" amino acid residue in the interface of the second polypeptide is replaced with DNA encoding at least one "import" amino acid residue which has a smaller side chain volume than the original amino acid residue. It will be appreciated that there can be more than one original and corresponding import residue. The upper limit for the number of original residues which are replaced is the total number of residues in the interface of the second polypeptide. The side chain volumes of the various amino residues are shown in Table 2 above. The preferred import residues for the formation of a cavity are usually naturally occurring amino acid residues and are preferably selected from alanine (A), serine (S), threonine (T) and valine (V). Most preferred are serine, alanine or threonine. In one embodiment, the original residue for the formation of the cavity has a large side chain volume, such as tyrosine, arginine, phenylalanine or tryptophan. Exemplary amino acid substitutions in the CH3 domain for generating the cavity include without limitation the T366S, L368A and Y407A substitutions.

An "original" amino acid residue is one which is replaced by an "import" residue which can have a smaller or larger side chain volume than the original residue. The import amino acid residue can be a naturally occurring or non-naturally occurring amino acid residue, but preferably is the former. "Naturally occurring" amino acid residues are those residues encoded by the genetic code and listed in Table 2 above. By "non-naturally occurring" amino acid residue is meant a residue which is not encoded by the genetic code, but which is able to covalently bind adjacent amino acid residue(s) in the polypeptide chain. Examples of non-naturally occurring amino acid residues are norleucine, ornithine, norvaline, homoserine and other amino acid residue analogues such as those described in Ellman et al., Meth. Enzym. 202:301-336 (1991), for example. To generate such non-naturally occurring amino acid residues, the procedures of Noren et al. Science 244: 182 (1989) and Ellman *et al., supra can* be used. Briefly, this involves chemically activating a suppressor tRNA with a non-naturally occurring amino acid residue followed by in vitro transcription and translation of the RNA. The methods provided herein involve replacing at least one original amino acid residue, but more than one original residue can be replaced. Normally, no more than the total residues in the interface of the first or second polypeptide will comprise original amino acid residues which are replaced. Typically, original residues for replacement are "buried". By "buried" is meant that the residue is essentially inaccessible to solvent. Generally, the import residue is not cysteine to prevent possible oxidation or mispairing of disulfide bonds.

The protuberance is "positionable" in the cavity which means that the spatial location of the protuberance and cavity on the interface of a first polypeptide and second polypeptide respectively and the sizes of the protuberance and cavity are such that the protuberance can be located in the cavity without significantly perturbing the normal association of the first and second polypeptides at the interface. Since protuberances such as Tyr, Phe and Trp do not typically extend perpendicularly from the axis of the interface and have preferred conformations, the alignment of a protuberance with a corresponding cavity relies on modeling the protuberance/cavity pair based upon a three-dimensional structure such as that obtained by X-ray crystallography or nuclear magnetic resonance (NMR). This can be achieved using widely accepted techniques in the art.

By "original or template nucleic acid" is meant the nucleic acid encoding a polypeptide of interest which can be "altered" (*i.e.* genetically engineered or mutated) to encode a protuberance or cavity. The original or starting nucleic acid may be a naturally occurring nucleic acid or may comprise a nucleic acid which has been subjected to prior alteration (e.g. a humanized antibody fragment). By "altering" the nucleic acid is meant that the original nucleic acid is mutated by inserting, deleting or replacing at least one codon encoding an amino acid residue of interest. Normally, a codon encoding an original residue is replaced by a codon encoding an import residue. Techniques for genetically modifying a DNA in this manner have been reviewed in Mutagenesis: a Practical Approach, M.J. McPherson, Ed., (IRL Press, Oxford, UK. (1991), and include site-directed mutagenesis, cassette mutagenesis and polymerase chain reaction (PCR) mutagenesis, for example. By mutating an original/template nucleic acid, an original/template polypeptide encoded by the original/template nucleic acid is thus correspondingly altered..

The protuberance or cavity can be "introduced" into the interface of a first or second polypeptide by synthetic means, *e.g.* by recombinant techniques, in vitro peptide synthesis, those techniques for introducing non-naturally occurring amino acid residues previously described, by enzymatic or chemical coupling of peptides or some combination of these techniques. Accordingly, the protuberance or cavity which is "introduced" is "non-naturally occurring" or "non-native", which means that it does not exist in nature or in the original polypeptide (e.g. a humanized monoclonal antibody).

Generally, the import amino acid residue for forming the protuberance has a relatively small number of "rotamers" (e.g. about 3-6). A "rotamer" is an energetically favorable conformation of an amino acid side chain. The number of rotamers of the various amino acid residues is reviewed in Ponders and Richards, J. Mol. Biol. 193: 775-791 (1987).

In one embodiment, a first Fc polypeptide and a second Fc polypeptide meet/interact at an interface. In some embodiments wherein the first and second Fc polypeptides meet at an interface, the interface of the second Fc polypeptide (sequence) comprises a protuberance (also termed a "knob") which is positionable in a cavity (also termed a "hole") in the interface of the first Fc polypeptide (sequence). In one embodiment, the first Fc polypeptide has been altered from a template/original polypeptide to encode the cavity or the second Fc polypeptide has been altered from a template/original polypeptide to encode the protuberance, or both. In one embodiment, the first Fc polypeptide has been altered from a template/original polypeptide to encode the cavity and the second Fc polypeptide has been altered from a template/original polypeptide to encode the protuberance. In one embodiment, the interface of the second Fc polypeptide comprises a protuberance which is positionable in a cavity in the interface of the first Fc polypeptide, wherein the cavity or protuberance, or both, have been introduced into the interface of the first and second Fc polypeptides, respectively. In some embodiments wherein the first and second Fc polypeptides meet at an interface, the interface of the first Fc polypeptide (sequence) comprises a protuberance which is positionable in a cavity in the interface of the second Fc polypeptide (sequence). In one embodiment, the second Fc polypeptide has been altered from a template/original polypeptide to encode the cavity or the first Fc polypeptide has been altered from a template/original polypeptide to encode the protuberance, or both. In one embodiment, the second Fc polypeptide has been altered from a template/original polypeptide to encode the cavity and the first Fc polypeptide has been altered from a template/original polypeptide to encode the protuberance. In one embodiment, the interface of the first Fc polypeptide comprises a protuberance which is positionable in a cavity in the interface of the second Fc polypeptide, wherein the protuberance or cavity, or both, have been introduced into the interface of the first and second Fc polypeptides, respectively.

In one embodiment, the protuberance and cavity each comprise a naturally occurring amino acid residue. In one embodiment, the Fc polypeptide comprising the protuberance is generated by replacing an original residue from the interface of a template/original polypeptide with an import residue having a larger side chain volume than the original residue. In one embodiment, the Fc polypeptide comprising the protuberance is generated by a method comprising a step wherein polynucleotide encoding an original residue from the interface of said polypeptide is replaced with polynucleotide encoding an import residue having a larger side chain volume than the original. In one embodiment, the original residue is threonine. In one embodiment, the original residue is T366. In one embodiment, the import residue is arginine (R). In one embodiment, the import residue is phenylalanine (F). In one embodiment, the import residue is tyrosine (Y). In one embodiment, the import residue is tryptophan (W). In one embodiment, the import residue is R, F, Y or W. In one embodiment, a protuberance is generated by replacing two or more residues in a template/original polypeptide. In one embodiment, the Fc polypeptide comprising a protuberance comprises replacement of threonine at position 366 with tryptophan, amino acid numbering according to the EU numbering scheme of Kabat et al. (pp. 688-696 in Sequences of proteins of immunological interest, 5th ed., Vol. 1 (1991; NIH, Bethesda, MD)).

In some embodiments, the Fc polypeptide comprising a cavity is generated by replacing an original residue in the interface of a template/original polypeptide with an import residue having a smaller side chain volume than the original residue. For example, the Fc polypeptide comprising the cavity may be generated by a method comprising a step wherein polynucleotide encoding an original residue from the interface of said polypeptide is replaced with polynucleotide encoding an import residue having a smaller side chain volume than the original. In one embodiment, the original residue is threonine. In one embodiment, the original residue is leucine. In one embodiment, the original residue is tyrosine. In one embodiment, the import residue is not cysteine (C). In one embodiment, the import residue is alanine (A). In one embodiment, the import residue is serine (S). In one embodiment, the import residue is threonine (T). In one embodiment, the import residue is valine (V). A cavity can be generated by replacing one or more original residues of a template/original polypeptide. For example, in one embodiment, the Fc polypeptide comprising a cavity comprises replacement of two or more original amino acids selected from the group consisting of threonine, leucine and tyrosine. In one embodiment, the Fc polypeptide comprising a cavity comprises two or more import residues selected from the group consisting of alanine, serine, threonine and valine. In some embodiments, the Fc polypeptide comprising a cavity comprises replacement of two or more original amino acids selected from the group consisting of threonine, leucine and tyrosine, and wherein said original amino acids are replaced with import residues selected from the group consisting of alanine, serine, threonine and valine. In some embodiments, an original amino acid that is replaced is T366, L368 and/or Y407. In one embodiment, the Fc polypeptide comprising a cavity comprises replacement of threonine at position 366 with serine, amino acid numbering according to the EU numbering scheme of Kabat *et al. supra.* In one embodiment, the Fc polypeptide comprising a cavity comprises replacement of leucine at position 368 with alanine, amino acid numbering according to the EU numbering scheme of Kabat *et al. supra.* In one embodiment, the Fc polypeptide comprising a cavity comprises replacement of tyrosine at position 407 with valine, amino acid numbering according to the EU numbering scheme of Kabat *et al. supra.* In one embodiment, the Fc polypeptide comprising a cavity comprises two or more amino acid replacements selected from the group consisting of T366S, L368A and Y407V, amino acid numbering according to the EU numbering scheme of Kabat *et al. supra.* In some embodiments of these antibody fragments, the Fc polypeptide comprising the protuberance comprises replacement of threonine at position 366 with tryptophan, amino acid numbering according to the EU numbering scheme of Kabat *et al. supra.*

In one embodiment, the antibody comprises Fc mutations constituting "knobs" and "holes" as described in WO2005/063816. For example, a hole mutation can be one or more of T366A, L368A and/or Y407V in an Fc polypeptide, and a knob mutation can be T366W in an IgGlor IgG4 backbone. Equivalent mutations in other immunoglobulin isotypes can be made by one skilled in the art. Further, the skilled artisan will readily appreciate that it is preferred that the two half-antibodies used for the bispecific be the same isotype.

### CrossMab Technology

Schaefer *et al.* (Roche Diagnostics GmbH), describe a method to express two heavy and two light chains, derived from two existing antibodies, as human bivalent bispecific IgG antibodies without use of artificial linkers (PNAS (2011) 108(27): 11187-11192 and US 2009/0232811). The method involves exchanging one or more heavy chain and light chain domains within the antigen-binding fragment (Fab) of one half of the bi-specific antibody (CrossMab). Correct association of the light chains and their cognate heavy chains is achieved by exchange of heavy-chain and light-chain domains within the antigen binding fragment (Fab) of one half of the bispecific antibody. This "crossover" retains the antigen-binding affinity but makes the two arms so different that light-chain mispairing can no longer occur. See WO2009/080251, WO2009/080252, WO2009/080253, WO2009/080254, WO 2010/115589, WO 2010/136172, WO 2010/145792, and WO 2010/145793, each incorporated herein by reference in its entirety. Despite these recent advantages e.g. due to development of methodologies like "knobs into holes (KiH) or the "CrossMab" technology the expression of multispecific antibodies may still lead to undesired formation of product-specific impurities specifically associated with their production. These product-specific impurities for example may include ½ antibodies (comprising a single heavy-chain/light-chain pair), ¾ antibodies (comprising a complete antibody lacking a single light chain) or a $^5/_4$ antibody by-product (comprising an additional heavy or light chain variable domain).

### BiTE Technology

Another format, used for Bispecific T cell Engager (BiTE) molecules (see, *e.g.,* Wolf et al. (2005) Drug Discovery Today 10:1237-1244)), is based on single chain variable fragment (scFv) modules. An scFv consists of an antibody's light and heavy chain variable regions fused via a flexible linker, which generally can fold appropriately and so that the regions can bind the cognate antigen. A BiTE concatenates two scFv's of different specificities in tandem on a single chain. This configuration precludes the production of molecules with two copies of the same heavy chain variable region. In addition, the linker configuration is designed to ensure correct pairing of the respective light and heavy chains.

### Other Bispecific Antibody Formats

Strop et al. (Rinat-Pfizer Inc.), describe a method of producing stable bi-specific antibodies by expressing and purifying two antibodies of interest separately, and then mixing them together under specified redox conditions (J. Mol. Biol. (2012) 420:204-19).

Other heterodimerization domain having a strong preference for forming heterodimers over homodimers can be incorporated into the instant multispecific antigen-binding proteins. Illustrative examples include but are not limited to, for example, WO2007147901 (Kjærgaard et al. - Novo Nordisk: describing ionic interactions); WO 2009089004 (Kannan et al. - Amgen: describing electrostatic steering effects); WO 2010/034605 (Christensen et al. - Genentech; describing coiled coils). See also, for example, Pack, P. & Plueckthun, A., Biochemistry 31, 1579-1584 (1992) describing leucine zipper or Pack et al., Bio/Technology 11, 1271-1277 (1993) describing the helix-turn-helix motif. The phrase "heteromultimerization domain" and "heterodimerization domain" are used interchangeably herein. In certain embodiments, the multispecific antigen-binding protein comprises one or more heterodimerization domains.

Zhu et al. (Genentech) have engineered mutations in the VL/VH interface of a diabody construct consisting of variant domain antibody fragments completely devoid of constant domains, and generated a heterodimeric diabody (Protein Science (1997) 6:781-788). Similarly, Igawa et al. (Chugai) have also engineered mutations in the VL/VH interface of a single-chain diabody to promote selective expression and inhibit conformational isomerization of the diabody (Protein Engineering, Design & Selection (2010) 23:667-677).

US Patent Publication No. 2009/0182127 (Novo Nordisk, Inc.) describes the generation of bi-specific antibodies by modifying amino acid residues at the Fc interface and at the CH1:CL interface of light-heavy chain pairs that reduce the ability of the light chain of one pair to interact with the heavy chain of the other pair.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229: 81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then reoxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy chain variable domain (V H) connected to a light chain variable domain (V L) by a linker that is too short to allow pairing between the two domains on the same chain. Accordingly, the VH and VL domains of one fragment are forced to pair with the complementary V L and V H domains of another fragment, thereby forming two antigen-binding sites. Another strategy for making bispecific antibody fragments by the use of single-chain Fv (scFv) dimers has also been reported. *See* Gruber et al., J. Immunol. 152:5368 (1994).

Reviews of various bispecific and multispecific antibody formats are provided in Klein et al., (2012) mAbs 4:6, 653-663; Spiess et al. (2015) "Alternative molecular formats and therapeutic applications for bispecific antibodies." Mol. Immunol. Published online January 27, 2015; doi:10.1016/j.molimm.2015.01.003; and Kontermann et al. (2015) Drug Discovery Today 20, 838-847.

### Polynucleotides, Vectors, Host Cells, and Recombinant Methods

The multispecific antibodies used in the methods of purification described herein may be obtained using methods well-known in the art, including the recombination methods. The following sections provide guidance regarding these methods.

### Polynucleotides

"Polynucleotide," or "nucleic acid," as used interchangeably herein, refer to polymers of nucleotides of any length, and include DNA and RNA.

Polynucleotides encoding polypeptides may be obtained from any source including, but not limited to, a cDNA library prepared from tissue believed to possess the polypeptide mRNA and to express it at a detectable level. Accordingly, polynucleotides encoding polypeptide can be conveniently obtained from a cDNA library prepared from human tissue. The polypeptide-encoding gene may also be obtained from a genomic library or by known synthetic procedures (e.g., automated nucleic acid synthesis).

For example, the polynucleotide may encode an entire immunoglobulin molecule chain, such as a light chain or a heavy chain. A complete heavy chain includes not only a heavy chain variable region ($V_H$) but also a heavy chain constant region ($C_H$), which typically will comprise three constant domains: $C_H1$, $C_H2$ and $C_H3$; and a "hinge" region. In some situations, the presence of a constant region is desirable.

Other polypeptides which may be encoded by the polynucleotide include antigen-binding antibody fragments such as single domain antibodies ("dAbs"), Fv, scFv, Fab' and F(ab')$_2$ and "minibodies." Minibodies are (typically) bivalent antibody fragments from which the $C_H1$ and $C_K$ or $C_L$ domain has been excised. As minibodies are smaller than conventional antibodies they should achieve better tissue penetration in clinical/diagnostic use, but being bivalent they should retain higher binding affinity than monovalent antibody fragments, such as dAbs. Accordingly, unless the context dictates otherwise, the term "antibody" as used herein encompasses not only whole antibody molecules but also antigen-binding antibody fragments of the type discussed above. Preferably each framework region present in the encoded polypeptide will comprise at least one amino acid substitution relative to the corresponding human acceptor framework. Thus, for example, the framework regions may comprise, in total, three, four, five, six, seven, eight, nine, ten, eleven, twelve, thirteen, fourteen, or fifteen amino acid substitutions relative to the acceptor framework regions.

Suitably, the polynucleotides described herein may be isolated and/or purified. In some embodiments, the polynucleotides are isolated polynucleotides.

The term "isolated polynucleotide" is intended to indicate that the molecule is removed or separated from its normal or natural environment or has been produced in such a way that it is not present in its normal or natural environment. In some embodiments, the polynucleotides are purified polynucleotides. The term purified is intended to indicate that at least some contaminating molecules or substances have been removed.

Suitably, the polynucleotides are substantially purified, such that the relevant polynucleotides constitute the dominant *(i.e.,* most abundant) polynucleotides present in a composition.

### Expression of Polynucleotides

The description below relates primarily to production of polypeptides by culturing cells transformed or transfected with a vector containing polypeptide-encoding polynucleotides. It is, of course, contemplated that alternative methods, which are well known in the art, may be employed to prepare polypeptides. For instance, the appropriate amino acid sequence, or portions thereof, may be produced by direct peptide synthesis using solid-phase techniques (*see, e.g.,* Stewart et al., Solid-Phase Peptide Synthesis W.H. Freeman Co., San Francisco, Calif. (1969); Merrifield, J. Am. Chem. Soc. 85:2149-2154 (1963)). In vitro protein synthesis may be performed using manual techniques or by automation.

Automated synthesis may be accomplished, for instance, using an Applied Biosystems Peptide Synthesizer (Foster City, Calif.) using manufacturer's instructions. Various portions of the polypeptide may be chemically synthesized separately and combined using chemical or enzymatic methods to produce the desired polypeptide.

Polynucleotides as described herein are inserted into an expression vector(s) for production of the polypeptides. The term "control sequences" refers to DNA sequences necessary for the expression of an operably linked coding sequence in a particular host organism. The control sequences include, but are not limited to, promoters (e.g., naturally-associated or heterologous promoters), signal sequences, enhancer elements, and transcription termination sequences.

A polynucleotide is "operably linked" when it is placed into a functional relationship with another polynucleotide sequence. For example, nucleic acids for a presequence or secretory leader is operably linked to nucleic acids for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the nucleic acid sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

For antibodies, the light and heavy chains can be cloned in the same or different expression vectors. The nucleic acid segments encoding immunoglobulin chains are operably linked to control sequences in the expression vector(s) that ensure the expression of immunoglobulin polypeptides.

For CrossMabs which comprise four different polypeptide chains four expression cassettes are used. These can be cloned in two to four different expression vectors. Each of the nucleic acid segments encoding immunoglobulin chains are operably linked to control sequences in the expression vector(s) that ensure the expression of immunoglobulin polypeptides. If two or more expression cassettes are comprised on the same expression vector these can be organized unidirectional or bidirectional.

The vectors containing the polynucleotide sequences (e.g., the variable heavy and/or variable light chain encoding sequences and optional expression control sequences) can be transferred into a host cell by well-known methods, which vary depending on the type of cellular host. For example, calcium chloride transfection is commonly utilized for prokaryotic cells, whereas calcium phosphate treatment, electroporation, lipofection, biolistics or viral-based transfection may be used for other cellular hosts. (*See generally* Sambrook et al., Molecular Cloning: A Laboratory Manual (Cold Spring Harbor Press, 2nd ed., 1989). Other methods used to transform mammalian cells include the use of polybrene, protoplast fusion, liposomes, electroporation, and microinjection. For production of transgenic animals, transgenes can be microinjected into fertilized oocytes, or can be incorporated into the genome of embryonic stem cells, and the nuclei of such cells transferred into enucleated oocytes.

*Vectors*

The term "vector" includes expression vectors and transformation vectors and shuttle vectors.

The term "expression vector" means a construct capable of *in vivo* or *in vitro* expression.

The term "transformation vector" means a construct capable of being transferred from one entity to another entity - which may be of the species or may be of a different species. If the construct is capable of being transferred from one species to another - such as from an *Escherichia coli* plasmid to a bacterium, such as of the genus *Bacillus,* then the transformation vector is sometimes called a "shuttle vector". It may even be a construct capable of being transferred from an *E. coli* plasmid to an *Agrobacterium* to a plant.

Vectors may be transformed into a suitable host cell as described below to provide for expression of a polypeptide. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Construction of suitable vectors containing one or more of these components employs standard ligation techniques which are known to the skilled artisan.

The vectors may be for example, plasmid, virus or phage vectors provided with an origin of replication, optionally a promoter for the expression of the said polynucleotide and optionally a regulator of the promoter. Vectors may contain one or more selectable marker genes which are well known in the art.

These expression vectors are typically replicable in the host organisms either as episomes or as an integral part of the host chromosomal DNA.

For multispecific antibody production, nucleic acid(s) encoding the multispecific antibody (or an arm of the multispecific antibody, *i.e.,* a heavy chain/light chain pair) are typically isolated and inserted into replicable vectors for further cloning, amplification, and/or for expression. DNA encoding the antibody is readily isolated and sequenced using conventional procedures (e.g., by using oligonucleotide probes that are capable of binding specifically to genes encoding the heavy and light chains of the antibody). Many vectors are available. The choice of vector depends in part on the host cell to be used. It will be appreciated that constant regions of any isotype can be used for this purpose, including IgG, IgM, IgA, IgD, and IgE constant regions, and that such constant regions can be obtained from any human or animal species.

**Host Cells**

The host cell may be a bacterium, a yeast or other fungal cell, insect cell, a plant cell, or a mammalian cell, for example.

A transgenic multicellular host organism which has been genetically manipulated may be used to produce a polypeptide. The organism may be, for example, a transgenic mammalian organism *(e.g.,* a transgenic goat or mouse line).

Suitable prokaryotes include but are not limited to eubacteria, such as Gram-negative or Gram-positive organisms, for example, *Enterobacteriaceae* such as *E. coli.* Various *E. coli* strains are publicly available, such as *E. coli* K12 strain MM294 (ATCC 31,446); *E. coli* X1776 (ATCC 31,537); E. *coli* strain W3110 (ATCC 27,325) and K5 772 (ATCC 53,635). Other suitable prokaryotic host cells include *Enterobacteriaceae* such as *Escherichia, e.g., E. coli, Enterobacter, Erwinia, Klebsiella, Proteus, Salmonella, e.g., Salmonella typhimurium, Serratia, e.g., Serratia marcescans,* and *Shigella,* as well as *Bacilli* such as *B. subtilis and B. licheniformis (e.g., B. licheniformis* 41P), *Pseudomonas* such as P. *aeruginosa,* and *Streptomyces.* These examples are illustrative rather than limiting. Strain W3110 is one particularly preferred host or parent host because it is a common host strain for recombinant polynucleotide product fermentations. Preferably, the host cell secretes minimal amounts of proteolytic enzymes. For example, strain W3110 may be modified to effect a genetic mutation in the genes encoding polypeptides endogenous to the host, with examples of such hosts including *E. coli* W3110 strain 1A2, which has the complete genotype *tonA; E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan'; E. coli* W3110 strain 37D6, which has the complete genotype *tonA ptr3 phoA E15 (argF-lac)169 degP rbs7 ilvG kan'; E. coli* W3110 strain 40B4, which is strain 37D6 with a non-kanamycin resistant *degP* deletion mutation; and an *E. coli* strain having mutant periplasmic protease. Alternatively, in vitro methods of cloning, *e.g.,* PCR or other nucleic acid polymerase reactions, are suitable. In some embodiments, the prokaryotic host cell *(e.g.,* an *E. coli* host cell) expresses one or more chaperones to facilitate folding and assembly of the antibody. In some embodiments, the chaperone is one or more of FkpA, DsbA or DsbC. In some embodiments, the chaperone is expressed from an endogenous chaperone gene. In some embodiments, the chaperone is expressed from an exogenous chaperone gene. In some embodiments, the chaperone gene is an *E. coli* chaperone gene *(e.g.,* an *E. coli* FkpA gene, an *E. coli* DsbA gene and/or an *E. coli* DsbC gene).

In these prokaryotic hosts, one can make expression vectors, which will typically contain expression control sequences compatible with the host cell *(e.g.,* an origin of replication). In addition, any number of a variety of well-known promoters will be present, such as the lactose promoter system, a tryptophan (trp) promoter system, a beta-lactamase promoter system, or a promoter system from phage lambda. The promoters will typically control expression, optionally with an operator sequence, and have ribosome binding site sequences and the like, for initiating and completing transcription and translation.

Eukaryotic microbes may be used for expression. Eukaryotic microbes such as filamentous fungi or yeast are suitable cloning or expression hosts for polypeptide-encoding vectors. *Saccharomyces cerevisiae* is a commonly used lower eukaryotic host microorganism. Others include *Schizosaccharomyces pombe; Kluyveromyces hosts* such as, *e.g., K. lactis* (MW98-8C, CBS683, CBS4574), *K. fragilis* (ATCC 12,424), *K. bulgaricus* (ATCC 16,045), *K. wickeramii* (ATCC 24,178), K. *waltii* (ATCC 56,500), *K. drosophilarum* (ATCC 36,906), *K. thermotolerans,* and *K. marxianus; yarrowia* (EP 402,226); *Pichia pastoris; Candida; Trichoderma reesia*; *Neurospora crassa; Schwanniomyces* such as *Schwanniomyces occidentalis;* and filamentous fungi such as, *e.g., Neurospora, Penicillium, Tolypocladium,* and Aspergillus hosts such as A. *nidulans,* and A. *niger.* Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula. Saccharomyces* is a preferred yeast host, with suitable vectors having expression control sequences (e.g., promoters), an origin of replication, termination sequences and the like as desired. Typical promoters include 3-phosphoglycerate kinase and other glycolytic enzymes. Inducible yeast promoters include, among others, promoters from alcohol dehydrogenase, isocytochrome C, and enzymes responsible for maltose and galactose utilization.

In addition to microorganisms, mammalian tissue cell culture may also be used to express and produce the polypeptides as described herein and in some instances are preferred (*See* Winnacker, From Genes to Clones VCH Publishers, N.Y., N.Y. (1987). For some embodiments, eukaryotic cells may be preferred, because a number of suitable host cell lines capable of secreting heterologous polypeptides (e.g., intact immunoglobulins) have been developed in the art, and include CHO cell lines, various Cos cell lines, HeLa cells, preferably, myeloma cell lines, or transformed B-cells or hybridomas. In some embodiments, the mammalian host cell is a CHO cell.

In some embodiments, the host cell is a vertebrate host cell. Examples of useful mammalian host cell lines are monkey kidney CV1 line transformed by SV40 (COS-7, ATCC CRL 1651); human embryonic kidney line (293 or 293 cells subcloned for growth in suspension culture); baby hamster kidney cells (BHK, ATCC CCL 10); Chinese hamster ovary cells/-DHFR(CHO or CHO-DP-12 line); mouse sertoli cells; monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells; MRC 5 cells; FS4 cells; and a human hepatoma line (Hep G2).

*Generating Multispecific Antibodies Using Prokaryotic Host Cells*

*Vector Construction*

Polynucleotide sequences encoding polypeptide components of the multispecific antibody to be purified according to a method provided herein can be obtained using standard recombinant techniques. Desired polynucleotide sequences may be isolated and sequenced from antibody producing cells, such as hybridoma cells. Alternatively, polynucleotides can be synthesized using nucleotide synthesizer or PCR techniques. Once obtained, sequences encoding the polypeptides (such as two or more heavy chains and/or two or more light chains) are inserted into a recombinant vector capable of replicating and expressing heterologous polynucleotides in a host cell (such as an *E. coli* cell). Many vectors that are available and known in the art can be used for the purpose of the methods and compositions provided herein. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, *E. coli* is typically transformed using pBR322, a plasmid derived from an *E. coli* species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Patent No. 5,648,237.

In addition, phage vectors containing replicon and control sequences that are compatible with the host microorganism can be used as transforming vectors in connection with these hosts. For example, bacteriophage such as GEM™-11 may be utilized in making a recombinant vector which can be used to transform susceptible host cells such as *E. coli* LE392.

An expression vector may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, *e.g.*, the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into a vector. Both the native promoter sequence and many heterologous promoters may be used to direct amplification and/or expression of the target genes. In some embodiments, heterologous promoters are utilized, as they generally permit greater transcription and higher yields of expressed target gene as compared to the native target polypeptide promoter.

Promoters suitable for use with prokaryotic hosts include the PhoA promoter, the -lactamase and lactose promoter systems, a tryptophan (trp) promoter system and hybrid promoters such as the tac or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al., (1980) Cell 20: 269) using linkers or adaptors to supply any required restriction sites.

The translational initiation region (TIR) is a major determinant of the overall translation level of a protein. The TIR includes the polynucleotide that encodes the signal sequence, and extends from immediately upstream of the Shine-Dalgarno sequence to approximately twenty nucleotides downstream of the initiation codon. Generally, the vector will comprise a TIR, TIRs and variant TIRs are known in the art and methods for generating TIRs are known in in the art A series of nucleic acid sequence variants can be created with a range of translational strengths, thereby providing a convenient means by which to adjust this factor for the optimal secretion of many different polypeptides. The use of a reporter gene fused to these variants, such as PhoA, provides a method to quantitate the relative translational strengths of different translation initiation regions. The variant or mutant TIRs can be provided in the background of a plasmid vector thereby providing a set of plasmids into which a gene of interest may be inserted and its expression measured, so as to establish an optimum range of translational strengths for maximal expression of mature polypeptide. Variant TIRs are disclosed in USP 8,241,901.

In one aspect, each cistron within the recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected should be one that is recognized and processed (i.e., cleaved by a signal peptidase) by the host cell. For prokaryotic host cells that do not recognize and process the signal sequences native to the heterologous polypeptides, the signal sequence is substituted by a prokaryotic signal sequence. Such sequences are well known in the art. In addition, the vector may comprise a signal sequence selected from the group consisting of alkaline phosphatase, penicillinase, Lpp, or heat-stable enterotoxin II (STII) leaders, LamB, PhoE, PelB, OmpA, and MBP.

In one aspect, one or more polynucleotides (e.g., expression vectors) collectively encode an antibody. In one embodiment, a single polynucleotide encodes the light chain of the antibody and a separate polynucleotide encodes the heavy chain of the antibody. In one embodiment, a single polynucleotide encodes the light chain and heavy chain of the antibody. In some embodiments, one or more polynucleotides (e.g., expression vectors) collectively encode a one-armed antibody. In one embodiment, a single polynucleotide encodes (a) the light and heavy chain of the one armed antibody, and (b) the Fc polypeptide. In one embodiment, a single polynucleotide encodes the light and heavy chain of the one armed antibody, and a separate polynucleotide encodes the Fc polypeptide. In one embodiment, separate polynucleotides encode the light chain component of the one-armed antibody, the heavy chain component of the one-armed antibody and the Fc polypeptide, respectively. Production of a one-armed antibody is described in, for example, in WO2005063816.

Prokaryotic host cells suitable for expressing antibodies include Archaebacteria and Eubacteria, such as Gram-negative or Gram-positive organisms. Examples of useful bacteria include Escherichia (e.g., E. coli), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. In one embodiment, gram-negative cells are used. In one embodiment, E. coli cells are used as host cells. Examples of E. coli strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (ΔtonA) ptr3 lac Iq lacL8 ΔompTΔ (nmpc-fepE) degP41 kanR (U.S. Pat. No. 5,639,635) and strains 63C1 and 64B4. In some embodiments, the E. coli strain is a W3110 derivative named 62A7 (ΔfhuA (ΔtonA) ptr3, lacIq, lacL8, ompTΔ(nmpc-fepE) ΔdegP ilvG repaired). Other strains and derivatives thereof, such as E. coli 294 (ATCC 31,446), E. coli B, E. coli λ 1776 (ATCC 31,537) and E. coli RV308(ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, E. coli, Serratia, or Salmonella species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture.

To improve the production yield and quality of the polypeptides in bacterial cultures, the bacterial cells can be modified. For example, to improve the proper assembly and folding of the secreted antibody polypeptides, the bacteria host cell may comprise additional vectors expressing chaperone proteins, such as FkpA and Dsb proteins (DsbB, DsbC, DsbD, and/or DsbG) can be used to co-transform the host prokaryotic cells. The chaperone proteins have been demonstrated to facilitate the proper folding and solubility of heterologous proteins produced in bacterial host cells.

### Multispecific Antibody Production

Host cells are transformed with the above-described expression vectors and cultured in conventional nutrient media modified as appropriate for inducing promoters, selecting transformants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

Prokaryotic cells used to produce the polypeptides purified according to the methods provided herein are grown in media known in the art and suitable for culture of the selected host cells. Examples of suitable media include Luria broth (LB) plus necessary nutrient supplements. In some embodiments, the media also contains a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. For *E. coli* growth, for example, the preferred temperature ranges from about 20 °C to about 39 °C, more preferably from about 25 °C to about 37 °C, even more preferably at about 30 °C. The pH of the medium may be any pH ranging from about 5 to about 9, depending mainly on the host organism. For *E. coli,* the pH is preferably from about 6.8 to about 7.4, and more preferably about 7.0.

If an inducible promoter is used in the expression vector, protein expression is induced under conditions suitable for the activation of the promoter. In one aspect, PhoA promoters are used for controlling transcription of the polypeptides. Accordingly, the transformed host cells are cultured in a phosphate-limiting medium for induction. Preferably, the phosphate-limiting medium is the C.R.A.P medium (see, *e.g.,* Simmons et al., J. Immunol. Methods (2002), 263:133-147) or media described in WO2002/061090. A variety of other inducers may be used, according to the vector construct employed, as is known in the art.

In one embodiment, the expressed polypeptides to be purified using methods provided herein are secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

In one aspect, antibody production is conducted in large quantity by a fermentation process. Various large-scale fed-batch fermentation procedures are available for production of recombinant polypeptides. Large-scale fermentations have at least 1000 liters of capacity, preferably about 1,000 to 100,000 liters of capacity. These fermentors use agitator impellers to distribute oxygen and nutrients, especially glucose (the preferred carbon/energy source). Small scale fermentation refers generally to fermentation in a fermenter that is no more than approximately 100 liters in volumetric capacity, and can range from about 1 liter to about 100 liters.

In a fermentation process, induction of protein expression is typically initiated after the cells have been grown under suitable conditions to a desired density, *e.g.*, an OD550 of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To improve the production yield and quality of the polypeptides, various fermentation conditions can be modified. For example, to improve the proper assembly and folding of the secreted antibody polypeptides, additional vectors expressing chaperone proteins, such as FkpA, DsbA and/or DsbC can be used to co-transform the host prokaryotic cells. The chaperone proteins have been demonstrated to facilitate the proper folding and solubility of heterologous proteins produced in bacterial host cells. In some embodiments, FkpA, DsbA and/or DsbC are expressed in the bacterial host cell.

To minimize proteolysis of expressed heterologous proteins (especially those that are proteolytically sensitive), certain host strains deficient for proteolytic enzymes can be used. For example, host cell strains may be modified to effect genetic mutation(s) in the genes encoding known bacterial proteases such as Protease III, OmpT, DegP, Tsp, Protease I, Protease Mi, Protease V, Protease VI, and combinations thereof. Some *E. coli* protease-deficient strains are available and described in, for example, Joly *et al.*, (1998), *supra;* Georgiou et al., U.S. Patent No. 5,264,365; Georgiou et al., U.S. Patent No. 5,508,192; Hara et al., Microbial Drug Resistance, 2:63-72 (1996).

In one embodiment, *E. coli* strains deficient for proteolytic enzymes and transformed with plasmids expressing one or more chaperone proteins are used as host cells in the expression system

### Generating Multispecific Antibodies Using Eukaryotic Host Cells

### Signal Sequence Component

A vector for use in a eukaryotic host cell may optionally contain a signal sequence or other polypeptide having a specific cleavage site at the N-terminus of the mature protein or polypeptide of interest. The heterologous signal sequence selected preferably is one that is recognized and processed *(i.e.,* cleaved by a signal peptidase) by the host cell. In mammalian cell expression, mammalian signal sequences as well as viral secretory leaders, for example, the herpes simplex gD signal, are available. The DNA for such precursor region is ligated in reading frame to DNA encoding the desired heteromultimeric protein(s) (e.g., antibodies).

*Origin of replication*

Generally, an origin of replication component is not needed for mammalian expression vectors. For example, the SV40 origin may typically be used, but only because it contains the early promoter.

*Selection gene component*

Expression and cloning vectors may contain a selection gene, also termed a selectable marker. Typical selection genes encode proteins that (a) confer resistance to antibiotics or other toxins, *e.g.*, ampicillin, neomycin, methotrexate, or tetracycline, (b) complement auxotrophic deficiencies, where relevant, or (c) supply critical nutrients not available from complex media.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II, preferably primate metallothionein genes, adenosine deaminase, ornithine decarboxylase, *etc.*

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity (e.g., ATCC CRL-9096).

Alternatively, host cells (particularly wild-type hosts that contain endogenous DHFR) transformed or co-transformed with DNA sequences encoding an antibody, wild-type DHFR protein, and another selectable marker such as aminoglycoside 3'phosphotransferase (APH) can be selected by cell growth in medium containing a selection agent for the selectable marker such as an aminoglycosidic antibiotic, *e.g.*, kanamycin, neomycin, or G418. See, for example, U.S. Patent No. 4,965,199.

*Promoter component*

Expression and cloning vectors usually contain a promoter that is recognized by the host organism and is operably linked to the desired hinge-containing polypeptide(s) (e.g., antibody) nucleic acid. Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Desired heavy chain and/or light chain transcription from vectors in mammalian host cells is controlled, for example, by promoters obtained from the genomes of viruses such as, for example, polyoma virus, fowl pox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g.*, the actin promoter or an immunoglobulin promoter, or from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a Hind III E restriction fragment. A system for expressing DNA in mammalian hosts using the bovine papilloma virus as a vector is disclosed in U.S. Patent No. 4,419,446. A modification of this system is described in U.S. Patent No. 4,601,978. See also Reyes et al., Nature 297:598-601 (1982) on expression of human b-interferon eDNA in mouse cells under the control of a thymidine kinase promoter from herpes simplex virus. Alternatively, the Rous Sarcoma Virus long terminal repeat can be used as the promoter.

*Enhancer Element Component*

Transcription of DNA encoding the desired hinge-containing polypeptide(s) (e.g., antibody) by higher eukaryotes can be increased by inserting an enhancer sequence into the vector. Many enhancer sequences are now known from mammalian genes (e.g., globin, elastase, albumin, a-fetoprotein, and insulin genes). Also, one may use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) for a description of elements for enhancing activation of eukaryotic promoters. The enhancer may be spliced into the vector at a position 5' or 3' to the antibody polypeptide-encoding sequence, provided that enhancement is achieved, but is generally located at a site 5' from the promoter.

### *Transcription Termination Component*

Expression vectors used in eukaryotic host cells will typically also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding an antibody. One useful transcription termination component is the bovine growth hormone polyadenylation region. See WO 94/11026 and the expression vector disclosed therein.

### *Selection and transformation of host cells*

Suitable host cells for cloning or expressing the DNA in the vectors herein include higher eukaryote cells described herein, including vertebrate host cells. Propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Viral. 36:59

(1977)); baby hamster kidney cells (BHK, ATCC CCL 10); Chinese hamster ovary cells/-DHFR (CHO, Urlaub et al., Proc. Nat/. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MOCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for desired hinge-containing polypeptide(s) (e.g., antibody) production and cultured in conventional nutrient media modified as appropriate for inducing promoters, selecting transformants, or amplifying the genes encoding the desired sequences.

### *Culturing the host cells*

The host cells used to produce a multispecific antibody (e.g., a bispecific antibody) may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al, Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### *Multispecific antibody Formation/Assembly*

In certain embodiments, provided herein are methods for producing multispecific antibodies. In certain embodiments, multispecific antibodies are produced by separately producing half-antibodies, each half antibody comprising a VH/VL unit that binds a specific epitope (e.g., different epitopes on a single target, or different epitopes on two or more targets). In some embodiments, each half-antibody is produced separately in a host cell. In some embodiments, each of the half-antibodies is produced in the same host cell. The half-antibodies are mixed and allowed to assemble into the multispecific antibody. In some embodiments, each of the half-antibodies is produced together in the same host cell. In some embodiments the host cell (such as a prokaryotic host cell, *e.g.,* an *E. coli* cell) expresses a chaperone, such as FkpA, DsbA and/or DsbC, to facilitate folding of the half-antibody.

In some embodiments, half-antibodies containing either the knob or hole mutations are generated in separate cultures by expressing the heavy and light chains constructs in a bacterial host cell, *e.g., E. coli.* Each half-antibody can be purified separately by Protein A affinity chromatography. Clarified cell extracts from the knob and hole half-antibody can be purified by a Protein A column. Protein A purified half antibodies with different specificity can be assembled to form a bispecific antibody in a redox reaction *in vitro* in the presence of a reducing agent.

In some embodiments, half-antibodies containing either the knob or hole mutations are generated in the same culture by expressing the heavy and light chains constructs in the same bacterial host cell, *e.g.,* an *E. coli* host cell or a CHO host cell. The half-antibodies can be purified by Protein A affinity chromatography. Clarified cell extracts from the knob and hole half-antibodies can be purified by a Protein A column. Protein A purified half antibodies with different specificity can be assembled to form a bispecific antibody in a redox reaction *in vitro* in the presence of a reducing agent.

Any suitable methods can be used to prepare a desired reducing condition. For example, a desired reducing condition can be prepared by adding a reductant/reducing agent to the reaction (such as an assembly mixture described herein). Suitable reductants include without limitation dithiothreitol (DTT), tris(2-carboxyethyl)phosphine (TCEP), thioglycolic acid, ascorbic acid, thiol acetic acid, glutathione (GSH), Beta-MercaptoEthylAmine, cysteine/cystine, GSH/glutathione disulfide (GSSG), cysteamine/cystamine, glycylcysteine, and beta-mercaptoethanol, preferably GSH. In certain particular embodiments, the reductant is a weak reductant including without limitation GSH, Beta-MercaptoEthylAmine, cysteine/cystine, GSH/GSSG, cysteamine/cystamine, glycylcysteine, and beta-mercaptoethanol, preferably GSH. In certain preferred embodiments, the reductant is GSH. In certain embodiments, the reductant is not DTT. It is within the ability of one of ordinary skill in the art to select suitable reductants at suitable concentrations and under suitable experimental conditions to achieve in a reaction the desired reducing condition. For example, 10 mM L-reduced glutathione in a solution with a bispecific antibody protein concentration of 10g/L at 20 °C will result in a starting redox potential of about - 400mV. For example, glutathione added to an assembly mixture creates a weakly reducing condition that is advantageous for knob-into-hole bispecific assembly. Other reductants in a similar class such as BMEA (Beta-MercaptoEthylAmine) may have a similar effect. See WO2013/055958, incorporated herein by reference in its entirety. The reducing condition of the reaction can be estimated and measured using any suitable methods known in the art. For example, the reducing condition can be measured using a resazurin indicator (discolorization from blue to colorless in reducing conditions). For more precise measurement, a redox-potential meter (such as an ORP Electrode made by BROADLEY JAMES®) can be used.

In certain particular embodiments, the reducing condition is a weak reducing condition. The term "weak reductant" or "weak reducing condition" as used herein refers to a reducing agent or a reducing condition prepared by the reducing agent having a negative oxidation potential at 25°C. The oxidation potential of the reductant is preferably between -50 to -600 mV, -100 to -600 mV, -200 to -600 mV, -100 to -500 mV, -150 to -300 mV, more preferably between about -300 to -500 mV, most preferably about -400mV, when the pH is between 7 and 9, and the temperature is between 15°C and 39°C. One skilled in the art will be able to select suitable reductants for preparing a desired reducing condition. The skilled researcher will recognize that a strong reductant, *i.e.,* one that has a more negative oxidation potential than above mentioned reductants for the same concentration, pH and temperature, may be used at a lower concentration. In a preferred embodiment, the proteins will be able to form disulfide bonds in the presence of the reductant when incubated under the above-recited conditions. Examples of a weak reductant include without limitation glutathione, Beta-MercaptoEthylAmine, cystine/cysteine, GSH/GSSG, cysteamine/cystamine, glycylcysteine, and beta-mercaptoethanol. In certain embodiments, an oxidation potential similar to that of 200X molar ratio of GSH:Antibody can be used as a point of reference for a weakly reducing condition at which efficient assembly using other reductants can be expected.

Glutathione concentrations can be expressed in terms of molarity or in terms of molar ratio or molar excess with respect to the amount of the half-antibodies present in the assembly mixture. Using a target molar ratio of reductant controls for the protein concentration in the assembly mixture; this prevents over reducing or under reducing as a result of variable protein concentrations. In certain other embodiments, the reductant is added to the assembly mixture in 2-600X, 2-200X, 2-300X, 2-400X, 2-500X, 2-20X, 2-8X, 20-50X, 50-600X, 50-200X, or 100-300X molar excess, preferably 50-400X or 50-150X, more preferably 100-300X, and most preferably 200X or 100X, molar excess with respect to the total amount of the half antibodies. In certain embodiments, the assembly mixture has a pH of between 7 and 9, preferably pH 8.5 or 8.3.

Also provided are immunoconjugates (interchangeably termed "antibody-drug conjugates" or "ADC"), comprising any of the antibodies described herein conjugated to, *e.g.,* a cytotoxic agent such as a chemotherapeutic agent, a drug, a growth inhibitory agent, a toxin *(e.g.,* an enzymatically active toxin of bacterial, fungal, plant, or animal origin, or fragments thereof), or a radioactive isotope *(i.e.,* a radioconjugate).

*Antigens/Target Molecules*

Examples of molecules that may be targeted by a multispecific antibody purified according to a method described herein include, but are not limited to, soluble serum proteins and their receptors and other membrane bound proteins (e.g., adhesins). In certain embodiments, a multispecific antibody purified according to a method described herein is capable of binding one, two, or more than two cytokines, cytokine-related proteins, and cytokine receptors selected from the group consisting of 8MPI, 8MP2, 8MP38 (GDFIO), 8MP4, 8MP6, 8MP8, CSFI (M-CSF), CSF2 (GM-CSF), CSF3 (G-CSF), EPO, FGF1 ($\alpha$FGF), FGF2 ($\beta$FGF), FGF3 (int-2), FGF4 (HST), FGF5, FGF6 (HST-2), FGF7 (KGF), FGF9, FGF1 0, FGF11, FGF12, FGF12B, FGF14, FGF16, FGF17, FGF19, FGF20, FGF21, FGF23, IGF1, IGF2, IFNA1, IFNA2, IFNA4, IFNA5, IFNA6, IFNA7, IFN81, IFNG, IFNWI, FEL1, FEL1 (EPSELON), FEL1 (ZETA), IL 1A, IL 1B, IL2, IL3, IL4, IL5, IL6, IL7, IL8, IL9, IL1 0, IL 11, IL 12A, IL 12B, IL 13, IL 14, IL 15, IL 16, IL 17, IL 17B, IL 18, IL 19, IL20, IL22, IL23, IL24, IL25, IL26, IL27, IL28A, IL28B, IL29, IL30, IL33, PDGFA, PDGFB, TGFA, TGFB1, TGFB2, TGFBb3, LTA (TNF-$\beta$), LTB, TNF (TNF-$\alpha$), TNFSF4 (OX40 ligand), TNFSF5 (CD40 ligand), TNFSF6 (FasL), TNFSF7 (CD27 ligand), TNFSF8 (CD30 ligand), TNFSF9 (4-1 BB ligand), TNFSF10 (TRAIL), TNFSF11 (TRANCE), TNFSF12 (APO3L), TNFSF13 (April), TNFSF13B, TNFSF14 (HVEM-L), TNFSF15 (VEGI), TNFSF18, HGF (VEGFD), VEGF, VEGFB, VEGFC, IL1R1, IL1R2, IL1RL1, IL1RL2, IL2RA, IL2RB, IL2RG, IL3RA, IL4R, IL5RA, IL6R, IL7R, IL8RA, IL8RB, IL9R, IL10RA, IL10RB, IL 11RA, IL12RB1, IL12RB2, IL13RA1, IL13RA2, IL15RA, IL17R, IL18R1, IL20RA, IL21R, IL22R, IL1HY1, IL1RAP, IL1RAPL1, IL1RAPL2, IL1RN, IL6ST, IL18BP, IL18RAP, IL22RA2, AIF1, HGF, LEP (leptin), PTN, and THPO.

In certain embodiments, a multispecific antibody purified according to a method described herein is capable of binding is a chemokine, chemokine receptor, or a chemokine-related protein selected from the group consisting of CCLI (1-309), CCL2 (MCP -1/MCAF), CCL3 (MIP-I$\alpha$), CCL4 (MIP-I$\beta$), CCL5 (RANTES), CCL7 (MCP-3), CCL8 (mcp-2), CCL11 (eotaxin), CCL 13 (MCP-4), CCL 15 (MIP-I$\delta$), CCL 16 (HCC-4), CCL 17 (TARC), CCL 18 (PARC), CCL 19 (MDP-3b), CCL20 (MIP-3$\alpha$), CCL21 (SLC/exodus-2), CCL22 (MDC/STC-1), CCL23 (MPIF-1), CCL24 (MPIF-2/eotaxin-2), CCL25 (TECK), CCL26 (eotaxin-3), CCL27 (CTACK / ILC), CCL28, CXCLI (GROI), CXCL2 (GR02), CXCL3 (GR03), CXCL5 (ENA-78), CXCL6 (GCP-2), CXCL9 (MIG), CXCL 10 (IP 10), CXCL 11 (1-TAC), CXCL 12 (SDFI), CXCL 13, CXCL 14, CXCL 16, PF4 (CXCL4), PPBP (CXCL7), CX3CL 1 (SCYDI), SCYEI, XCLI (lymphotactin), XCL2 (SCM-I$\beta$), BLRI (MDR15), CCBP2 (D6/JAB61 ), CCRI (CKRI/HM145), CCR2 (mcp-IRB IRA), CCR3 (CKR3/CMKBR3), CCR4, CCR5 (CMKBR5/ChemR13), CCR6 (CMKBR6/CKR-L3/STRL22/DRY6), CCR7 (CKR7/EBII), CCR8 (CMKBR8/TER1/CKR-L1), CCR9 (GPR-9-6), CCRL1 (VSHK1), CCRL2 (L-CCR), XCR1 (GPR5/CCXCR1), CMKLR1, CMKOR1 (RDC1), CX3CR1 (V28), CXCR4, GPR2 (CCR10), GPR31, GPR81 (FKSG80), CXCR3 (GPR9/CKR-L2), CXCR6 (TYMSTR/STRL33/Bonzo), HM74, IL8RA (IL8R$\alpha$), IL8RB (IL8R$\beta$), LTB4R (GPR16), TCP10, CKLFSF2, CKLFSF3, CKLFSF4, CKLFSF5, CKLFSF6, CKLFSF7, CKLFSF8, BDNF, C5R1, CSF3, GRCC10 (C10), EPO, FY (DARC), GDF5, HDF1, HDF1$\alpha$, DL8, PRL, RGS3, RGS13, SDF2, SLIT2, TLR2, TLR4, TREM1, TREM2, and VHL.

In certain embodiments, a multispecific antibody purified according to a method described herein is capable of binding one or more targets selected from the group consisting of ABCF1; ACVR1; ACVR1B; ACVR2; ACVR2B; ACVRL1; ADORA2A; Aggrecan; AGR2; AICDA; AIF1; AIG1; AKAP1; AKAP2; AMH; AMHR2; ANGPTL; ANGPT2; ANGPTL3; ANGPTL4; ANPEP; APC; APOC1; AR; AZGP1 (zinc-a-glycoprotein); B7.1; B7.2; BAD; BAFF (BLys); BAG1; BAI1; BCL2; BCL6; BDNF; BLNK; BLRI (MDR15); BMP1; BMP2; BMP3B (GDF10); BMP4; BMP6; BMP8; BMPR1A; BMPR1B; BMPR2; BPAG1 (plectin); BRCA1; C19orf10 (IL27w); C3; C4A; C5; C5R1; CA125; CA15-3; CA19-9; CANT1; CASP1; CASP4; CAV1; CCBP2 (D6/JAB61); CCL1 (1-309); CCL11 (eotaxin); CCL13 (MCP-4); CCL15 (MIP1$\delta$); CCL16 (HCC-4); CCL17 (TARC); CCL18 (PARC); CCL19 (MIP-3$\beta$); CCL2 (MCP-1); MCAF; CCL20 (MIP-3$\alpha$); CCL21 (MTP-2); SLC; exodus-2; CCL22 (MDC/STC-1); CCL23 (MPIF-1); CCL24 (MPIF-2/eotaxin-2); CCL25 (TECK); CCL26 (eotaxin-3); CCL27 (CTACK/ILC); CCL28; CCL3 (MTP-I$\alpha$); CCL4 (MDP-I$\beta$); CCL5(RANTES); CCL7 (MCP-3); CCL8 (mcp-2); CCNA1; CCNA2; CCND1; CCNE1; CCNE2; CCR1 (CKRI/HM145); CCR2 (mcp-IR$\beta$/RA);CCR3 (CKR/ CMKBR3); CCR4; CCR5 (CMKBR5/ChemR13); CCR6 (CMKBR6/CKR-L3/STRL22/DRY6); CCR7 (CKBR7/EBI1); CCR8 (CMKBR8/TER1/CKR-L1); CCR9 (GPR-9-6); CCRL1 (VSHK1); CCRL2 (L-CCR); CD11a; CD13; CD164; CD19; CD1C; CD20; CD200; CD22; CD23; CD24; CD28; CD3; CD30; CD31; CD33; CD34; CD35; CD37; CD38; CD39; CD3E; CD3G; CD3Z; CD4; CD40; CD40L; CD41; CD44; LCA/CD45; CD45RA; CD45RB; CD45RO; CD5; CD52; CD69; CD7; CD71; CD72; CD74; CD79A; CD79B; CD8; CD80; CD81; CD83; CD86; CD95/Fas; CD99; CD100; CD106; CDH1 (E-cadherin); CD9/p24; CDH10; CD11a; CD11c; CD13; CD14; CD19, CD20; CDH12; CDH13; CDH18; CDH19; CDH20; CDH5; CDH7; CDH8; CDH9; CDK2; CDK3; CDK4; CDK5; CDK6; CDK7; CDK9; CDKN1A (p21/WAF1/-Cip1); CDKN1B (p27/Kip1); CDKN1C; CDKN2A (P16INK4a); CDKN2B; CDKN2C; CDKN3; CEA; CEBPB; CER1; CHGA; CHGB; Chitinase; CHST10; CKLFSF2; CKLFSF3; CKLFSF4; CKLFSF5; CKLFSF6; CKLFSF7; CKLFSF8; CLDN3;CLDN7 (claudin-7); CLN3; CLU (clusterin); C-MET; CMKLR1; CMKOR1 (RDC1); CNR1; COL 18A1; COL1A1; COL4A3; COL6A1; CR2; CRP; CSFI (M-CSF); CSF2 (GM-CSF); CSF3 (GCSF); CTLA4; CTNNB1 (b-catenin); CTSB (cathepsin B); CTSD (cathepsin D); CX3CL1 (SCYDI); CX3CR1 (V28); CXCL1 (GRO1); CXCL10 (IP-10); CXCL11 (I-TAC/IP-9); CXCL12 (SDF1); CXCL13; CXCL14; CXCL16; CXCL2 (GRO2); CXCL3 (GRO3); CXCL5 (ENA-78/LIX); CXCL6 (GCP-2); CXCL9 (MIG); CXCR3 (GPR9/CKR-L2); CXCR4; CXCR6 (TYMSTR/STRL33/Bonzo); CYB5; CYC1; CYSLTR1; cytokeratins; DAB2IP; DES; DKFZp451J0118; DNCLI; DPP4; E2F1; ECGF1; EDG1; EFNA1; EFNA3; EFNB2; EGF; EGFR; ELAC2; ENG; ENO1; ENO2; ENO3; EPHB4; EPO; ERBB2 (Her-2); EREG; ERK8; ESR1; estrogen receptor; progesterone receptor; ESR2; F3 (TF); FADD; FasL; FASN; FCER1A; FCER2; FCGR3A; FGF; FGF1 ($\alpha$FGF); FGF10;

FGF11; FGF12; FGF12B; FGF13; FGF14; FGF16; FGF17; FGF18; FGF19; FGF2 (bFGF); FGF20; FGF21; FGF22; FGF23; FGF3 (int-2); FGF4 (HST); FGF5; FGF6 (HST-2); FGF7 (KGF); FGF8; FGF9; FGFR1; FGFR3; FIGF (VEGFD); FEL1 (EPSILON); fibrin; FIL1 (ZETA); FLJ12584; FLJ25530; FLRTI (fibronectin); FLT1; FOS; FOSL1 (FRA-1); FY (DARC); GABRP (GABAa); GAGEB1; GAGEC1; GALNAC4S-6ST; GATA3; GDF5; GFI1; GGT1; GM-CSF; GNASI; GNRHI; GPR2 (CCR10); GPR31; GPR44; GPR81 (FKSG80); GRCCIO (C10); GRP; GSN (Gelsolin); GSTP1; HAVCR2; HDAC4; HDAC5; HDAC7A; HDAC9; HGF; HIF1A; HOP1; histamine and histamine receptors; HLA-A; HLA-DRA; HM74; HMOXI ; HPV proteins; HUMCYT2A; ICEBERG; ICOSL; 1D2; IFN-a; IFNA1; IFNA2; IFNA4; IFNA5; IFNA6; IFNA7; IFNB1; IFNgamma; ITGB7; DFNW1; IGBP1; IGF1; IGF1R; IGF2; IGFBP2; IGFBP3; IGFBP6; interleukins such as IL1-IL36 or their receptors, including IL-1; IL10; IL10RA; IL10RB; IL11; IL11RA; IL-12; IL12A; IL12B; IL12RB1; IL12RB2; IL13; IL13RA1; IL13RA2; IL14; IL15; IL15RA; IL16; IL17; IL17B; IL17C; IL17R; IL18; IL18BP; IL18R1; IL18RAP; IL19; IL1A; IL1B; ILIF10; IL1F5; IL1F6; IL1F7; IL1F8; IL1F9; IL1HY1; IL1R1; IL1R2; IL1RAP; IL1RAPL1; IL1RAPL2; IL1RL1; IL1RL2, ILIRN; IL2; IL20; IL20RA; IL21 R; IL22; IL22R; IL22RA2; IL23; IL24; IL25; IL26; IL27; IL28A; IL28B; IL29; IL2RA; IL2RB; IL2RG; IL3; IL30; IL3RA; IL33; IL4; IL4R; IL5; IL5RA; IL6; IL6R; IL6ST (glycoprotein 130); P-glycoprotein; EL7; EL7R; EL8; IL8RA; DL8RB; IL8RB; DL9; DL9R; DLK; INHA; INHBA; INSL3; INSL4; IRAK1; ERAK2; ITGA1; ITGA2; ITGA3; ITGA6 (a6 integrin); ITGAV; ITGB3; ITGB4 (b4 integrin); JAG1; JAK1; JAK3; JUN; K6HF; KAI1; KDR; keratin; KITLG; KLF5 (GC Box BP); KLF6; KLKIO; KLK12; KLK13; KLK14; KLK15; KLK3; KLK4; KLK5; KLK6; KLK9; KRT1; KRT19 (Keratin 19); KRT2A; KHTHB6 (hair-specific type H keratin); kappa light chain; lambda light chain; LAMAS; LEP (leptin); Lingo-p75; Lingo-Troy; LPS; LTA (TNF-b); LTB; LTB4R (GPR16); LTB4R2; LTBR; LEWIS-xMACMARCKS; MAG or Omgp; MAP2K7 (c-Jun); MDK; MIB1; melanosome proteins; midkine; MEF; MIP-2; MKI67; (Ki-67); MMP2; MMP9; MS4A1; MSMB; MT3 (metallothionectin-111); MTSS1; MUC1 (mucin); MYC; MY088; NCK2; neurocan; NFKB1; NFKB2; NGFB (NGF); NGFR; NgR-Lingo; NgR- Nogo66 (Nogo); NgR-p75; NgR-Troy; NME1 (NM23A); NOX5; NPPB; NR0B1; NR0B2; NR1D1; NR1D2; NR1H2; NR1H3; NR1H4; NR112; NR113; NR2C1; NR2C2; NR2E1; NR2E3; NR2F1; NR2F2; NR2F6; NR3C1; NR3C2; NR4A1; NR4A2; NR4A3; NR5A1; NR5A2; NR6A1; NRP1; NRP2; NT5E; NTN4; ODZI; OPRD1; P2RX7; PAP; PART1; PATE; PAWR; PCA3; PCNA; POGFA; POGFB; PECAM1; PF4 (CXCL4); PGF; PGR; phosphacan; PIAS2; PIK3CG; PLAU (uPA); PLG; PLXDC1; PPBP (CXCL7); PPID; PRI; PRKCQ; PRKDI; PRL; PROC; PROK2; PSA; PSAP; PSCA; PTAFR; PTEN; PTGS2 (COX-2); PTN; p53; RAC2 (p21 Rac2); RAS; Rb; RARB; RGSI; RGS13; RGS3; RNF110 (ZNF144); ROBO2; S100A2; SCGB1D2 (lipophilin B); SCGB2A1 (mammaglobin2); SCGB2A2 (mammaglobin 1); SCYEI (endothelial Monocyte-activating cytokine);S-100 SDF2; SERPINA1; SERPINA3; SERP1NB5 (maspin); SERPINE1(PAI-1); SERPDMF1; SHBG; SLA2; SLC2A2; SLC33A1; SLC43A1; SLIT2; SPPI; SPRR1B (Sprl); ST6GAL1; STABI; STAT6; STEAP; STEAP2; TB4R2; TBX21; TCPIO; TOGFI; TEK; TGFA; TGFBI; a transmembrane or cell surface tumor specific antigen (TAA) such as a TAA described in USP 7,521, 541;TAU; TGFB1II; TGFB2; TGFB3; TGFBI; TGFBRI; TGFBR2; TGFBR3; THIL; THBSI (thrombospondin-1 ); THBS2; THBS4; THPO; TIE (Tie-1 ); TMP3; tissue factor; TLR1; TLR2; TLR3; TLR4; TLR5; TLR6; TLR7; TLR8; TLR9; TLR10; Tn antigen TNF; TNF-a; TNFAEP2 (B94); TNFAIP3; TNFRSFIIA; TNFRSF1A; TNFRSF1B; TNFRSF21; TNFRSF5; TNFRSF6 (Fas); TNFRSF7; TNFRSF8; TNFRSF9; TNFSF10 (TRAIL); TNFSF11 (TRANCE); TNFSF12 (AP03L); TNFSF13 (April); TNFSF13B; TNFSF14 (HVEM-L); TNFSF15 (VEGI); TNFSF18; TNFSF4 (OX40 ligand); TNFSF5 (CD40 ligand); TNFSF6 (FasL); TNFSF7 (CD27 ligand); TNFSFS (CD30 ligand); TNFSF9 (4-1 BB ligand); TOLLIP; Toll-like receptors; TOP2A (topoisomerase Ea); TP53; TPM1; TPM2; TRADD; TRAF1; TRAF2; TRAF3; TRAF4; TRAF5; TRAF6; TREM1; TREM2; TRPC6; TSLP; TWEAK; ubiquitin; VEGF; VEGFB; VEGFC; versican; VHL C5; vimentins; VLA-4; XCL1 (lymphotactin); XCL2 (SCM-1b); XCRI(GPR5/ CCXCRI); YY1; and ZFPM2.

In certain embodiments, target molecules for multispecific antibodies purified according to a method provided herein include CD proteins such as CD3, CD4, CD8, CD16, CD19, CD20, CD34; CD64, CD200 members of the ErbB receptor family such as the EGF receptor, HER2, HER3 or HER4 receptor; cell adhesion molecules such as LFA-1, Mac1, p150.95, VLA-4, ICAM-1, VCAM, alpha4/beta7 integrin, and alphav/beta3 integrin including either alpha or beta subunits thereof (e.g., anti-CD11a, anti-CD18, or anti-CD11b antibodies); growth factors such as VEGF (VEGF-A), FGFR, Ang1, KLB, VEGF-C; tissue factor (TF); alpha interferon (alphaIFN); TNFalpha, an interleukin, such as IL-1 beta, IL-3, IL-4, IL-5, IL-S, IL-9, IL-13, IL 17 AF, IL-1S, IL13; IL-13R alpha1, IL13R alpha2, IL14 IL-4R, IL-5R, IL-9R, IgE; blood group antigens; flk2/flt3 receptor; obesity (OB) receptor; mpl receptor; CTLA-4; RANKL, RANK, RSV F protein, protein C, BR3, etc.

In certain embodiments, target molecules for multispecific antibodies purified according to a method provided herein include low density lipoprotein receptor-related protein (LRP)-1 or LRP-8 or transferrin receptor, and at least one target selected from the group consisting of 1) beta-secretase (BACE1 or BACE2), 2) alpha-secretase, 3) gamma-secretase, 4) tau-secretase, 5) amyloid precursor protein (APP), 6) death receptor 6 (DR6), 7) amyloid beta peptide, 8) alpha-synuclein, 9) Parkin, 10) Huntingtin, 11) p75 NTR, and 12) caspase-6.

In certain embodiments, target molecules for multispecific antibodies purified according to a method provided herein include at least two target molecules selected from the group consisting of: IL-1 alpha and IL- 1 beta, IL-12 and IL-1S; IL-13 and IL-9; IL-13 and IL-4; IL-13 and IL-5; IL-5 and IL-4; IL-13 and IL-1beta; IL-13 and IL- 25; IL-13 and TARC; IL-13 and MDC; IL-13 and MEF; IL-13 and TGF-~; IL-13 and LHR agonist; IL-12 and TWEAK, IL-13 and CL25; IL-13 and SPRR2a; IL-13 and SPRR2b; IL-13 and ADAMS, IL-13 and PED2, IL13 and IL17; IL13 and IL4; IL13 and IL33; IL17A and IL 17F, CD3 and CD19, CD138 and CD20; CD138 and CD40; CD19 and CD20; CD20 and CD3; CD3S and CD13S; CD3S and CD20; CD3S and CD40; CD40 and CD20; CD-S and IL-6; CD20 and BR3, TNF alpha and TGF-beta, TNF alpha and IL-1 beta; TNF alpha and IL-2; TNF alpha and IL-3; TNF alpha and IL-4; TNF alpha and IL-5; TNF alpha and IL6; TNF alpha and IL8; TNF alpha and IL-9, TNF alpha and IL-10, TNF alpha and IL-11, TNF alpha and IL-12, TNF alpha and IL-13, TNF alpha and IL-14, TNF alpha and IL-15, TNF alpha and IL-16, TNF alpha and IL-17, TNF alpha and IL-18, TNF alpha and IL-19, TNF alpha and IL-20, TNF alpha and IL-23, TNF alpha and IFN alpha, TNF alpha and CD4, TNF alpha and VEGF, TNF alpha and MIF, TNF alpha and ICAM-1, TNF alpha and PGE4, TNF alpha and PEG2, TNF alpha and RANK ligand, TNF alpha and Te38, TNF alpha and BAFF,TNF alpha and CD22, TNF alpha and CTLA-4, TNF alpha and GP130, TNF a and IL-12p40, FGFR1 and KLB;VEGF and HER2, VEGF-A and HER2, VEGF-A and PDGF, HER1 and HER2, VEGFA and ANG2,VEGF-A and VEGF-C, VEGF-C and VEGF-D, HER2 and DR5,VEGF and IL-8, VEGF and MET, VEGFR and MET receptor, EGFR and MET, VEGFR and EGFR, HER2 and CD64, HER2 and CD3, HER2 and CD16, HER2 and HER3; EGFR (HER1) and HER2, EGFR and HER3, EGFR and HER4, IL-14 and IL-13, IL-13 and CD40L, IL4 and CD40L, TNFR1 and IL-1 R, TNFR1 and IL-6R and TNFR1 and IL-18R, EpCAM and CD3, MAPG and CD28, EGFR and CD64, CSPGs and RGM A; CTLA-4 and BTN02; IGF1 and IGF2; IGF1/2 and Erb2B; MAG and RGM A; NgR and RGM A; NogoA and RGM A; OMGp and RGM A; POL-1 and CTLA-4; and RGM A and RGM B.

Soluble antigens or fragments thereof, optionally conjugated to other molecules, can be used as immunogens for generating antibodies. For transmembrane molecules, such as receptors, fragments of these (e.g., the extracellular domain of a receptor) can be used as the immunogen. Alternatively, cells expressing the transmembrane molecule can be used as the immunogen. Such cells can be derived from a natural source *(e.g.,* cancer cell lines) or may be cells which have been transformed by recombinant techniques to express the transmembrane molecule. Other antigens and forms thereof useful for preparing antibodies will be apparent to those in the art.

### *Formulations and Methods of Making of the Formulations*

Also provided herein are formulations and methods of making the formulation comprising the multispecific antibodies purified by the methods described herein. For example, the purified polypeptide may be combined with a pharmaceutically acceptable carrier.

The polypeptide formulations in some embodiments may be prepared for storage by mixing a polypeptide having the desired degree of purity with optional pharmaceutically acceptable carriers, excipients or stabilizers *(*Remington's Pharmaceutical Sciences 16th edition, Osol, A. Ed. (1980)), in the form of lyophilized formulations or aqueous solutions.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution.

Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

In some embodiments, the polypeptide in the polypeptide formulation maintains functional activity.

The formulations to be used for *in vivo* administration must be sterile. This is readily accomplished by filtration through sterile filtration membranes.

The formulations herein may also contain more than one active compound as necessary for the particular indication being treated, preferably those with complementary activities that do not adversely affect each other. For example, in addition to a polypeptide, it may be desirable to include in the one formulation, an additional polypeptide (e.g., antibody). Alternatively, or additionally, the composition may further comprise a chemotherapeutic agent, cytotoxic agent, cytokine, growth inhibitory agent, anti-hormonal agent, and/or cardioprotectant. Such molecules are suitably present in combination in amounts that are effective for the purpose intended

### *Articles of Manufacture*

The multispecific antibodies purified by the methods described herein and/or formulations comprising the polypeptides purified by the methods described herein may be contained within an article of manufacture. The article of manufacture may comprise a container containing the polypeptide and/or the polypeptide formulation. Preferably, the article of manufacture comprises:(a) a container comprising a composition comprising the polypeptide and/or the polypeptide formulation described herein within the container; and (b) a package insert with instructions for administering the formulation to a subject.

The article of manufacture comprises a container and a label or package insert on or associated with the container. Suitable containers include, for example, bottles, vials, syringes, etc. The containers may be formed from a variety of materials such as glass or plastic. The container holds or contains a formulation and may have a sterile access port (for example the container may be an intravenous solution bag or a vial having a stopper pierceable by a hypodermic injection needle). At least one active agent in the composition is the polypeptide. The label or package insert indicates that the composition's use in a subject with specific guidance regarding dosing amounts and intervals of polypeptide and any other drug being provided. The article of manufacture may further include other materials desirable from a commercial and user standpoint, including other buffers, diluents, filters, needles, and syringes. In some embodiments, the container is a syringe. In some embodiments, the syringe is further contained within an injection device. In some embodiments, the injection device is an autoinjector.

A "package insert" is used to refer to instructions customarily included in commercial packages of therapeutic products, that contain information about the indications, usage, dosage, administration, contraindications, other therapeutic products to be combined with the packaged product, and/or warnings concerning the use of such therapeutic products.

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

The foregoing written description is considered to be sufficient to enable one skilled in the art to practice the methods and/or obtain the compositions described herein. The following examples and detailed description are offered by way of illustration and not by way of limitation.

The disclosures of all references in the specification are expressly incorporated herein by reference.

DESCRIPTION OF THE SEQUENCE LISTING

| SEQ ID NO: 1 | variable heavy chain domain VH of <VEGF> of vanucizumab |
| SEQ ID NO: 2 | variable light chain domain VL of <VEGF> of vanucizumab |
| SEQ ID NO: 3 | variable heavy chain domain VH of <ANG-2> of vanucizumab |
| SEQ ID NO: 4 | variable light chain domain VL of < ANG-2> of vanucizumab |
| SEQ ID NO: 5 | variable heavy chain domain VH of <VEGF> of RG7716 |
| SEQ ID NO: 6 | variable light chain domain VL of <VEGF> of RG7716 |
| SEQ ID NO: 7 | variable heavy chain domain VH of <ANG-2> of RG7716 |
| SEQ ID NO: 8 | variable light chain domain VL of < ANG-2> of RG7716 |
| SEQ ID NO: 9 | heavy chain of <ANG-2> of vanucizumab |
| SEQ ID NO: 10 | heavy chain of <VEGF> of vanucizumab |
| SEQ ID NO: 11 | light chain of <ANG-2> of vanucizumab |
| SEQ ID NO: 12 | light chain of <VEGF> of vanucizumab' |
| SEQ ID NO: 13 | heavy chain of <VEGF> of RG7716 |
| SEQ ID NO: 14 | heavy chain of <ANG-2> of RG7716 |
| SEQ ID NO: 15 | light chain of <VEGF> of RG7716 |
| SEQ ID NO: 16 | light chain of <ANG-2> of RG7716 |

EXAMPLES

The Examples are offered for illustrative purposes only, and are not intended to limit the scope of the present invention in any way. Indeed, various modifications in addition to those shown and described herein will become apparent to those skilled in the art from the foregoing description and fall within the scope of the appended claims.

***Example 1: Assembly and Purification of an anti-X1/anti-Y1 Bispecific Antibody***

A bispecific antibody against target proteins X1 and Y1 - anti-X1/anti-Y1 bispecific antibody or aX1/Y1 bispecific - was assembled as follows. Each half-antibody (aX1 (knob) and aY1 (hole)) was independently subject to an affinity chromatography step using protein A resin (MabSelect SuRe, GE Healthcare). The protein A step is completed independently for each half-antibody using similar process conditions but different load density targets. Protein A columns were run at ambient temperature (15 - 30°C) and the load was chilled to 12 - 18°C. Protein A columns were prepared by applying three column volumes of elution buffer followed by three column volumes of regeneration buffer. The columns were then equilibrated, loaded, washed three times (equilibration buffer wash, potassium phosphate wash, equilibration buffer wash), eluted, and regenerated for sufficient cycles to process the load material. The pooled material from the protein A column was pH adjusted, if necessary, by the addition of elution buffer to achieve pH ≤ 3.60 and held for a minimum of 120 minutes. Following this step, the pH of the pooled material was adjusted to pH 5.0 ± 0.3 for further processing.

The half-antibody pools obtained from the protein A chromatography step were then combined in a 1:1 molar ratio, the pH was adjusted to pH 8.2. 200 mM L-glutathione (91/9% GSH/GSSG) buffer was added to the combined pools to achieve a ratio of 165 moles of L-glutathione for every 1 mole of bispecific being formed. The material was heated to 32.0 ± 2.0°C for 8 - 24 hours. The resulting assembled pool was cooled to 15 - 25°C and then adjusted to pH 5.5.

The pH adjusted assembled pool was then subjected to a multimodal cation exchange chromatography using Capto™ MMC resin in a bind and elute mode. The column was equilibrated with 100 mM sodium acetate, pH 5.5. The adjusted assembly pool was loaded onto the column to 45 g/L resin and followed by a wash with equilibration buffer and a second wash phase of 50 mM HEPES with 25 mM sodium acetate at pH 8.0. The bispecific antibody was eluted off the column by increasing both salt and pH in a step elution, using 50 mM HEPES with 245 mM sodium acetate at pH 8.0. Cation exchange pooling was initiated and terminated based on absorbance at 280 nm.

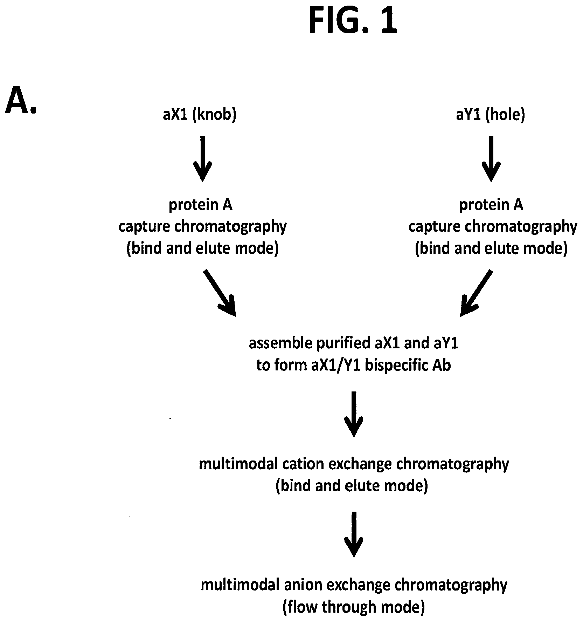

Eluate from the multimodal cation chromatography step was then subjected to multimodal anion exchange chromatography using Capto™ Adhere resin in a flow-through mode. The Capto™ Adhere column was equilibrated with 50 mM Tris, 85 mM sodium acetate, pH 8.0. The product pool from the preceding step was adjusted to a conductivity of 9.0 mS/cm with purified water and loaded onto the column. The bispecific antibody flowed through the column, which was then washed with equilibration buffer. Anion exchange pooling was initiated and terminated based on absorbance at 280 nm. The purification scheme described above is depicted in FIG. 1A.

The third chromatography step removed residual impurities like DNA, host cell protein, and endotoxins as well as product-variants including half-antibodies, homodimers and aggregates. When the Capto™ Adhere load was spiked with 20% aY1 homodimer, the chromatography process decreased the aY1 homodimer approximately 2-fold (8% by MS and 10% by a cell based assay for detecting product-related impurities).

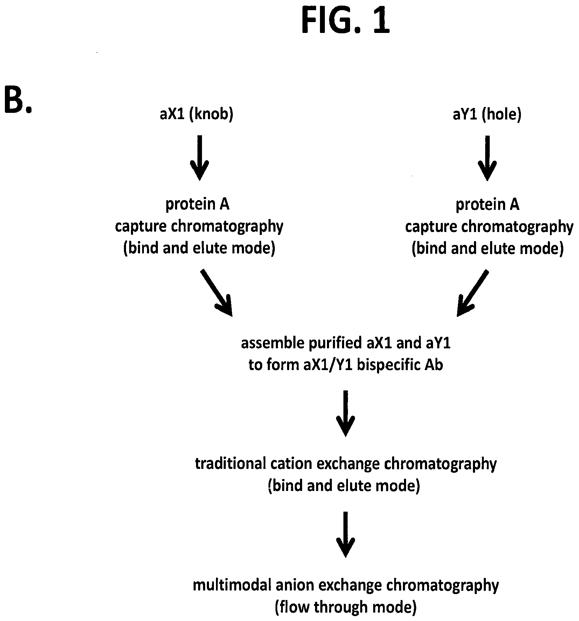

Comparison of the purification scheme shown in **FIG. 1A)** with a purification scheme using a traditional cation exchange (CEX) resin (POROS XS) (*see* **FIG. 1B)** showed that use of the multimodal resin improved separation of the bispecific antibody from product-related variants, especially homodimers. Thus, when the pooled material from the affinity chromatography step was spiked with 20% hole homodimers, multimodal cation exchange chromatography decreased the hole homodimer to below 2% by mass spectrometry (MS) and below limit of quantitation of 0.5% by a cell based assay for product-related impurities.

*Table 7: Separation of anti-X1/anti-Y1 Bispecific Antibody from Host Cell- and Product-Related Impurities (POROS XS Resin, Bind/Elute Mode)*

| Pool | Elution Buffer | Yield (%) | CHOP (ng/mg) | SEC % (HMWS, 150 kD, 75 kD, LMWS)‡ | | | |
|------|---------------|-----------|--------------|------|------|-----|-----|
| LOAD | -- | -- | 2060 | 8.8 | 80.5 | 6.9 | 3.8 |
| Pool | 127 mM NaOAc, pH 6.5 | 90 | 654 | 0.3 | 90.3 | 8.0 | 1.4 |

*Table 8: Separation of Bispecific Antibody from Host Cell- and Product-Related Impurities (Capto MMC Resin, Bind/Elute Mode)*

| Pool | Elution Buffer | Yield (%) | CHOP (ng/mg) | SEC % (HMWS, 150 KD, 75 kD, LMWS)‡ | | | |
|------|---------------|-----------|--------------|------|------|-----|-----|
| LOAD | -- | -- | 4080 | 8.4 | 89.3 | 1.4 | 0.3 |
| Pool | 244 mM NaOAc, pH 8.0 | 80 | 236 | 0.9 | 98.5 | 0.5 | 0.1 |
| **CHOP** = CHO cell protein; **HMWS** = high molecular weight species; **150 kD** = bispecific and homodimer; **75kD** = half antibody; **LMWS** = low molecular weight species | | | | | | | |

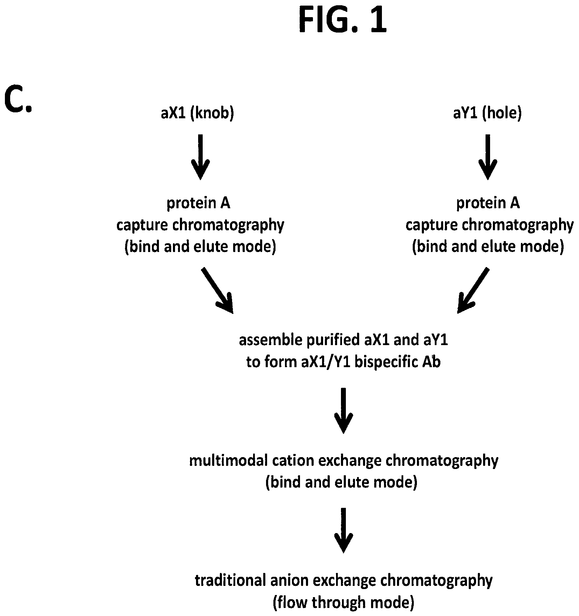

Comparison between a traditional anion exchange (AEX) resin (QSFF) *(see* **FIG. 1C)** and a multimodal AEX resin (Capto™ Adhere) *(see* **FIG. 1A),** as shown in Tables 9 and 10, demonstrates that the multimodal AEX resin achieved significantly better separation of the bispecific antibody from product-related impurities as compared to the traditional AEX QSFF resin. QSFF enriched main peak by 1%, and Adhere enriched main peak by 10%, due to the removal of half-antibody (75 kD) and aggregates, *i.e.,* high molecular weight species (HMWS). A direct comparison of Capto Adhere and QSFF using the same Capto MMC pool showed that the Capto Adhere resin achieved better clearance of size-variants, product related variants including half antibody, and host-cell protein clearance as compared to QSFF (Table 11).

*Table 9: Separation of Bispecific Antibody from Host Cell- and Product-Related Impurities (QSFF Resin, Flow-Through Mode)*

| Load pH | Load Conductivity (mS/cm) | Yield (%) | CHOP (ng/mg) | SEC % (HMWS, 150 kD, 75 kD, LMWS)‡ | | | | Anti-Y1 (hole-hole) Homodimer Mass Spec (%) |
|---|---|---|---|---|---|---|---|---|
| LOAD | -- | -- | ~300 | 0.5 | 92.5 | 5.9 | 1.1 | -- |
| 8.0 | 5.1 | 93 | <15 | 0.6 | 93.5 | 4.4 | 1.5 | 3 |
| 8.0 | 9.0 | 97 | <9.8 | 0.6 | 92.4 | 5.3 | 1.7 | 3 |

**CHOP** = CHO cell protein; **HMWS** = high molecular weight species; **150 kD** = bispecific and homodimer; **75kD** = half antibody; **LMWS** = low molecular weight species

*Table 10: Separation of Bispecific Antibody from Host Cell- and Product-Related Impurities (Capto™ Adhere Resin, Flow-Through Mode)*

| Column Run | | Load pH | Load Conductivity (mS/cm) | Total Protein Yield (%) | CHOP (ppm) | SEC-HPLC % HMWS | SEC-HPLC % 75 kD | Hole-Hole homodimer Mass Spec (%) |
|---|---|---|---|---|---|---|---|---|
| 1 | **Load** | 8.0 | 9.0 | - | 276 | 0.5 | 1.6 | |
| | **Pool** | -- | -- | 85 | 1.5 | 0.2 | 0.0 | <2% (LOQ) |
| 2 | **Load** | 8.5 | 9.0 | -- | 276 | 0.3 | 1.5 | |
| | **Pool** | -- | -- | 76 | 2.0 | 0.1 | 1.4 | <2% (LOQ) |

**CHOP** = CHO cell protein; **HMWS** = high molecular weight species; **75kD** = half antibody; **LOQ** = limit of quantification

*Table 11: Comparison of Capto Adhere and QSFF*

| | Load pH | Load Conductivity (mS/cm) | Total Protein Yield (%) | CHOP (ppm) | SEC-HPLC % HMWS | SEC-HPLC % 75 kD |
|---|---|---|---|---|---|---|
| **Load** | -- | -- | - | 32 | 1.1 | 1.3 |
| **Capto Adhere Pool** | 8.0 | 9.0 | 91 | 0.9 | 0.55 | 0.48 |
| **QSFF Pool** | 8.0 | 9.0 | 100 | < 8.5 | 1.4 | 1.4 |

**CHOP** = CHO cell protein; **HMWS** = high molecular weight species; **75kD** = half antibody; **LOQ** = limit of quantification

The experiments described above demonstrate that the purification scheme depicted in **FIG. 1A** achieved significantly better separation of aX1/Y1 bispecific antibody from product-related impurities as compared to the purification schemes in **FIG. 1B** or **FIG. 1C.** First, when the pooled material from the affinity chromatography step was spiked with 20% hole homodimers, Capto MMC *(i.e.,* multimodal cation exchange resin) decreased the hole homodimer to below 2% by mass spectrometry (MS) and was below limit of quantitation of 0.5% by a cell based assay for product-related impurities. (See Tables 7 and 8). Such separation was not achieved using POROS XS, *i.e.,* a traditional cation exchange resin. In addition, CaptoAdhere *(i.e.,* a multimodal anion exchange resin) enriched main peak by 10%, due to the removal of high molecular weight species, such as half-antibody (75 kD) and aggregates, whereas QSFF *(i.e.,* a traditional anion exchange resin) enriched main peak by only 1%. Further, the combination of two multi-model resins, a multimodal cation resin (CaptoMMC) followed by a multimodal anion resin CaptoAdhere achieved better clearance of size-variants, product related variants including half antibody, and host-cell protein clearance as compared to the combination of the multimodal cation resin followed by a traditional anion exchange resin, QSFF (*see e.g.,* Table 11).

Similar improvement in the removal of product-related variants and size variants was found when the order of the multimodal resins was reversed: In a separate experiment, better separation of aX1/Y1 bispecific antibody from product-related impurities was also achieved by subjecting the pooled material from the affinity chromatography step first to a multimodal anion resin followed by a multimodal cation resin.

***Example 2: Assembly and Purification of a F(ab')$_2$ Bispecific***

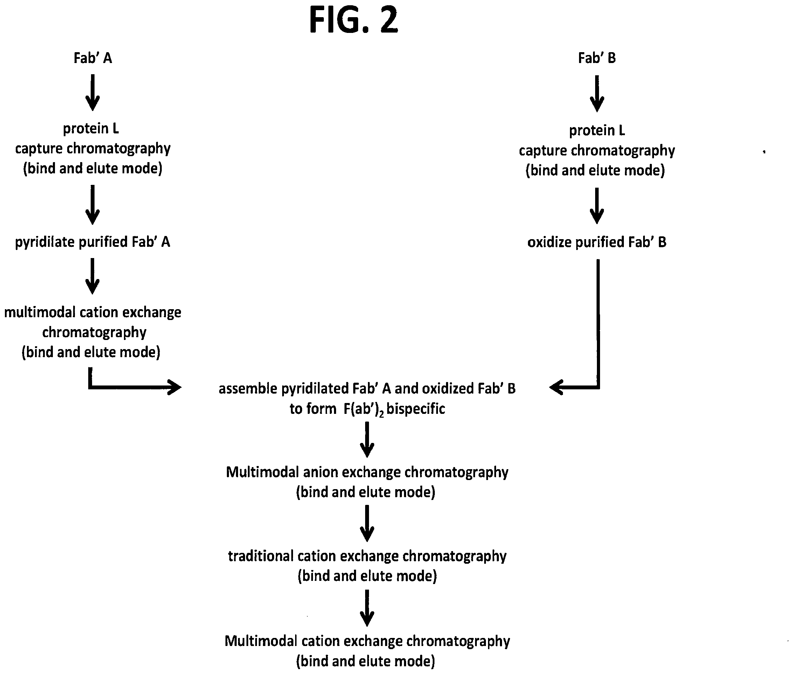

Initial attempts to achieve 90% pure F(ab')$_2$ bispecific resulted in low yields (less than 10% starting material). Adding to the problem of maintaining acceptable yields without a loss in purity were several challenges, including the instability of process intermediates and the presence of product-related variants, such as homodimers, free light chains and heavy chains, and unreacted Fab' leaving groups. Novel unit operations were developed in order to achieve effective assembly and purification of the desired bispecific F(ab')$_2$. A bispecific F(ab')2 comprising two different Fab' molecules was assembled and purified as depicted in the schematic provided in **FIG. 2.**

First, a capture step was implemented as follows. Each Fab' was first captured from separate *E. coli* extract supernatants. Supernatants containing one of the two Fab' half-molecules were subjected to a capture step using CaptoL Protein L affinity chromatography resin. The column is equilibrated using a 25mM Tris sodium chloride equilibration buffer (pH 7.7). Following application of the load material to the column, the column was washed with equilibration buffer (pH 7.7), followed by a wash with 0.4M potassium phosphate (pH 7), a wash with reductant to remove cysteine caps, and an additional wash with equilibration buffer, pH 7.7. The Fab' product of interest was then eluted from the CaptoL column using an elution buffer of 0.1M acetic acid pH 2.9. The product was collected using absorbance at 280nm.

The pool from Capto L chromatography step containing a first Fab' half-molecule (Fab' A) was adjusted to pH 5.5. DPDS (Dipyridyl disulfide) was added to the pH 5.5 adjusted CaptoL pool. DPDS reacts with free hinge cysteine in the Fab' molecule to form a pyridylated Fab' which reacts with available Fab' free thiol, thereby promoting the formation of F(ab')$_2$ heterodimers. Once formed, the pyridylated Fab' A was loaded on a second chromatography column for purification.

In order to identify the chromatography conditions under which pyridilated Fab' can be separated from impurities (*i.e.,* Fab' homodimer, high molecular weight species (HMWS), Fab' monomer), the binding behaviors of Fab' homodimer, high molecular weight species (HMWS), Fab' monomer, and pyridylated Fab'(pyr-Fab') on Capto MMC resin were characterized in a 96-point partition coefficient screen of chromatography resin binding conditions. A pyridylated Fab' pool was loaded onto a Capto™ MMC resin. Fab' monomer was predicted to elute earlier in the gradient the pyr-Fab', whereas HMWS and Fab' homodimer were predicted to elute later in the gradient. The second Fab' molecule, Fab' B, was eluted from CaptoL chromatography resin and then oxidized.

The two Fab' pools, containing pyridylated Fab'A and oxidized Fab'B, were then subjected to conditions suitable for assembly of the F(ab')$_2$ bispecific molecule. Pyridylated Fab'A and oxidized Fab' B CaptoL pool were combined. The combined pool was held for a minimum assembly time to allow for the formation of the F(ab')$_2$ bispecific. The assembly pool was then conditioned for loading onto the next chromatography column.

A low-resolution Kp (*i.e.,* partition coefficient) screen was performed to characterize the binding behavior of the F(ab')$_2$ assembly mixture on different chromatography resins. An assembly mixture (0 % aggregate, 21.5 % Fab' A homodimer, 43.9 % F(ab')$_2$, 10.3 % Fab' A monomer, 8.8% pyridylated Fab' A, and 15.5 % Fab' B monomer, as measured by SEC-HPLC) was tested by loading onto the following resins at 5 g/L$_{resin}$ load density: Capto™ MMC resin, Capto™ Adhere resin, QSFF resin, or POROS® 50HS resin. The protein composition and protein concentration of each flow-through plate was analyzed via SEC-HPLC. These data were de-convoluted and used to generate contour plots corresponding to the behavior of the F(ab')$_2$, Fab' A homodimer, Fab' B homodimer, Fab' A monomer, and Fab' B monomer on each of the four resins under the test conditions.

Based on the contour plots, Capto™ Adhere was predicted to resolve Fab' A monomers, Fab' A homodimers, and Fab' B homodimers. Specifically, in a bind and pH-gradient elution on Capto™ Adhere resin, Fab' A monomer and Fab' B homodimer were predicted to elute earlier than the F(ab')$_2$ main peak, whereas the Fab' A homodimer was predicted to remain bound to the resin. Contour plots for QSFF showed that none of the species were predicted to bind to QSFF resin for the pH ranges tested, indicating that separation of the F(ab')$_2$ from product-related impurities would not be achieved via QSFF chromatography. The mixed mode resins provided the best separation of the F(ab')$_2$ from the product related impurities under the experimental conditions. Capto™ MMC contour plots showed that Capto™ MMC was predicted to separate the F(ab')$_2$ from its product-specific impurities effectively at pH 5.5.

Following assembly of the F(ab')$_2$ bispecific, the assembly pool is subjected to multimodal AEX chromatography, using CaptoAdhere resin. The assembly pool is titrated to pH 7.5 and diluted to a conductivity of $\leq$ 5.5 mS/cm. The column is equilibrated with 25mM sodium acetate 50mM Tris pH 7.5 equilibration buffer. The assembly pool is loaded onto to column at a load density of 25 g/L resin. The column is then washed with equilibration buffer. The column is eluted using 25mM sodium acetate 45mM MES 5mM Tris pH 5.5 elution buffer and the elution pool is pooled by A280 absorbance.

Following, multimodal AEX, the material is loaded onto a Poros 50 HS resin operated in bind and elute mode. The Poros 50 HS column is equilibrated with 52mM sodium acetate pH 4.9. The load is conditioned to pH 5 and conductivity ≤ 3.3 mS/cm. The column is washed with equilibration buffer. The column is then washed with 169 mM sodium acetate pH 4.9. The column is eluted using a step elution using a 247 mM sodium acetate pH 4.9 elution buffer. The pool is collected by A280.

The pool from the POROS 50 HS step is then subjected to multimodal CEX chromatography using CaptoMMC resin. The multimodal CEX step is operated in bind and elute mode. The column is equilibrated using 50 mM sodium acetate pH 5.5 equilibration buffer. The load is adjusted to pH 5.0 and conductivity ≤5 mS/cm and loaded onto the column to a load density of 15 g/L. The column is washed with equilibration buffer. The column is then washed with 140mM sodium acetate pH 5.5 wash 2 buffer. The column is eluted with a gradient elution using equilibration buffer and a 350mM sodium acetate pH 5.5 elution buffer. The pool is collected by A280.

As shown in **Table 12,** improved separation of the F(ab')$_2$ bispecific product of interest (100 kD) from *E. coli* proteins, and 71kD misformed disulfide product related variant was achieved when using Capto™ MMC resin compared to when using POROS®HS resin. Purity of greater than 95% Fab'2 bispecific as measured by SEC was achieved using CaptoMMC as the fourth column. Less than 5% of 71kD misformed disulfide product related variant was also achieved using CaptoMMC as the fourth column.

**Table 12: Separation of F(ab')$_2$ from Host Cell- and Product-Related Impurities (POROS® HS Resin, Bind and Elute Mode vs. Capto™ MMC, Bind and Elute Mode)**

| Pool | Yield (%) | % F(ab')$_2$ (SEC) | % 100 kD Species | % 71 kD Species | ECP (ppm) |
|---|---|---|---|---|---|
| **Load material (Capto Adhere Pool)** | -- | 72.7 | 66.6 | 4.7 | 547 |
| **Poros® Pool (4$^{th}$ Column)** | 72 | 93.9 | 90.1 | 5.2 | 42 |
| **Capto™ MMC Pool (4$^{th}$ Column)** | 60 | 96.0 | 93.1 | 4.5 | 21 |
| **% F(ab')$_2$** = % Bispecific measured by SEC; **100 kD** = F(ab')$_2$ bispecific product of interest measured by CE-SDS; **71 kD** = product related variant with misformed disulfide measured by CE-SDS; **ECP** = E. coli proteins | | | | | |

Thus, the purification scheme depicted in **FIG. 2** significantly improved yield of pure F(ab')$_2$ and reduced the amount of host cell protein in the purified F(ab')$_2$ pool by over 99%, as compared to the load material.

### *Example 3: Assembly and Purification of an anti-X2/anti-Y2 Bispecific Antibody*

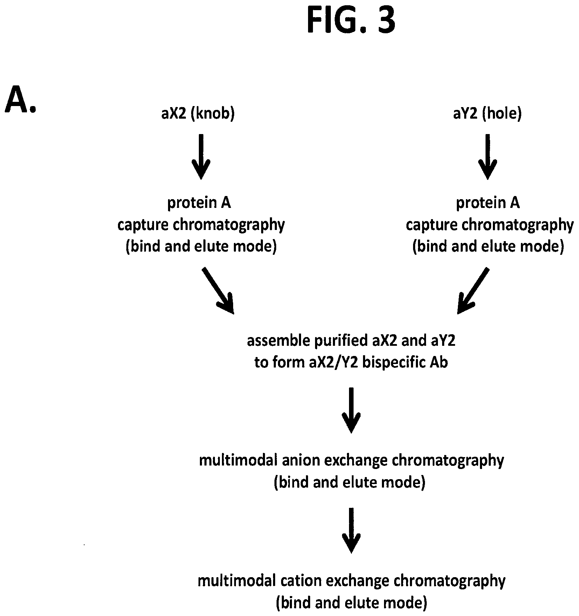

In another example, a bispecific antibody was purified as follows. Each half antibody was produced separately and subjected to affinity chromatography, followed by assembly as described herein. Following assembly, the assembly material was first subjected to multimodal anion exchange chromatography using Capto™ Adhere resin in a bind and elute mode. The assembly material was adjusted to pH 7.5 and loaded onto the column that was pre-equilibrated with 150 mM acetate/Tris buffer, pH 7.5. Following loading, the column was washed with equilibration buffer and the bound protein was eluted with 25mM acetate, pH 5.0. Collection of elution pool was triggered based on A280nm signal. The Capto™ Adhere elution pool was then subjected to multimodal cation exchange chromatography using Capto™ MMC resin in a bind and elute mode. The Capto Adhere elution pool was adjusted to pH 6.5 and loaded on to Capto MMC column pre-equilibrated in 25mM acetate, 25mM MES pH 6.5 buffer. Following loading, the Capto™ MMC column was washed with the equilibration buffer and the bound protein was eluted with 150mM Na-acetate, 25mM MES pH 6.5. Capto™ MMC elution buffer. Pool collection was based on A280nm signal. This purification scheme is depicted in **FIG. 3A**.

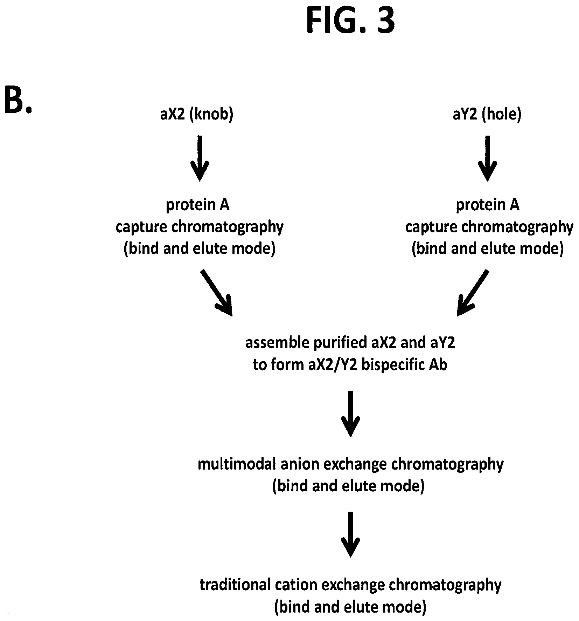

As shown below in **Tables 13** and **14,** greater separation of bispecific antibody from process-specific impurities such as E. *coli* proteins and chaperones (*e.g.,* fkpA, dsbA, and dsbC) was achieved when Capto™ Adhere chromatography was followed by Capto™ MMC chromatography (*see* **FIG. 3A**), as compared to Capto™ Adhere chromatography followed by QSFF chromatography (*see* **FIG. 3B**). Moreover, greater separation of bispecific antibody from product-specific impurities such as very high molecular weight species (vHMWS), high molecular weight species (HMWS), and low molecular weight species (LMWS) was achieved when Capto™ Adhere chromatography was followed by Capto™ MMC chromatography (*see* **FIG. 3A**), as compared to Capto™ Adhere chromatography followed by QSFF chromatography (*see* **FIG. 3B**). *See* **Tables 13** and **14.**

*Table 13: Clearance of process- and product-specific impurities achieved with Capto™ Adhere-QSFF steps in anti-X2/Y2 process*

| Step | Levels in ppm | | | | Levels in % | | | |
|---|---|---|---|---|---|---|---|---|
| | ECP | FkpA | DsbA | DsbC | vHMWS | HMWS | Main | LMWS |
| **anti-X2 MSS** | 6872 | 3983 | 38 | 80 | 0.1 | 4.1 | NA | NA |
| **anti-Y2 MSS** | 7640 | 2660 | 46 | 120 | 0.5 | 11.5 | NA | NA |
| **Assembly** | 3441 | 1841 | 31 | 94 | 3.4 | 11.0 | 82 | 0.5 |
| **Capto™ Adhere** | 118 | 1689 | 5 | 35 | 1.6 | 5.8 | 95 | 0.0 |
| **QSFF** | 72 | 520 | 3 | 31 | 0.0 | 1.4 | 98 | 0.1 |

*Table 14: Clearance of process and product related impurities achieved with Capto™ Adhere-Capto™ MMC steps in anti-X2/Y2 process*

| Step | Levels in ppm | | | | Levels in % | | | |
|---|---|---|---|---|---|---|---|---|
| | ECP | FkpA | DsbA | DsbC | vHMWS | HMWS | Main | LMWS |
| **anti-X2 MSS** | 27090.5 | 3124 | 17 | 59 | 0.35 | 5.6 | NA | NA |
| **anti-Y2MSS** | 5113.5 | 2632 | 23 | 79 | 3 | 11.6 | NA | NA |
| **Assembly** | 1566 | 1782 | 18 | 39 | 6.1 | 8.2 | 83.7 | 2.1 |
| **Capto™ Adhere** | 49.5 | 1387 | 5 | 107 | 1.45 | 2.1 | 95.8 | 0.6 |
| **Capto™ MMC** | 15 | 96 | 1 | 2 | 0.15 | 0.25 | 99.55 | 0 |

The experiments described above demonstrate that the purification scheme depicted in **FIG. 3A** (*i.e.,* in which multimodal anion exchange chromatography was followed by multimodal cation exchange chromatography) achieved improved separation of anti-X2Y2 bispecific antibody from process and product related impurities as compared to the purification scheme depicted in **FIG. 3B** (*i.e.,* in which multimodal anion exchange chromatography was followed by traditional cation exchange chromatography). Additionally, the purification scheme depicted in **FIG. 3A** also achieved improved yield of anti-X2Y2 bispecific antibody as compared to the purification scheme depicted in **FIG. 3B**.

### Example 4: Assembly and Purification of an anti-X3/anti-Y3 Bispecific Antibody

The anti-X3 knob half antibody was captured on a Protein A column. The column was first equilibrated using 25mM Tris 25mM sodium chloride pH 7.7 equilibration buffer. *E.coli* extract supernatant containing anti-X3 half antibody was then loaded onto the column. Following loading of the extract supernatant, the column was washed with equilibration buffer, followed by 0.4M potassium phosphate pH 7 wash buffer, and then washed with equilibration buffer. The anti-X3 half antibody was then eluted using 0.15M acetic acid pH 2.9 elution buffer. The elution pool was collected by A280. The elution pool was titrated to pH 5.0 and then stored until combination with anti-Y3 hole half antibody. The anti-Y3 hole half antibody was captured using the same Protein A process described for anti-X3 half antibody.

The two half antibodies were combined in a 1:1 mass ratio. Arginine is added to the assembly pool to a final concentration of 50mM. The pool of combined half antibodies was diluted 1:1 with 200mM histidine, 8% PVP pH 8. L-reduced glutathione was added to a molar excess of 200X (200 moles of glutathione per mole of bispecific antibody) to assemble the two half antibodies. The assembly pool was titrated to pH 8.0 and then heated to 35 degrees Celsius for six hours. The pool was then cooled to room temperature and adjusted for loading on the next chromatography column.

The assembly pool was loaded onto a QSFF anion exchange column. The column was first pre-equilibrated with 25mM Tris 350mM sodium chloride pH 9.1, followed by 25mM Tris 70mM Sodium Chloride pH 9.1 equilibration buffer. The adjusted load was then applied to the column at pH 8.5 conductivity $\leq$ 4.9 mS/cm. The column was then washed with equilibration buffer. The pool was then eluted using equilibration buffer and 25mM Tris 350mM sodium chloride elution buffer. The elution pool was collected by A280.

The QSFF pool was then adjusted to load onto the next column. A CaptoAdhere multimodal anion exchange column was pre-equilibrated with a 500mM sodium acetate pH 6.0 pre-equilibration buffer, followed by equilibration with eight column volumes of 50mM sodium acetate pH 6.0 equilibration buffer. The adjusted load at pH 6.0 conductivity $\leq$ 12 mS/cm was applied to the column. The column was then washed with equilibration buffer, followed by 0.1M arginine pH 7.0 conductivity 7.5 mS/cm wash buffer, and then washed with equilibration buffer. The column was then eluted with a gradient elution using 50mM sodium acetate pH 5.0 elution buffer. The elution pool was collected by A280.

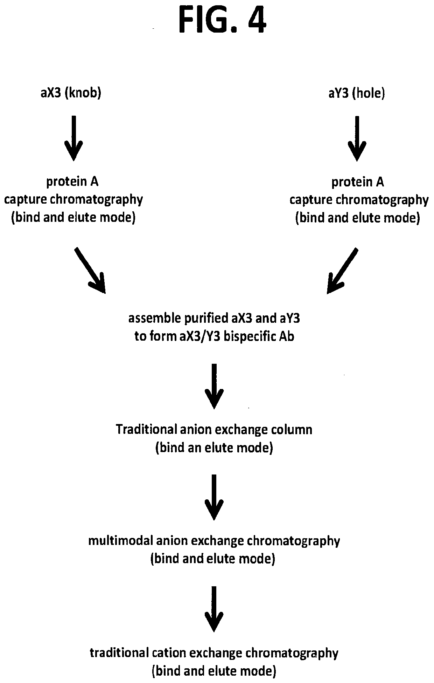

The CaptoAdhere pool was then adjusted to load onto the next column. A CaptoMMC multimodal cation exchange column was pre-equilibrated with a 350mM sodium acetate pH 6.0 pre-equilibration buffer, followed by equilibration with 50mM sodium acetate pH 6.0 equilibration buffer. The adjusted load at pH 6.0 conductivity ≤ 6.5 mS/cm was applied to the column. The column was then washed with 80mM sodium acetate pH 6.0 wash buffer. The column was then eluted with a gradient elution using 350 mM sodium acetate pH 6.0 elution buffer. The elution pool was collected by A280. This purification scheme is depicted in **FIG. 4.**

As shown below in **Table 15,** a three-column process (*i.e.,* Protein A, followed by QSFF, followed by Capto™ Adhere) comprising only one multimodal column did not achieve sufficient ECP removal. Subjecting the eluate of Capto™ Adhere chromatography to a fourth chromatography column using Capto™ MMC chromatography reduced the level of *E. coli* protein by greater than threefold relative to the Capto™ Adhere pool, lowered the HMWS to less than 1%, and increased the bispecific content to 100%.

**Table 15**

| Step | % Yield | % HMWS | % Bispecific | ECPs (ng/mg) |
|---|---|---|---|---|
| **anti-X3 MSS** | 101 | 4 | - | 1513 |
| **anti-Y3 MSS** | 89 | 6 | - | 1864 |
| **Assembly** | 100 | 11.4 | 88 | 2022 |
| **QSFF** | 65 | 1.6 | 96 | 597 |
| **CaptoAdhere** | 89 | 1.3 | 98 | 297 |
| **CaptoMMC** | 76 | 0.7 | 100 | 86 |
| **HMWS** = high molecular weight species measured by SEC; % **Bispecific** = % of Bispecific antibody measured by Reverse Phase HPLC; **ECPs** = E.coli host cell proteins | | | | |

### Example 5: Materials & Methods for Examples 6 and 7

### Antibodies

Examples 6-7 use various exemplary antibodies, including: a bispecific antibody that binds Ang2 and VEGF-A (anti-Ang2/VEGF-A antibody; vanucizumab; RG7221) as described in WO 2011/117329 or SEQ ID NO: 1 to SEQ ID NO: 4 or a bispecific antibody against VEGF-A and Ang2 (anti-VEGF-A/Ang2 antibody; RG7716) as described in WO 2014/009465 or SEQ ID NO: 5 to SEQ ID NO: 8. Also included herein are a number of antibodies, as described in the Examples below.

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.); Antibodies, A Laboratory Manual, and Animal Cell Culture (R.I. Freshney, ed. (1987)); Methods in Molecular Biology, Humana Press; Cell Biology: Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); and The Antibodies (M. Zanetti and J. D. Capra, eds., Harwood Academic Publishers, 1995).

### Purity by SE (Size Exclusion) HPLC

SE HPLC is used to monitor the size heterogeneity under native conditions by employing an SE HPLC column to separate antibody aggregates, monomer and fragments. The eluate is monitored by UV absorbance. Purity is determined as percentage (area) main peak, Sum of HMWS forms and Sum of LMWS forms relative to the total of all protein peaks detected.

### Purity by IE (Ion Exchange) HPLC

Gradient ion exchange chromatography is used to quantitatively monitor charge heterogeneity of samples treated with carboxypeptidase B by employing a cation exchange column to separate the sample into main peak, acidic region and basic region. Detection is performed by UV absorbance. Purity is determined as percentage (area) main peak, acidic region, and basic region relative to the total of all protein peaks detected.

### Purity by CE-SDS (Caliper)

Conventional SDS-PAGE methods for electrophoretic separation of proteins have been transferred to a chip format in the Caliper GXII/LabChip GX assay systems (Caliper LifeScience, Inc./Perkin Elmer). Proteins are separated by their respective size. Samples are prepared before separation by labeling with fluorescent dyes which can be detected and analyzed according to the manufacturer's instructions.

Purity and antibody integrity were analyzed after each purification step by CE-SDS using microfluidic Labchip technology (Caliper Life Science, USA). Therefore analyte solution was prepared and analyzed on LabChip GXII system using a HT Protein Express Chip. Data were analyzed using LabChip GX Software.

### Content of protein by UV

The protein concentration of the sample is determined by UV. The protein absorption is corrected by subtracting the absorption at 320 nm from the absorption at 280 nm. This absorbance value is directly proportional to the protein concentration. The protein concentration is calculated using the extinction coefficient of 1.5 mL mg-1 cm-1.

### Detection methods for host cell protein (HCP) and DNA content

### a) CHO HCP assay

The residual CHO HCP content in process samples is determined by an electrochemiluminescence immunoassay (ECLIA) on cobas e 411 immunoassay analyzer (Roche Diagnostics GmbH, Mannheim, Germany).

The assay is based on a sandwich principle using polyclonal anti-CHO HCP antibody from sheep.

First incubation: Chinese hamster ovary host cell protein (CHO HCP) from 15 $\mu$L sample (neat and/or diluted) and a biotin conjugated polyclonal CHO HCP specific antibody form a sandwich complex, which becomes bound to streptavidin-coated microparticles via interaction of biotin with streptavidin.

Second incubation: After addition of polyclonal CHO HCP-specific antibody labeled with ruthenium complex (Tris(2,2'-bipyridyl)ruthenium(II)-complex) a ternary sandwich complex is formed on the microparticles.

The reaction mixture is aspirated into the measuring cell where the microparticles are magnetically captured onto the surface of the electrode. Unbound substances are then removed in a washing step. Application of a voltage to the electrode then induces chemiluminescent emission which is measured by a photomultiplier.

The concentration of CHO HCP in the test sample is finally calculated from a CHO HCP standard curve of known concentration.

### b) DNA content

A q PCR based assay is used for the detection and quantification of CHO DNA. DNA from samples is extracted with a commercial RNA extraction kit using a silica gel based membrane. The extracted DNA is subject to quantitative real time PCR using PCR primers and probe with a sequence detection system. The amplicons (amplified product) are quantified in direct proportion to the increase in fluorescence emission measured continuously during the DNA amplification. A standard curve is used to quantify the amount of CHO cell DNA in the sample.

### Example 6: Purification of a bispecific antibody that bindsAng2 and VEGF-A (anti-Ang2/anti-VEGF-A antibody as described in WO 2011/117329)

Harvested cell culture fluid (HCCF) from a CHO expression culture was processed by MabSelect SuRe affinity chromatography in bind-elute mode. After loading of the HCCF onto the column to a maximum load density of 38 $g_{mAb}/l_{resin}$, the column was washed with 25 mM Tris, 25 mM NaCl, pH 7.2 for 5 column volumes. Then, an additional wash with 0.7 M Tris/HCl, pH 7.2 for five column volumes was performed. The third wash step was conducted using highly purified water or 10 mM Tris/HCl pH 7.5. The column-bound antibody was eluted using 50 mM Acetate, pH 3.4. The elution pool was collected based on $OD_{280}$ from 500 to 250 mAU (path length 1cm), over a maximum of three column volumes.

The affinity elution pool was adjusted to pH 3.5 with acetic acid and held for 30 min. The pool was then conditioned with 1.5 M Tris Base to pH 5.0 and cleared by depth filtration. The depth filtration pool was conditioned to pH 7 using 1.5 M Tris Base and served as feedstock for the second chromatography step using the multimodal anion exchange resin Capto adhere ImpRes.

The Capto adhere ImpRes column was equilibrated with 50 mM Tris Acetate, pH 7.0. The equilibrated column was loaded up to a load density of 180 $g_{mAb}/l_{resin}$ and washed with 20 mM Tris Acetate, pH 7.0. The elution pool was collected based on $OD_{280}$ from 1000 to 4000 mAU (path length 1cm).

The pool of the second chromatography step was conditioned to pH 5.0 with acetic acid and served as feedstock for the final third chromatography step using the multimodal cation exchange resin Capto MMC ImpRes. The third chromatography step was run in bind-and-elute mode. The Capto MMC ImpRes column was equilibrated with 30mM Tris/ Acetate pH 5.0 (equilibration buffer). The equilibrated column was loaded up to a load density of 45 $g_{mAb}/l_{resin}$ and washed with equilibration buffer for five column volumes. The second wash was performed using 30mM Tris/ Acetate pH 6.8 for ten column volumes followed by equilibration buffer for five column volumes. The final wash step was performed using 30mM Tris/ Acetate pH 4.9, 500 mM sodium sulfate for ten column volumes. The column-bound antibody was eluted using 30mM Tris/ Acetate pH 6.0, 500mM sodium sulfate. The elution pool was collected based on $OD_{280}$ from 3600 to 1000 mAU (path length 1cm).

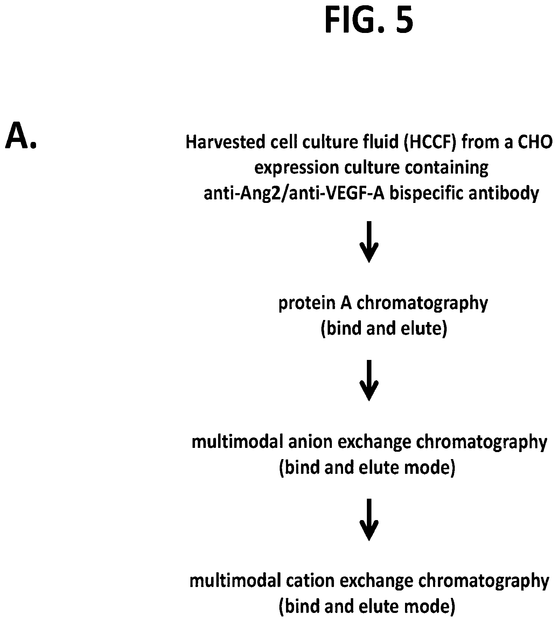

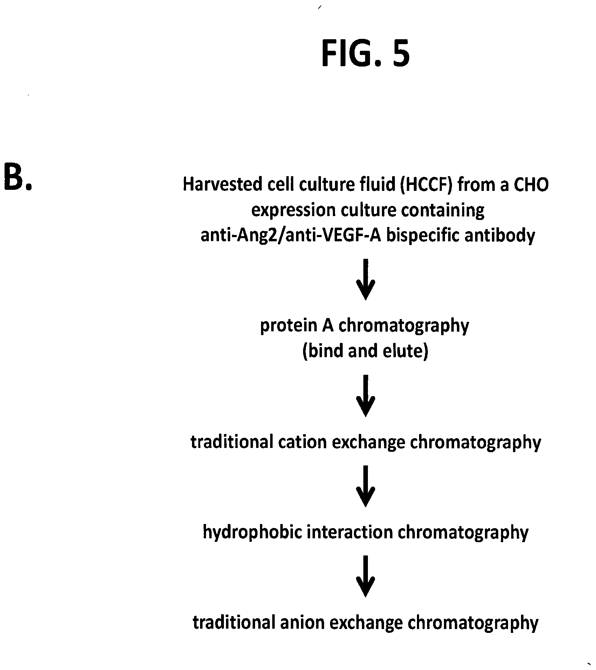

The pool of the third chromatography step was concentrated and buffer-exchanged into formulation buffer. The purification scheme described above is depicted in **FIG. 5A.**

*Table 16: Analytical data of product- and process-specific impurities after the respective chromatography steps*

| | Caliper not reduced | | |
|---|---|---|---|
| **Ang2/VEGF** | **¾ antibody [%]** | **Prepeaks [%]** | **Mainpeak [%]** |
| **MabSelect SuRe Elution Pool** | 1.8 | 8.4 | 91.6 |
| **Capto adhere ImpRes Elution Pool** | 1.1 | 5.9 | 94.1 |
| **Capto MMC ImpRes (C3) Elution Pool** | 0.4 | 2 | 98 |
| | **SE-HPLC** | | |
| **Ang2/VEGF** | **HMW [%]** | **Main Peak [%]** | **LMW [%]** |
| **MabSelect SuRe Elution Pool** | 5.5 | 94.2 | 0.32 |
| **Capto adhere ImpRes Elution Pool** | 0.9 | 98.9 | 0.25 |
| **Capto MMC ImpRes (C3) Elution Pool** | 0.7 | 99.2 | 0.02 |
| | **IE-HPLC** | | |
| **Ang2/VEGF** | **acidic [%]** | **Main Peak [%]** | **basic [%]** |
| **MabSelect SuRe Elution Pool** | 29.8 | 29.8 | 29.8 |
| **Capto adhere ImpRes Elution Pool** | 24.4 | 24.4 | 24.4 |
| **Capto MMC ImpRes (C3) Elution Pool** | 26.4 | 26.4 | 26.4 |
| | **HCP** | **DNA** | |
| **Ang2/VEGF** | **HCP [ng/mg]** | **DNA [pg/mg]** | |
| **MabSelect SuRe Elution Pool** | 159 | 17 | |

(continued)

| Ang2/VEGF | HCP | DNA |
|---|---|---|
|  | HCP [ng/mg] | DNA [pg/mg] |
| Capto adhere ImpRes Elution Pool | 11 | 1 |
| Capto MMC ImpRes (C3) Elution Pool | 2 | n.d. |

*Table 17: Comparison with process using 4 chromatography columns comprising affinity chromatography, cation exchange chromatography, hydrophobic interaction chromatography and anion exchange chromatography (4 column (4C) process)*

|  | Ang2/VEGF 3C Process (*see* FIG.5A) | Ang2/VEGF 4C Process (*see* FIG. 5B) |
|---|---|---|
| **Overall Yield [%]** | ~ 50 | ~ 48 |
| **SE-HPLC area [%]** |  |  |
| Main Peak | 99.3 | 98.7 |
| Sum of HMW Forms | 0.7 | 1.1 |
| **HCP [ng/mg]** | 2 | 3 |
| **DNA [pg/mg]** | <0.1 | <0.1 |
| **Mainpeak [%] (Caliper)** | 98 | 96 |
| **IE-HPLC area [%]** |  |  |
| Main Peak | 57 | 57 |
| Acidic Peak | 26 | 30 |
| Basic Peak | 17 | 12 |

It can be seen that by using a three column process comprising MabSelectSure, Capto adhere and Capto MMC ImpRes HHL, product-related impurities such as 3/4 antibodies, prepeaks, HMWS, LMWS, and process-related impurities such as HCP and DNA can be reduced as compared to the using a four column process comprising capture chromatography, traditional cation exchange chromatography, hydrophobic interaction chromatography, and traditional anion exchange chromatography.

*Example 7: Purification of a bispecific antibody against VEGF-A and Ang2 (anti-VEGF-A/anti-Ang2 antibody as described in WO 2014/009465)*

Harvested cell culture fluid (HCCF) from a CHO expression culture was processed by Capture Select FcXL affinity chromatography in bind-elute mode. After loading of the HCCF onto the column to a maximum load density of 25 $g_{mAb}/l_{resin}$, the column was washed with 25 mM Tris/HCl, 25 mM NaCl, pH 7.2 for 2 column volumes. Then, an additional wash with purified water PWII for five column volumes was performed. The column-bound antibody was eluted using 30 mM acetic acid, pH 3.2. The elution pool was collected based on $OD_{280}$ from 2500 to 1000 mAU (path length 1cm).

The affinity elution pool was adjusted to pH 3.4 with acetic acid and held for 60min. The pool was then conditioned with 1.5 M Tris Base to pH 5.0 and cleared by depth filtration. The depth filtration pool was conditioned to pH 7 using 1.5 M Tris Base and served as feedstock for the second chromatography step using the multimodal anion exchange resin Capto adhere. As the conductivity of the load was <5 mS/cm, no adjustment of conductivity was necessary.

The Capto adhere column was equilibrated with 50 mM Tris/Acetate, pH 7.0. The equilibrated column was loaded up to a load density of 170 $g_{mAb}/l_{resin}$ and washed with 50 mM Tris/Acetate, pH 7.0 (=equilibration buffer). The elution pool was collected based on $OD_{280}$ from 1000 to 2500 mAU (path length 1cm) over a maximum of 3 CV wash.

The pool of the second chromatography step was conditioned to pH 5.0 with acetic acid and served as feedstock for the final third chromatography step using the multimodal cation exchange resin Capto MMC ImpRes. The third chromatography step was run in bind-and-elute mode. The CaptoMMC ImpRes column was equilibrated with 30mM Tris/Acetate, 30 mM Tris/Citrate pH 5.0. The equilibrated column was loaded up to a load density of 30 $g_{mAb}/l_{resin}$ and washed with equilibration buffer for five column volumes. The second wash was performed using 30mM Tris/Acetate, 30 mM Tris/Citrate, 150mM NaCl pH 5.0 for ten column volumes followed by equilibration buffer for five column volumes. The final wash step was performed using 30mM Tris/Acetate, 30 mM Tris/Citrate, 500mM NaCl pH 4.5 for ten column volumes. The column-bound antibody was eluted using a pH/salt gradient from 0-50% B in 40 column volumes. Buffer A was the equilibration buffer 30mM Tris/Acetate, 30 mM Tris/Citrate pH 5.0 and buffer B was 30mM Tris/Acetate, 30 mM Tris/Citrate, 1.5M NaCl, pH 8.5. The elution pool was collected based on $OD_{280}$ from 250 to 4500 mAU (path length 1cm).

**Table 18: Analytical data of product- and process-specific impurities after the respective chromatography steps**

| | Caliper not reduced | | |
|---|---|---|---|
| **VEGF/Ang2** | **¼ antibody [%]** | **Prepeaks [%]** | **Mainpeak [%]** |
| Capture Select FcXL Elution Pool | 2.57 | 8.43 | 91.16 |
| Capto adhere Elution Pool | 1.64 | 5.84 | 94.16 |
| Capto MMC ImpRes Elution Pool | 1.23 | 2.37 | 97.63 |
| | SE-HPLC | | |
| **VEGF/Ang2** | **HMW [%]** | **Mainpeak [%]** | **LMW [%]** |
| Capture Select FcXL Elution Pool | 10.1 | 89.5 | 0.5 |
| Capto adhere Elution Pool | 2.1 | 97.3 | 0.55 |
| Capto MMC ImpRes | 0.7 | 99.3 | 0.04 |

| Elution Pool | | | |
|---|---|---|---|
| | IE-HPLC | | |
| **VEGF/Ang2** | **acidic [%]** | **Mainpeak [%]** | **basic[%]** |
| Capture Select FcXL Elution Pool | 29.7 | 57.2 | 13.1 |
| Capto adhere Elution Pool | 26.2 | 62.7 | 11.1 |
| Capto MMC ImpRes Elution Pool | 25.8 | 67.3 | 7 |
| | HCP | | DNA |
| **VEGF/Ang2** | **HCP [ng/mg]** | | **DNA [pg/mg]** |
| Capture Select FcXL Elution Pool | 8615 | | 210 |
| Capto adhere Elution Pool | 484 | | 1.4 |
| Capto MMC ImpRes Elution Pool | 7 | | 1.7 |

*Table 19: Comparison with process using 4 chromatography columns comprising affinity chromatography, cation exchange chromatography, hydrophobic interaction chromatography and anion exchange chromatography (4 column (4C) process)*

| | VEGF/Ang2 3C Process | VEGF/Ang2 4C Process |
|---|---|---|
| **Overall Yield [%]** | ~ 40 | ~ 25-35 |
| **SE-HPLC area [%]** | | |
| Main Peak | 99.3 | 98.8 |
| Sum of HMW Forms | 0.7 | |
| **HCP [ng/mg]** | 7 | 1 |
| **DNA [pg/mg]** | 1.7 | <0.1 |
| **Mainpeak [%] (Caliper / CE SDS)** | 97.6 | 94.6 |
| **IE-HPLC area [%]** | | |
| Main Peak | 67.3 | 72.2 |
| Acidic Peak | 25.8 | 23.1 |
| Basic Peak | 7.0 | 4.7 |

It can be seen that by using Capto Adhere and Capto MMC ImpRes that HHL, product-related impurities such as 3/4 antibodies, prepeaks, HMWS, LMWS, and process-related impurities such as HCP and DNA can be reduced.

### Example 8: Assembly and Purification of an anti-X1/anti-Y1 Bispecific Antibody

A bispecific antibody against target proteins X1 and Y1 - anti-X1/anti-Y1 bispecific antibody or aX1/Y1 bispecific - is assembled as follows. Each half-antibody (aX1 (knob) and aY1 (hole)) is independently subject to an affinity chromatography step using protein A resin (MabSelect SuRe, GE Healthcare), as described in Example 1.

The half-antibody pools obtained from the protein A chromatography step are then combined in a 1:1 molar ratio and assembled as described in Example 1. The pH adjusted assembled pool is then subjected to multimodal anion exchange chromatography using Capto™ Adhere resin in a flow-through mode. The Capto™ Adhere column is equilibrated as described in Example 1. The product pool from the bispecific assembly step is adjusted to a conductivity of 9.0 mS/cm with purified water and loaded onto the column. The bispecific antibody is flowed through the column, which is then washed with equilibration buffer. Anion exchange pooling is initiated and terminated based on absorbance at 280 nm.

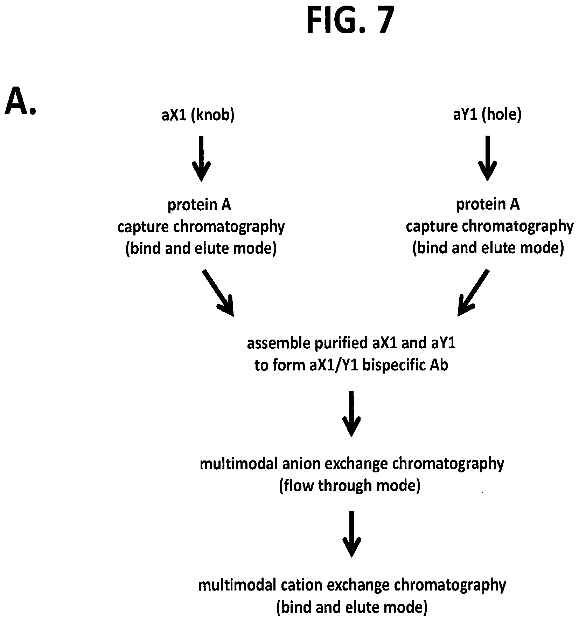

The multimodal anion chromatography product pool is then subjected to a multimodal cation exchange chromatography using Capto™ MMC resin in a bind and elute mode. The column is equilibrated as described in Example 1. The multimodal anion chromatography product pool is loaded and washed as described in Example 1. The bispecific antibody is eluted off the column by increasing both salt and pH in a step elution, as described in Example 1. Cation exchange pooling is initiated and terminated based on absorbance at 280 nm. The purification scheme described above is depicted in **FIG. 7A.**

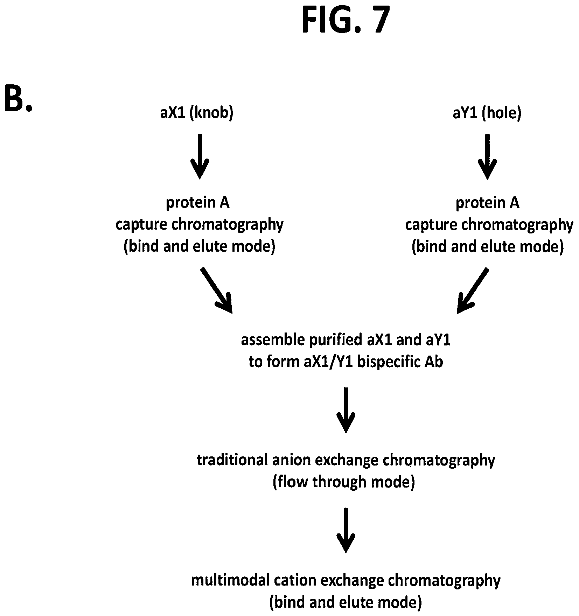

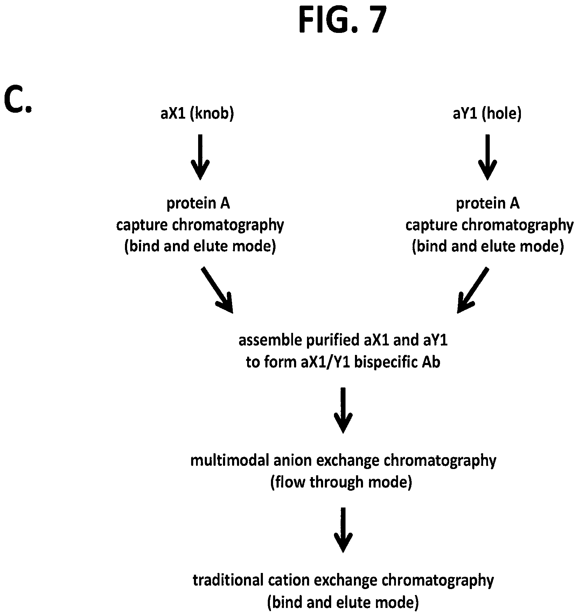

The degree of separation of the bispecific antibody from product-related and process-related impurities achieved using the purification scheme shown in **FIG. 7A** is compared to that achieved using the purification schemes shown in **FIGs. 7B** and **7C.**

The experiment described above is repeated, and the pooled material from the affinity chromatography step is spiked with 20% hole homodimers. The degree of separation of the bispecific antibody from product-related and process-related impurities achieved using the purification scheme shown in **FIG. 7A** is once again compared to that achieved using the purification schemes shown in **FIGs. 7B** and **7C.**

LIST OF EMBODIMENTS

1. A method for purifying a multispecific antibody from a composition comprising the multispecific antibody and an impurity, wherein the multispecific antibody comprises multiple arms, each arm comprising a VH/VL unit, the method comprising the sequential steps of:

a) subjecting the composition to a capture chromatography to produce a capture chromatography eluate;
b) subjecting the capture chromatography eluate to a first mixed mode chromatography to generate a first mixed mode eluate; and
c) subjecting the first mixed mode eluate to a second mixed mode chromatography to generate a second mixed mode eluate; and
d) collecting a fraction comprising the multispecific antibody,
wherein the method reduces the amount of a product-specific impurity from the composition.

2. The method of embodiment 1, wherein the capture chromatography eluate is subjected to ion exchange or HIC chromatography prior to the first mixed mode chromatography.

3. A method for purifying a multispecific antibody from a composition comprising the multispecific antibody and an impurity, wherein the multispecific antibody comprises multiple arms, each arm comprising a VH/VL unit, wherein each arm of the multispecific antibody is produced separately, the method comprising the sequential steps of

a) subjecting each arm of the multispecific antibody to capture chromatography to produce capture eluates for each arm of the multispecific antibody,
b) forming a mixture comprising capture eluates of each arm of the multispecific antibody under conditions sufficient to produce a composition comprising the multispecific antibody,
c) subjecting the composition comprising the multispecific antibody to a first mixed mode chromatography to generate a first mixed mode eluate, and
d) subjecting the first mixed mode eluate to a second mixed mode chromatography to generate a second mixed mode eluate; and
e) collecting a fraction comprising the multispecific antibody,
wherein the method reduces the amount of a product-specific impurity from the composition.

4. The method of embodiment 3, wherein the composition comprising the multispecific antibody is subjected to ion exchange or HIC chromatography prior to the first mixed mode chromatography.

5. The method of any one of embodiments 1-4, wherein the capture chromatography is affinity chromatography.

6. The method of embodiment 5, wherein the affinity chromatography is protein L chromatography, protein A chromatography, protein G chromatography, protein A and protein G chromatography.

7. The method of embodiment 5 or embodiment 6, wherein the affinity chromatography is protein A chromatography.

8. The method of any one of embodiments 1-7, wherein the capture chromatography is carried out in bind and elute mode.

9. The method of any one of embodiments 1-8, wherein the first mixed mode chromatography and the second mixed mode chromatography are contiguous.

10. The method any one of embodiments 1-9, wherein the first mixed mode chromatography is a mixed mode anion exchange chromatography.

11. The method of any one of embodiments 1-10, wherein the second mixed mode chromatography is a mixed mode cation exchange chromatography.

12. The method any one of embodiments 1-9, wherein the first mixed mode chromatography is a mixed mode cation exchange chromatography.

13. The method of any one of embodiments 1-10 and 12, wherein the second mixed mode chromatography is a mixed mode anion exchange chromatography.

14. The method of any one of embodiments 1-13, wherein the first mixed mode chromatography is carried out in bind and elute mode.

15. The method of embodiment 14, wherein the elution is a gradient elution.

16. The method of any one of embodiments 1-13, wherein the first mixed mode chromatography is carried out in flow through mode.

17. The method of any one of embodiments 1-16, wherein the second mixed mode chromatography is carried out in bind and elute mode.

18. The method of embodiment 17, wherein the elution is a gradient elution.

19. The method of any one of embodiments 1-16, wherein the second mixed mode chromatography is carried out in flow through mode.

20. The method of any one of embodiments 1-19 further comprising the step of subjecting the second mixed mode eluate to ultrafiltration.

21. The method of embodiment 20 wherein the ultrafiltration comprises sequentially a first ultrafiltration, a diafiltration and a second ultrafiltration.

22. The method of any one of embodiments 7-21, wherein the protein A chromatography comprises protein A linked to agarose.

23. The method of any one of embodiments 7-21, wherein the protein A chromatography is a MAbSelect™, MAbSelect™ SuRe and MAbSelect™ SuRe LX, Prosep-VA, Prosep-VA Ultra Plus, Protein A sepharose fast flow, or Toyopearl Protein A chromatography.

24. The method of any one of embodiments 7-23, wherein the protein A chromatography uses one or more of a protein A equilibration buffer, a protein A loading buffer or a protein A wash buffer wherein the equilibration buffer, a loading buffer, and/or wash buffer is between about pH 7 and about pH 8.

25. The method of embodiment 24, wherein the protein A equilibration buffer is about pH 7.7.

26. The method of embodiment 24 or embodiment 25, wherein the protein A equilibration buffer comprises about 25 mM Tris and about 25 mM NaCl.

27. The method of any one of embodiments 24-26, wherein the protein A chromatography is washed with equilibration buffer following load.

28. The method of any one of embodiments 7-27, wherein the multispecific antibody is eluted from the protein A chromatography by a pH step elution.

29. The method of any one of embodiments 7-28, wherein the multispecific antibody is eluted from the protein A by applying a protein A elution buffer with low pH to the protein A chromatography.

30. The method of embodiment 29, wherein the protein A elution buffer comprises about 150 mM acetic acid, about pH 2.9.

31. The method of any one of embodiments 7-30 wherein the protein A eluate is pooled where the $OD_{280}$ of the eluate is greater than about 0.5.

32. The method of any one of embodiments 10-31, wherein the anion exchange mixed mode chromatography comprises a quaternary amine and a hydrophobic moiety.

33. The method of embodiment 32, wherein the anion exchange mixed mode chromatography comprises a quaternary amine and a hydrophobic moiety linked to highly crosslinked agarose.

34. The method of embodiment 33, wherein the mixed mode chromatography is a Capto™ Adhere chromatography or a Capto™ Adhere ImpRes chromatography.

35. The method of any one of embodiments 11-34, wherein the cation exchange mixed mode chromatography comprises a N-benzyl-n-methyl ethanolamine.

36. The method of embodiment 35, wherein the mixed mode chromatography is a Capto™ MMC chromatography or a Capto™ MMC ImpRes chromatography.

37. The method of any one of embodiments 1-36 wherein the first mixed mode chromatography uses one or more of a mixed mode pre-equilibration buffer, a mixed mode equilibration buffer, a mixed mode loading buffer, or a mixed mode wash buffer, and wherein the mixed mode pre-equilibration buffer, the mixed mode equilibration buffer, the mixed mode loading buffer and/or the mixed mode wash buffer is between about pH 6 and about pH 7.

38. The method of any one of embodiments 1-37 wherein the second mixed mode chromatography uses one or more of a mixed mode pre-equilibration buffer, a mixed mode equilibration buffer, a mixed mode loading buffer or a mixed mode wash buffer wherein the mixed mode pre-equilibration buffer, the mixed mode equilibration buffer, and/or mixed mode wash buffer is between about pH 5 and about pH 8.

39. The method of embodiment 37 or embodiment 38, wherein the mixed mode pre-equilibration buffer, the mixed mode equilibration buffer, and/or mixed mode wash buffer is about pH 6.5.

40. The method of any one of embodiments 37-39, wherein the mixed mode pre-equilibration buffer comprises about 500 mM acetate.

41. The method of any one of embodiments 37-40, wherein the mixed mode equilibration buffer comprises about 50 mM acetate.

42. The method of any one of embodiments 37-41, wherein the first mixed mode chromatography is washed with wash buffer following load.

43. The method of any one of embodiments 37-42, wherein the second mixed mode chromatography is washed with wash buffer following load.

44. The method of any one of embodiments 15 and 18-43 wherein the multispecific antibody is eluted from the first mixed mode chromatography by pH gradient.

45. The method of any one of embodiments 15 and 18-44, wherein the multispecific antibody is eluted from the first mixed mode chromatography by applying a mixed mode elution buffer with low pH to the mixed mode ion exchange chromatography.

46. The method of any one of embodiments 15 and 18-45 wherein the multispecific antibody is eluted from the second mixed mode chromatography by pH gradient.

47. The method of any one of embodiments 15 and 18-46, wherein the multispecific antibody is eluted from the second mixed mode chromatography by applying a mixed mode elution buffer with low pH to the mixed mode exchange chromatography.

48. The method of any one of embodiments 2 and 4-47, wherein the ion exchange chromatography comprises a quaternary amine.

49. The method of embodiment 48, wherein the ion exchange chromatography is an anion exchange chromatography, and wherein the anion exchange chromatography comprises a quaternary amine linked to crosslinked agarose.

50. The method of embodiment 49, wherein the mixed mode anion exchange chromatography is a CaptoAdhere chromatography.

51. The method of any one of embodiments 48-50, wherein the anion exchange chromatography uses one or more of an anion exchange pre-equilibration buffer, an anion exchange equilibration buffer or an anion exchange loading buffer wherein the anion exchange pre-equilibration buffer, the anion exchange equilibration buffer and/or anion exchange the load buffer is between about pH 6 and about pH 8.

52. The method of embodiment 51, wherein the anion exchange pre-equilibration buffer, the anion exchange equilibration buffer and/or the anion exchange load is about pH 6.5.

53. The method of embodiment 51 or embodiment 52, wherein the anion exchange pre-equilibration buffer comprises about 50 mM Tris, 500 mM sodium acetate.

54. The method of any one of embodiments 51-53, wherein the anion exchange equilibration buffer comprises about 50 mM Tris.

55. The method of any one of embodiments 51-54, wherein the anion exchange chromatography is washed with anion exchange equilibration buffer following load.

56. The method of any one of embodiments 51-55, wherein the multispecific antibody is eluted from the anion exchange chromatography by salt gradient.

57. The method of any one of embodiments 51-56, wherein the multispecific antibody is eluted from the anion exchange chromatography by applying an anion exchange elution buffer with increased salt concentration to the anion exchange chromatography.

58. The method of embodiment 57, wherein the anion exchange elution buffer comprises about 50 mM Tris, 100 mM sodium acetate at about pH 8.5.

59. The method of any one of embodiments 51-58 wherein the anion exchange eluate is pooled where the $OD_{280}$ of the eluate is greater than about 0.5 to about 2.0.

60. The method of any one of embodiments 1-59, wherein the arms of the multispecific antibody are produced in a cell.

61. The method of embodiment 60, wherein the cell is a prokaryotic cell.

62. The method of embodiment 61, wherein the prokaryotic cell is an *E. coli* cell.

63. The method of embodiment 61 or embodiment 62, wherein the cell is engineered to express one or more chaperones.

64. The method of embodiment 63, wherein the chaperone is one or more of FkpA, DsbA or DsbC.

65. The method of embodiment 63 or embodiment 64, wherein the chaperone is an *E. coli* chaperone.

66. The method of any one of embodiments 1-60, wherein the cell is a eukaryotic cell.

67. The method of embodiment 66, wherein the eukaryotic cell is a yeast cell, an insect cell, or a mammalian cell.

68. The method of embodiment 66 or embodiment 67, wherein the eukaryotic cell is a CHO cell.

69. The method of any one of embodiments 60-68, wherein the cells are lysed to generate a cell lysate comprising the multispecific antibody or an arm of the multispecific antibody prior to capture chromatography.

70. The method of embodiment 69, wherein the cells are lysed using a microfluidizer.

71. The method of embodiment 69 or embodiment 70, wherein polyethlyeneimine (PEI) is added to the cell lysate prior to chromatography.

72. The method of any one of embodiments 1-71 wherein the method reduces the amount of any one of host cell protein (HCP), leached protein A, nucleic acid, cell culture media components, or viral impurities in the composition.

73. The method of any one of embodiments 1-72, wherein the multispecific antibody is a bispecific antibody.

74. The method of embodiment 73, wherein the bispecific antibody is a knob-in-hole (KiH) bispecific antibody.

75. The method of embodiment 73 or embodiment 74, wherein the bispecific antibody is a CrossMab bispecific antibody.

76. The method of any one of embodiments 1-75, wherein the fraction contains at least about 95% multispecific antibody.

77. The method of any one of embodiments 1-76, wherein the product-specific impurity is one or more of non-paired antibody arms, antibody homodimers, aggregates, high molecular weight species (HMWS), low molecular weight species (LMWS), acidic variants, or basic variants.

78. The method of embodiment 77, wherein the fraction contains no more than about 5% non-paired antibody arms.

79. The method of embodiment 77 or embodiment 78, wherein the fraction contains no more than about 5% antibody homodimers.

80. The method of any one of embodiments 77-79, wherein the fraction contains no more than about 2% aggregates or high molecular weight species (HMWS).

81. The method of any one of embodiments 77-80, wherein the fraction contains no more than about 2% LMWS.

82. The method of any one of embodiments 77-81 wherein the fraction contains no more than about 50% acidic variants.

83. The method of any one of embodiments 77-82, wherein the fraction contains no more than about 35% basic variants.

84. The method of any one of embodiments 77-83, wherein the fraction contains no more than about 5% of ¾ antibodies.

85. The method of embodiment 77, wherein the fraction contains

a) at least about 95% -100% multispecific antibody;
b) no more than about 1%-5% non-paired antibody arms;
c) no more than about 1%-5% antibody homodimers;
d) no more than about 1% or 2% HMWS;
e) no more than about 1% or 2% LMWS; and
f) no more than about 5% of ¾ antibodies.

86. A composition comprising a multispecific antibody purified by the method of any one of embodiments 1-85.

87. A composition comprising a multispecific antibody purified by the method of any one of embodiments 1-85 for the treatment of cancer or eye disease.

88. A method for purifying an Fc-region containing heterodimeric polypeptide with a multi-step chromatography method wherein the method comprises an affinity chromatography step followed by two different multimodal ion exchange chromatography steps,
and thereby purifying the Fc-region containing heterodimeric polypeptide.

89. The method according to embodiment 88, wherein the multi-step chromatography method comprises

i. an affinity chromatography step, followed by a multimodal anion exchange chromatography step, followed by a multimodal cation exchange chromatography step
or
ii. an affinity chromatography step, followed by a multimodal cation exchange chromatography step, followed by a multimodal anion exchange chromatography step.

90. The method according to any one of embodiments 88-89, wherein the multi-step chromatography method comprises an affinity chromatography step, followed by a multimodal anion exchange chromatography step, followed by a multimodal cation exchange chromatography step.

91. The method according any one of embodiments 88-90, wherein the multi-step chromatography method comprises exactly three chromatography steps.

92. The method according to any one of embodiments 89-91, wherein the multimodal anion exchange chromatography step is performed in flow-through mode.

93. The method according to any one of embodiments 89-92, wherein in the multimodal anion exchange chromatography step the Fc-region containing heterodimeric polypeptide is applied in a solution with a conductivity value of less than 7 mS/cm.

94. The method according to any one of embodiments 89- 93, wherein in the multimodal anion exchange chromatography step the Fc-region containing heterodimeric polypeptide is applied in a solution with a conductivity value in the range of about 6 mS/cm to about 2 mS/cm.

95. The method according to any one of embodiments 89-94, wherein in the multimodal anion exchange chromatography step the Fc-region containing heterodimeric polypeptide is applied in a solution with a conductivity value of about 4.5 mS/cm.

96. The method according to any one of embodiments 89-95, wherein the multimodal anion exchange chromatography step is performed at a pH of about 7.

97. The method according to any one of embodiments 89-96, wherein in the multimodal anion exchange chromatography step the Fc-region containing heterodimeric polypeptide is applied in a solution with a conductivity of about 4.5 mS/cm and a pH of about 7.

98. The method according to any one of embodiments 89-97, wherein in the multimodal anion exchange chromatography step the Fc-region containing heterodimeric polypeptide is applied in the range of from about 100 g to about 300 g per liter of chromatography material.

99. The method according to any one of embodiments 89-98, wherein the multimodal anion exchange chromatography material is a multimodal strong anion exchange chromatography material.

100. The method according to any one of embodiments 89-99, wherein the multimodal anion exchange chromatography material has a matrix of high-flow agarose, a multimodal strong anion exchanger as ligand, an average particle size of 36-44 $\mu$m and an ionic capacity of 0.08 to 0.11 mmol Cl-/mL medium.

101. The method according to any one of embodiments 89-100, wherein the multimodal cation exchange chromatography medium is a multimodal weak cation exchange chromatography medium.

102. The method according to any one of embodiments 89-101, wherein the multimodal cation exchange chromatography medium has a matrix of high-flow agarose, a multimodal weak cation exchanger as ligand, an average particle size of 36-44 $\mu$m and ionic capacity of 25 to 39 $\mu$mol/mL.

103. The method according to any one of embodiments 89-102, wherein the multimodal anion exchange chromatography step is performed in bind and elute mode.

104. The method according to any one of embodiments 88-103, wherein the affinity chromatography is a protein A affinity chromatography or a Protein G affinity chromatography or a single chain Fv ligand affinity chromatography or a chromatography step with CaptureSelect chromatography material or a chromatography step with CaptureSelect FcXL chromatography material.

105. The method according to any one of embodiments 88-104, wherein the affinity chromatography step is a protein A chromatography step.

106. The method according to any one of embodiments 88-105, wherein the affinity chromatography step is a chromatography step with CaptureSelect™ chromatography material.

107. The method according to any one of embodiments 88-106, wherein the Fc-region containing heterodimeric polypeptide is an antibody, a bispecific antibody or Fc-fusion proteins.

108. The method according to any one of embodiments 88-107, wherein the Fc-region containing heterodimeric polypeptide is a bispecific antibody.

109. The method according to any one of embodiments 90-108, wherein the Fc-region containing heterodimeric polypeptide is a CrossMab.

110. The method according to any one of embodiments 90-109, wherein the Fc-region containing heterodimeric polypeptide is a bispecific antibody comprising

a) a heavy chain and a light chain of a first full length antibody that specifically binds to a first antigen; and

b) a modified heavy chain and a modified light chain of a full length antibody that specifically binds to a second antigen, wherein the constant domains CL and CH1 are replaced by each other.

111. The method according to any one of embodiments 108-110, wherein the bispecific antibody binds to ANG2 and VEGF.

112. The method according to any one of embodiments 108-110, wherein the CrossMab binds to ANG2 and VEGF.

113. The method according to any one of embodiments 108-112, wherein the bispecific antibody is vanucizumab.

114. The method according to any one of embodiments 108-112 wherein the bispecific antibody comprises a first antigen-binding site that comprises as heavy chain variable domain (VH) the SEQ ID NO: 1, and as light chain variable domain (VL) the SEQ ID NO: 2; and a second antigen-binding site that comprises as heavy chain variable domain (VH) the SEQ ID NO: 3, and as light chain variable domain (VL) the SEQ ID NO: 4.

115. The method according to any one of embodiments 108-112, wherein the bispecific antibody comprises a first heavy chain with the amino acid sequence of SEQ ID NO: 9 and a second heavy chain with the amino acid sequence of SEQ ID NO: 10 and a first light chain with the amino acid sequence of SEQ ID NO: 11 and a second light chain with the amino acid sequence of SEQ ID NO: 12.

116. The method according to any one of embodiments 108-112, wherein the bispecific antibody comprises a first antigen-binding site that comprises as heavy chain variable domain (VH) the SEQ ID NO: 5, and as light chain variable domain (VL) the SEQ ID NO: 6; and a second antigen-binding site that comprises as heavy chain variable domain (VH) the SEQ ID NO: 7, and as light chain variable domain (VL) the SEQ ID NO: 8.

117. The method according to any one of embodiments 108-112, wherein the bispecific antibody comprises a first heavy chain with the amino acid sequence of SEQ ID NO: 13 and a second heavy chain with the amino acid sequence of SEQ ID NO: 14 and a first light chain with the amino acid sequence of SEQ ID NO: 15 and a second light chain with the amino acid sequence of SEQ ID NO: 16.

118. The method of any one of embodiments 108-117, wherein the purified Fc-region containing heterodimeric polypeptide contains no more than about 5% of ¾ antibodies.

119. A method for purifying a bispecific antibody that binds to ANG-2 and VEGF with a multi-step chromatography method wherein the method comprises an affinity chromatography step, followed by a multimodal anion exchange chromatography step, followed by a multimodal cation exchange chromatography step,
and thereby purifying the bispecific antibody that binds to ANG-2 and VEGF,

wherein bispecific antibody comprises a first antigen-binding site that comprises as heavy chain variable domain (VH) the SEQ ID NO: 1, and as light chain variable domain (VL) the SEQ ID NO: 2; and a second antigen-binding site that comprises as heavy chain variable domain (VH) the SEQ ID NO: 3, and as light chain variable domain (VL) the SEQ ID NO: 4
or that comprises a first antigen-binding site that comprises as heavy chain variable domain (VH) the SEQ ID NO: 5, and as light chain variable domain (VL) the SEQ ID NO: 6; and a second antigen-binding site that comprises as heavy chain variable domain (VH) the SEQ ID NO: 7, and as light chain variable domain (VL) the SEQ ID NO: 8.

120. The method according to embodiment 119, wherein the bispecific antibody binds to ANG-2 and VEGF comprises

a) the heavy chain and the light chain of a first full length antibody that comprises the first antigen-binding site; and
b) the modified heavy chain and modified light chain of a full length antibody that comprises the second antigen-binding site, wherein the constant domains CL and CH1 are replaced by each other.

121. A composition comprising a bispecific antibody, wherein the composition contains at least about 95%, bispecific antibody.

122. The composition of embodiment 121, wherein the composition contains no more than about 5% non-paired antibody arms.

123. The composition of embodiment 121 or 122, wherein the composition contains no more than about 5% antibody homodimers.

124. A composition comprising a CrossMab antibody, wherein the composition contains at least 95% CrossMab antibody.

125. The composition of any one of embodiments 121-124, wherein the composition contains no more than about 2% aggregates or high molecular weight species (HMWS).

126. The composition of any one of embodiments 121-125, wherein the composition contains no more than about 2% low molecular weight species (LMWS).

127. The composition of any one of embodiments 121-126 wherein the composition contains no more than about 50% acidic variants.

128. The composition of any one of embodiments 121-127, wherein the composition contains no more than about 35% basic variants.

129. The composition of any one of embodiments 121-128, wherein the composition contains no more than about 5% of ¾ antibodies.

130. The composition of embodiment 121 or embodiment 122, wherein the composition contains

a) at least about 95% -100% multispecific antibody;
b) no more than about 1%-5% non-paired antibody arms;
c) no more than about 1%-5% antibody homodimers;
d) no more than about 1% or 2% HMWS;
e) no more than about 1% or 2% LMWS; and
f) no more than about 5% of ¾ antibodies.

131. The composition of any one of embodiments 121-130, wherein the bispecific antibody binds to ANG-2 and VEGF.

132. The composition of embodiment 131, wherein the bispecific antibody binds to ANG-2 and VEGF comprises

a) the heavy chain and the light chain of a first full length antibody that comprises the first antigen-binding site; and
b) the modified heavy chain and modified light chain of a full length antibody that comprises the second antigen-binding site, wherein the constant domains CL and CH1 are replaced by each other.

133. A composition comprising a bispecific antibody that binds to ANG-2 and VEGF, wherein the composition contains no more than about 5% , about 4%, about 3%, about 2%, or about 1% of ¾ antibodies.

134. The composition of embodiment 133, wherein the bispecific antibody that binds to ANG-2 and VEGF comprises

a) the heavy chain and the light chain of a first full length antibody that comprises the first antigen-binding site; and

b) the modified heavy chain and modified light chain of a full length antibody that comprises the second antigen-binding site, wherein the constant domains CL and CH1 are replaced by each other.

135. The composition of embodiment 133 or 134, wherein the composition is obtained using the method of embodiment 119.

136. Use of the method according to any one of embodiments 88-118 for the purification of an Fc-containing heterodimeric polypeptide.

137. Use of the method according to any one of embodiments 88-118 for the reduction of Fc-containing heterodimeric polypeptide related impurities.

138. An Fc-containing heterodimeric polypeptide obtained with the method according to any one of embodiments 88-118 or a bispecific antibody that binds to ANG-2 and VEGF obtained using the method of any one of embodiments 119-120 for the manufacture of a medicament for the treatment of cancer or eye disease.

139. An Fc-containing heterodimeric polypeptide obtained from the method according to any one of embodiments 88-118 or a bispecific antibody that binds to ANG-2 and VEGF obtained using the method of any one of embodiments 119-120 for use in the treatment of cancer or eye disease.

140. A method for producing an Fc-containing heterodimeric polypeptide comprising the steps of

i. cultivating a cell comprising a nucleic acid encoding an Fc-containing heterodimeric polypeptide,
ii. recovering the Fc-containing heterodimeric protein from the cell or the cultivation medium,
iii. purifying the Fc-containing heterodimeric polypeptide with a method according to any one of embodiments 88 to 118,
and thereby producing the Fc-containing heterodimeric polypeptide.

141. A method for producing a bispecific antibody that binds to ANG-2 and VEGF obtained using the method of any one of embodiments 119-120 comprising the steps of

i. cultivating a cell comprising a nucleic acid encoding the bispecific antibody,
ii. recovering the bispecific antibody from the cell or the cultivation medium,
iii. purifying the bispecific antibody with a method according to any one of embodiments 119-120,

and thereby producing the bispecific antibody that binds to ANG-2 and VEGF.

**Claims**

1. A method for purifying a multispecific antibody from a composition comprising the multispecific antibody and an impurity, wherein the multispecific antibody comprises multiple arms, each arm comprising a VH/VL unit, wherein each arm of the multispecific antibody is produced separately, the method comprising the sequential steps of

a) subjecting each arm of the multispecific antibody to capture chromatography to produce capture eluates for each arm of the multispecific antibody,
b) forming a mixture comprising capture eluates of each arm of the multispecific antibody under conditions sufficient to produce a composition comprising the multispecific antibody,
c) subjecting the composition comprising the multispecific antibody to a first mixed mode chromatography to generate a first mixed mode eluate, and
d) subjecting the first mixed mode eluate to a second mixed mode chromatography to generate a second mixed mode eluate; and
e) collecting a fraction comprising the multispecific antibody,

wherein the method reduces the amount of a product-specific impurity from the composition, wherein the multispecific antibody is a bispecific antibody.

2. The method of claim 1, wherein the first mixed mode chromatography is a mixed mode anion exchange chromatography or a mixed mode cation exchange chromatography.

3. The method of any one of claims 1-2, wherein the second mixed mode chromatography is a mixed mode cation exchange chromatography or a mixed mode anion exchange chromatography.

4. The method of any one of claims 1-3, wherein the first mixed mode chromatography is carried out in bind and elute mode or in flow through mode.

5. The method of any one of claims 1-4, wherein the second mixed mode chromatography is carried out in bind and elute mode or in flow through mode.

6. The method of any one of claims 2-5, wherein the anion exchange mixed mode chromatography comprises a quaternary amine and a hydrophobic moiety.

7. The method of any one of claims 2-6, wherein the anion exchange mixed mode chromatography comprises a quaternary amine and a hydrophobic moiety linked to highly crosslinked agarose.

8. The method of any one of claims 2-7, wherein the mixed mode chromatography is a Capto™ Adhere chromatography or a Capto™ Adhere ImpRes chromatography.

9. The method of any one of claims 3-6, wherein the cation exchange mixed mode chromatography comprises a N-benzyl-n-methyl ethanolamine.

10. The method of any one of claims 2-9, wherein the mixed mode chromatography is a Capto™ MMC chromatography or a Capto™ MMC ImpRes chromatography.

11. The method of claim 1, wherein the bispecific antibody is a knob-in-hole (KiH) bispecific antibody.

12. The method of any one of claims 1-11, wherein the fraction contains at least 95% multispecific antibody.

13. The method of any one of claims 1-12, wherein the product-specific impurity is one or more of non-paired antibody arms, antibody homodimers, aggregates, high molecular weight species (HMWS), low molecular weight species (LMWS), acidic variants, or basic variants.

14. The method of claim 13, wherein the fraction contains no more than 5% non-paired antibody arms, no more than 5% antibody homodimers, no more than 2% aggregates or high molecular weight species (HMWS), no more than 2% LMWS, no more than 50% acidic variants, no more than 35% basic variants or no more than 5% of ¾ antibodies.

15. The method of claim 13, wherein the fraction contains

    a) at least 95% -100% multispecific antibody;
    b) no more than 1%-5% non-paired antibody arms;
    c) no more than 1%-5% antibody homodimers;
    d) no more than 1% or 2% HMWS;
    e) no more than 1% or 2% LMWS; and
    f) no more than 5% of ¾ antibodies.

# FIG. 1

**A.**

aX1 (knob) → protein A capture chromatography (bind and elute mode)

aY1 (hole) → protein A capture chromatography (bind and elute mode)

assemble purified aX1 and aY1 to form aX1/Y1 bispecific Ab

multimodal cation exchange chromatography (bind and elute mode)

multimodal anion exchange chromatography (flow through mode)

# FIG. 1

**B.**

aX1 (knob)             aY1 (hole)

↓                   ↓

protein A
capture chromatography
(bind and elute mode)          protein A
capture chromatography
(bind and elute mode)

↘           ↙

assemble purified aX1 and aY1
to form aX1/Y1 bispecific Ab

↓

traditional cation exchange chromatography
(bind and elute mode)

↓

multimodal anion exchange chromatography
(flow through mode)

EP 4 549 463 A2

# FIG. 1

C.

aX1 (knob)                                    aY1 (hole)

↓                                              ↓

protein A                                     protein A
capture chromatography                        capture chromatography
(bind and elute mode)                         (bind and elute mode)

↘                              ↙

assemble purified aX1 and aY1
to form aX1/Y1 bispecific Ab

↓

multimodal cation exchange chromatography
(bind and elute mode)

↓

traditional anion exchange chromatography
(flow through mode)

EP 4 549 463 A2

# FIG. 2

Fab′ A

↓

protein L
capture chromatography
(bind and elute mode)

↓

pyridilate purified Fab′ A

↓

multimodal cation exchange
chromatography
(bind and elute mode)

Fab′ B

↓

protein L
capture chromatography
(bind and elute mode)

↓

oxidize purified Fab′ B

assemble pyridilated Fab′ A and oxidized Fab′ B
to form F(ab′)$_2$ bispecific

↓

Multimodal anion exchange chromatography
(bind and elute mode)

↓

traditional cation exchange chromatography
(bind and elute mode)

↓

Multimodal cation exchange chromatography
(bind and elute mode)

# FIG. 3

**A.**

aX2 (knob)

↓

protein A
capture chromatography
(bind and elute mode)

aY2 (hole)

↓

protein A
capture chromatography
(bind and elute mode)

assemble purified aX2 and aY2
to form aX2/Y2 bispecific Ab

↓

multimodal anion exchange chromatography
(bind and elute mode)

↓

multimodal cation exchange chromatography
(bind and elute mode)

EP 4 549 463 A2

# FIG. 3

**B.**

aX2 (knob)

↓

protein A
capture chromatography
(bind and elute mode)

↘

aY2 (hole)

↓

protein A
capture chromatography
(bind and elute mode)

↙

assemble purified aX2 and aY2
to form aX2/Y2 bispecific Ab

↓

multimodal anion exchange chromatography
(bind and elute mode)

↓

traditional cation exchange chromatography
(bind and elute mode)

EP 4 549 463 A2

# FIG. 4

aX3 (knob)                                    aY3 (hole)

↓                                              ↓

protein A                                      protein A
capture chromatography                         capture chromatography
(bind and elute mode)                          (bind and elute mode)

↘                                          ↙

assemble purified aX3 and aY3
to form aX3/Y3 bispecific Ab

↓

Traditional anion exchange column
(bind an elute mode)

↓

multimodal anion exchange chromatography
(bind and elute mode)

↓

traditional cation exchange chromatography
(bind and elute mode)

EP 4 549 463 A2

# FIG. 5

EP 4 549 463 A2

**A.**

Harvested cell culture fluid (HCCF) from a CHO
expression culture containing
anti-Ang2/anti-VEGF-A bispecific antibody

↓

protein A chromatography
(bind and elute)

↓

multimodal anion exchange chromatography
(bind and elute mode)

↓

multimodal cation exchange chromatography
(bind and elute mode)

# FIG. 5

B.

Harvested cell culture fluid (HCCF) from a CHO
expression culture containing
anti-Ang2/anti-VEGF-A bispecific antibody

↓

protein A chromatography
(bind and elute)

↓

traditional cation exchange chromatography

↓

hydrophobic interaction chromatography

↓

traditional anion exchange chromatography

# FIG. 6

**A.**

Harvested cell culture fluid (HCCF) from a CHO
expression culture containing
anti-VEGF-A/anti-Ang2 bispecific antibody

↓

anti-IgG affinity chromatography
(bind and elute)

↓

multimodal anion exchange chromatography
(bind and elute mode)

↓

multimodal cation exchange chromatography
(bind and elute mode)

EP 4 549 463 A2

# FIG. 6

EP 4 549 463 A2

**B.**

Harvested cell culture fluid (HCCF) from a CHO
expression culture containing
anti-VEGF-A/anti-Ang2 bispecific antibody

↓

anti-IgG affinity chromatography
(bind and elute)

↓

traditional cation exchange chromatography

↓

hydrophobic interaction chromatography

↓

traditional anion exchange chromatography

# FIG. 7

**A.**

aX1 (knob)              aY1 (hole)

↓                  ↓

protein A
capture chromatography
(bind and elute mode)
      
protein A
capture chromatography
(bind and elute mode)

↘          ↙

assemble purified aX1 and aY1
to form aX1/Y1 bispecific Ab

↓

multimodal anion exchange chromatography
(flow through mode)

↓

multimodal cation exchange chromatography
(bind and elute mode)

# FIG. 7

**B.**

aX1 (knob)          aY1 (hole)

protein A
capture chromatography
(bind and elute mode)

protein A
capture chromatography
(bind and elute mode)

assemble purified aX1 and aY1
to form aX1/Y1 bispecific Ab

traditional anion exchange chromatography
(flow through mode)

multimodal cation exchange chromatography
(bind and elute mode)

EP 4 549 463 A2

# FIG. 7

C.

aX1 (knob)          aY1 (hole)

↓             ↓

protein A         protein A
capture chromatography   capture chromatography
(bind and elute mode)    (bind and elute mode)

↘       ↙

assemble purified aX1 and aY1
to form aX1/Y1 bispecific Ab

↓

multimodal anion exchange chromatography
(flow through mode)

↓

traditional cation exchange chromatography
(bind and elute mode)

EP 4 549 463 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62351908
- US 5641870 A
- EP 404097 A
- WO 9311161 A
- US 20110287009 A
- US 20070178552 A
- WO 96027011 A
- WO 98050431 A
- US 5731168 A
- US 5807706 A
- US 5821333 A
- US 7695936 B
- US 8216805 B
- WO 2005035572 A
- WO 03035694 A
- WO 0029004 A
- WO 02051870 A
- US 4816567 A
- US 5693780 A
- US 5500362 A
- US 5821337 A
- WO 2015024896 A
- WO 2011150110 A
- WO 9316185 A
- US 5571894 A
- US 5587458 A
- WO 9308829 A
- WO 2009089004 A1
- US 4676980 A
- WO 2009080251 A
- WO 2009080252 A
- WO 2009080253 A
- WO 2009080254 A
- WO 2010112193 A
- WO 2010115589 A
- WO 2010136172 A
- WO 2010145792 A
- WO 2010145793 A
- US 20060025576 A1
- US 20080069820 A
- WO 9404690 A
- WO 2009089004 A
- US 20090182127 A
- WO 2005063816 A
- US 20090232811 A
- WO 2007147901 A, Kjærgaard
- WO 2010034605 A, Christensen
- WO 9100360 A
- WO 92200373 A
- EP 03089 A
- EP 402226 A
- US 5648237 A, Carter
- US 8241901 B
- US 5639635 A
- WO 2002061090 A
- US 5264365 A, Georgiou
- US 5508192 A, Georgiou
- US 4965199 A
- US 4419446 A
- US 4601978 A
- WO 9411026 A
- US 4767704 A
- US 4657866 A
- US 4927762 A
- US 4560655 A
- US 5122469 A
- WO 9003430 A
- WO 8700195 A
- US RE30985 E
- WO 2013055958 A
- US 7521541 A
- WO 2011117329 A
- WO 2014009465 A

**Non-patent literature cited in the description**

- **CHEN et al.** *J. Mol. Biol.*, 1999, vol. 293, 865-881
- **KABAT et al.** Sequences of Proteins of Immunological Interest. National Institutes of Health, 1991
- **CHOTHIA ; LESK.** *J. Mol. Biol.*, 1987, vol. 196, 901-917
- **BURTON.** *Molec. Immunol.*, 1985, vol. 22, 161-206
- **ZAPATA et al.** *Protein Eng.*, 1995, vol. 8 (10), 1057-1062
- **PLÜCKTHUN.** The Pharmacology of Monoclonal Antibodies. Springer-Verlag, 1994, vol. 113, 269-315

- **HOLLINGER et al.** *Proc. Natl. Acad. Sci. USA*, 1993, vol. 90, 6444-6448
- **ZHU et al.** *Protein Science*, 1997, vol. 6, 781-788
- *Nature*, 1989, vol. 341, 544-546
- *Dev Comp Immunol*, 2006, vol. 30, 43-56
- *Trend Biochem Sci*, 2001, vol. 26, 230-235
- *Trends Biotechnol*, 2003, vol. 21, 484-490
- *FEBS Lett*, 1994, vol. 339, 285-290
- **KOHLER et al.** *Nature*, 1975, vol. 256, 495
- **CLACKSON et al.** *Nature*, 1991, vol. 352, 624-628
- **MARKS et al.** *J. Mol. Biol.*, 1991, vol. 222, 581-597
- **MORRISON et al.** *Proc. Natl. Acad. Sci. USA*, 1984, vol. 81, 6851-6855
- **JONES et al.** *Nature*, 1986, vol. 321, 522-525
- **RIECHMANN et al.** *Nature*, 1988, vol. 332, 323-329
- **PRESTA**. *Curr. Op. Struct. Biol.*, 1992, vol. 2, 593-596
- **GAZZANO-SANTORO et al.** *J. Immunol. Methods*, 1996, vol. 202, 163
- **RAVETCH ; KINET**. *Annu. Rev. Immunol*, 1991, vol. 9, 457-92
- **CLYNES et al.** *Proc. Natl. Acad. Sci.*, 1998, vol. 95, 652-656
- **DAËRON**. *Annu. Rev. Immunol.*, 1997, vol. 15, 203-234
- **CAPEL et al.** *Immunomethods*, 1994, vol. 4, 25-34
- **DE HAAS et al.** *J. Lab. Clin. Med.*, 1995, vol. 126, 330-41
- **GUYER et al.** *J. Immunol.*, 1976, vol. 117, 587
- **KIM et al.** *J. Immunol.*, 1994, vol. 24, 249
- **KINDT et al.** Kuby Immunology. W.H. Freeman and Co., 2007, 91
- **PORTOLANO et al.** *J. Immunol.*, 1993, vol. 150, 880-887
- **CLARKSON et al.** *Nature*, 1991, vol. 352, 624-628
- **GODING**. Monoclonal Antibodies: Principles and Practice. Academic Press, 1986, 59-103
- **KOZBOR**. *J. Immunol.*, 1984, vol. 133, 3001
- **BRODEUR et al.** Monoclonal Antibody Production Techniques and Applications. Marcel Dekker, Inc., 1987, 51-63
- **MUNSON et al.** *Anal. Biochem.*, 1980, vol. 107, 220
- **SKERRA et al.** *Curr. Opinion in Immunol.*, 1993, vol. 5, 256-262
- **PLÜCKTHUN**. *Immunol. Revs.*, 1992, vol. 130, 151-188
- **MCCAFFERTY et al.** *Nature*, 1990, vol. 348, 552-554
- **MARKS et al.** *Bio/Technology*, 1992, vol. 10, 779-783
- **WATERHOUSE et al.** *Nuc. Acids. Res.*, 1993, vol. 21, 2265-2266
- **MORRISON et al.** *Proc. Natl Acad. Sci. USA*, 1984, vol. 81, 6851
- **MORIMOTO et al.** *Journal of Biochemical and Biophysical Methods*, 1992, vol. 24, 107-117
- **BRENNAN et al.** *Science*, 1985, vol. 229, 81
- **CARTER et al.** *Bio/Technology*, 1992, vol. 10, 163-167
- **CUNNINGHAM ; WELLS**. *Science*, 1989, vol. 244, 1081-1085
- **A. L. LEHNINGER**. Biochemistry. Worth Publishers, 1975, 73-75
- **MILSTEIN ; CUELLO**. *Nature*, 1983, vol. 305, 537
- **TRAUNECKER et al.** *EMBO J.*, 1991, vol. 10, 3655
- **KOSTELNY et al.** *J. Immunol.*, 1992, vol. 148 (5), 1547-1553
- **GRUBER et al.** *J. Immunol.*, 1994, vol. 152, 5368
- **TUTT et al.** *J. Immunol.*, 1991, vol. 147, 60
- **BOSTROM et al.** *Science*, 2009, vol. 5921, 1610-1614
- **SURESH et al.** *Methods in Enzymology*, 1986, vol. 121, 210
- **MARVIN ; ZHU**. *Acta Pharmacologica Sincia*, 2005, vol. 26 (6), 649-658
- **KONTERMANN**. *Acta Pharacol. Sin.*, 2005, vol. 26, 1-9
- Molecular weight amino acid minus that of water. Values from Handbook of Chemistry and Physics. Chemical Rubber Publishing Co., 1961
- **A.A. ZAMYATNIN**. *Prog. Biophys. Mol. Biol.*, 1972, vol. 24, 107-123
- **C. CHOTHIA**. *J. Mol. Biol.*, 1975, vol. 105, 1-14
- **ELLMAN et al.** *Meth. Enzym.*, 1991, vol. 202, 301-336
- **NOREN et al.** *Science*, 1989, vol. 244, 182
- Mutagenesis: a Practical Approach. IRL Press, 1991
- **PONDERS ; RICHARDS**. *J. Mol. Biol.*, 1987, vol. 193, 775-791
- **KABAT et al.** Sequences of proteins of immunological interest. 1991, vol. 1, 688-696
- *PNAS*, 2011, vol. 108 (27), 11187-11192
- **WOLF et al.** *Drug Discovery Today*, 2005, vol. 10, 1237-1244
- *J. Mol. Biol.*, 2012, vol. 420, 204-19
- **PACK, P. ; PLUECKTHUN, A.** *Biochemistry*, 1992, vol. 31, 1579-1584
- **PACK et al.** *Bio/Technology*, 1993, vol. 11, 1271-1277
- *Protein Science*, 1997, vol. 6, 781-788

- *Protein Engineering, Design & Selection*, 2010, vol. 23, 667-677
- **KLEIN et al.** *mAbs*, 2012, vol. 4 (6), 653-663
- **SPIESS et al.** Alternative molecular formats and therapeutic applications for bispecific antibodies. *Mol. Immunol.*, 27 January 2015
- **KONTERMANN et al.** *Drug Discovery Today*, 2015, vol. 20, 838-847
- **STEWART et al.** Solid-Phase Peptide Synthesis. W.H. Freeman Co., 1969
- **MERRIFIELD**. *J. Am. Chem. Soc.*, 1963, vol. 85, 2149-2154
- **SAMBROOK et al.** Molecular Cloning: A Laboratory Manual. Cold Spring Harbor Press, 1989
- **WINNACKER**. From Genes to Clones. VCH Publishers, 1987
- **SIEBENLIST et al.** *Cell*, 1980, vol. 20, 269
- **BACHMANN**. Cellular and Molecular Biology. American Society for Microbiology, 1987, vol. 2, 1190-1219
- **BASS et al.** *Proteins*, 1990, vol. 8, 309-314
- **SIMMONS et al.** *J. Immunol. Methods*, 2002, vol. 263, 133-147
- **HARA et al.** *Microbial Drug Resistance*, 1996, vol. 2, 63-72
- **REYES et al.** *Nature*, 1982, vol. 297, 598-601
- **YANIV**. *Nature*, 1982, vol. 297, 17-18
- **GRAHAM et al.** *J. Gen Viral.*, 1977, vol. 36, 59
- **URLAUB et al.** *Proc. Nat/. Acad. Sci. USA*, 1980, vol. 77, 4216
- **MATHER**. *Biol. Reprod.*, 1980, vol. 23, 243-251
- **MATHER et al.** *Annals N.Y. Acad. Sci.*, 1982, vol. 383, 44-68
- **HAM et al.** *Meth. Enz.*, 1979, vol. 58, 44
- **BARNES et al.** *Anal. Biochem.*, 1980, vol. 102, 255
- Remington's Pharmaceutical Sciences. 1980
- **SAMBROOK et al.** Molecular Cloning: A Laboratory Manual. Cold Spring Harbor Laboratory Press, 2001
- Current Protocols in Molecular Biology. 2003
- Methods in Enzymology. Academic Press, Inc.
- Antibodies, A Laboratory Manual, and Animal Cell Culture. 1987
- Methods in Molecular Biology. Humana Press
- **J.P. MATHER** ; **P.E. ROBERTS**. Cell Biology: Introduction to Cell and Tissue Culture. Plenum Press, 1998
- Cell and Tissue Culture: Laboratory Procedures. J. Wiley and Sons, 1993
- Handbook of Experimental Immunology
- Current Protocols in Immunology. 1991
- Short Protocols in Molecular Biology. Wiley and Sons, 1999
- **C.A. JANEWAY** ; **P. TRAVERS**. *Immunobiology*, 1997
- **P. FINCH**. *Antibodies*, 1997
- Antibodies: A Practical Approach. IRL Press, 1988
- Monoclonal Antibodies: A Practical Approach. Oxford University Press, 2000
- **E. HARLOW** ; **D. LANE**. Using Antibodies: A Laboratory Manual. Cold Spring Harbor Laboratory Press, 1999
- The Antibodies. Harwood Academic Publishers, 1995